# EUROPEAN PATENT SPECIFICATION

(11) **EP 3 200 775 B1**
(45) Date of publication and mention of the grant of the patent: **20.11.2019**
(21) Application number: 15782164.6
(22) Date of filing: 02.10.2015
(51) Int. Cl.: A61K 39/395, A61K 31/506, C07K 16/28, A61P 35/00

(54) **COMBINATION THERAPIES**
KOMBINATIONSTHERAPIEN
POLYTHÉRAPIES

(30) Priority: 03.10.2014 US 201462059832 P
(43) Date of publication of application: 09.08.2017
(73) Proprietor: Novartis AG, 4056 Basel (CH)
(72) Inventor: CAO, Zhu, Alexander, Cambridge, MA 02139 (US); RONG, Xianhui, Cambridge, MA 02139 (US); PINZON-ORTIZ, Maria, Consuelo, Cambridge, MA 02139 (US); LONGMIRE, Tyler, Cambridge, MA 02139 (US); LEE, Benjamin, Hyun, Cambridge, MA 02139 (US)
(74) Representative: Elkington and Fife LLP
(86) International application number: PCT/US2015/053799
(87) International publication number: WO 2016/054555

(56) References cited:
- WO-A1-2011/011027
- WO-A1-2012/022814
- WO-A1-2015/112900
- WO-A1-2015/117002
- WO-A1-2015/138920
- WO-A1-2016/028672
- WO-A1-2016/040880
- WO-A1-2016/040892
- WO-A2-2007/113648
- WO-A2-2010/051502
- WO-A2-2015/095423
- WO-A2-2015/095811
- US-A- 5 968 511
- YUAN Z ET AL: "Blockade of inhibitors of apoptosis (IAPs) in combination with tumor-targeted delivery of tumor necrosis factor-[alpha] leads to synergistic antitumor activity", CANCER GENE THERAPY, APPLETON & LANGE, GB, vol. 20, no. 1, 1 January 2013 (2013-01-01), pages 46-56, XP008178507, ISSN: 0929-1903, DOI: 10.1038/CGT.2012.83 [retrieved on 2012-11-16]
- NAKAE S ET AL: "Mast cells enhance T cell activation: importance of mast cell costimulatory molecules and secreted TNF", THE JOURNAL OF IMMUNOLOGY, THE AMERICAN ASSOCIATION OF IMMUNOLOGISTS, US, vol. 176, no. 4, 15 February 2006 (2006-02-15), pages 2238-2248, XP002752656, ISSN: 0022-1767, DOI: 10.4049/JIMMUNOL.176.4.2238
- KNIGHTS A J ET AL: "Inhibitor of apoptosis protein (IAP) antagonists demonstrate divergent immunomodulatory properties in human immune subsets with implications for combination therapy", CANCER IMMUNOLOGY AND IMMUNOTHERAPY, SPRINGER-VERLAG, BERLIN, DE, vol. 62, no. 2, 1 February 2013 (2013-02-01), pages 321-335, XP002752661, ISSN: 0340-7004, DOI: 10.1007/S00262-012-1342-1 [retrieved on 2012-08-26]
- R. MOREIRA DA SILVA: "Nivolumab: Anti-PD-1 monoclonal antibody cancer immunotherapy", DRUGS OF THE FUTURE, vol. 39, no. 1, 1 January 2014 (2014-01-01), pages 15-24, XP055199597, ES ISSN: 0377-8282, DOI: 10.1358/dof.2014.039.01.2103754
- MATTHEW VANNEMAN ET AL: "Combining immunotherapy and targeted therapies in cancer treatment", NATURE REVIEWS CANCER, vol. 12, no. 4, 1 January 2012 (2012-01-01), pages 237-251, XP055045378, ISSN: 1474-175X, DOI: 10.1038/nrc3237
- GARRISON K ET AL: "The small molecule TGF-[beta] signaling inhibitor SM16 synergizes with agonistic OX40 antibody to suppress established mammary tumors and reduce spontaneous metastasis", CANCER IMMUNOLOGY, IMMUNOTHERAPY, SPRINGER, BERLIN, DE, vol. 61, no. 4, 1 April 2012 (2012-04-01), pages 511-521, XP002752658, ISSN: 1432-0851, DOI: 10.1007/S00262-011-1119-Y [retrieved on 2011-10-05]
- ZHUANG J ET AL: "Selective IAP inhibition results in sensitization of unstimulated but not CD40-stimulated chronic lymphocytic leukaemia cells to TRAIL-induced apoptosis", PHARMACOLOGY RESEARCH & PERSPECTIVES, JOHN WILEY & SONS LTD, GB, vol. 2, no. 6, E00081, 1 December 2014 (2014-12-01), pages 1-14, XP002752660, ISSN: 2052-1707, DOI: 10.1002/PRP2.81 [retrieved on 2014-09-01]
- JOSE LUIS PEREZ-GRACIA ET AL: "Orchestrating immune check-point blockade for cancer immunotherapy in combinations", CURRENT OPINION IN IMMUNOLOGY, vol. 27, 1 April 2014 (2014-04-01), pages 89-97, XP055200525, ISSN: 0952-7915, DOI: 10.1016/j.coi.2014.01.002
- VERBRUGGE INGE ET AL: "The curative outcome of radioimmunotherapy in a mouse breast cancer model relies on mTOR signaling", RADIATION RESEARCH, RADIATION RESEARCH SOCIETY, GB, vol. 182, no. 2, 1 August 2014 (2014-08-01), pages 219-229, XP002745809, ISSN: 1938-5404, DOI: 10.1667/RR13511.1 [retrieved on 2014-06-24]
- Q. JIANG ET AL: "mTOR Kinase Inhibitor AZD8055 Enhances the Immunotherapeutic Activity of an Agonist CD40 Antibody in Cancer Treatment", CANCER RESEARCH, vol. 71, no. 12, 3 May 2011 (2011-05-03), pages 4074-4084, XP055099556, ISSN: 0008-5472, DOI: 10.1158/0008-5472.CAN-10-3968
- Wang et al: "The Mdm2 inhibitor, NVP-CGM097, in combination with the BRAF inhibitor NVP-LGX818 elicits synergistic antitumor effects in melanoma", Cancer Research , 1 October 2014 (2014-10-01), XP002756552, Retrieved from the Internet: URL:http://cancerres.aacrjournals.org/cont ent/74/19_Supplement/5466 [retrieved on 2016-04-14]
- Wang et al: "Abstract 2929: The Mdm2 inhibitorNVP-CGM097 enhances the anti-tumor activityof NVP-LDK378 in ALK mutant neuroblastomamodels", Cancer Research , 1 October 2014 (2014-10-01), XP002756553, Retrieved from the Internet: URL:http://cancerres.aacrjournals.org/cont ent/74/19_Supplement/2929 [retrieved on 2016-04-14]
- JIANG X ET AL: "The activation of MAPK in melanoma cells resistant to BRAF inhibition promotes PD-L1 expression that is reversible by MEK and PI3K inhibition", CLINICAL CANCER RESEARCH, THE AMERICAN ASSOCIATION FOR CANCER RESEARCH, US, vol. 19, no. 3, 1 February 2013 (2013-02-01), pages 598-609, XP002746139, ISSN: 1078-0432, DOI: 10.1158/1078-0432.CCR-12-2731 [retrieved on 2012-10-24]
- ANWESHA DEY ET AL: "Nutlin-3 inhibits the NF[kappa]B Pathway in a p53 Dependent Manner: Implications in Lung Cancer Therapy", CELL CYCLE, vol. 6, no. 17, 1 September 2007 (2007-09-01), pages 2178-2185, XP055203309, US ISSN: 1538-4101, DOI: 10.4161/cc.6.17.4643
- Garcia et al: "The Pan-PIM Kinase Inhibitor LGH447 Shows Activity In PIM2-Dependent Multiple Myeloma and In AML Models", Blood , 15 November 2013 (2013-11-15), XP002756554, Retrieved from the Internet: URL:http://www.bloodjournal.org/content/12 2/21/1666 [retrieved on 2016-04-14]
- M PINZON-ORTIZ ET AL: "S710: THE COMBINATION OF JAK INHIBITOR RUXOLITINIB, PAN-PIM INHIBITOR LGH447, AND CDK4/6 INHIBITOR LEE011 IN A PRECLINICAL MOUSE MODEL OF MYELOPROLIFERATIVE NEOPLASIA", HAEMATOLOGICA, THE HEMATOLOGY JOURNAL : OFFICIAL ORGAN OF THE EUROPEAN HEMATOLOGY ASSOCIATION, vol. 99, no. Suppl. 1, 1 June 2014 (2014-06-01), - 15 June 2014 (2014-06-15), page 252, XP055171531, IT ISSN: 0390-6078
- CHERVONTSEVA A M ET AL: "Effect of cytarabine on expression of cell adhesion molecules and on endothelium-leukocyte interaction in vitro.", TERAPEVTICHESKII ARKHIV 2006, vol. 78, no. 7, 2006, pages 67-72, XP008179894, ISSN: 0040-3660
- A. J. CHRISTIANSEN ET AL: "Eradication of solid tumors using histone deacetylase inhibitors combined with immune-stimulating antibodies", PROCEEDINGS OF THE NATIONAL ACADEMY OF SCIENCES, vol. 108, no. 10, 22 February 2011 (2011-02-22), pages 4141-4146, XP055121803, ISSN: 0027-8424, DOI: 10.1073/pnas.1011037108
- Woods et al: "Abstract 4090: Inhibition of class I histone deacetylases promotes robust and durable enhancement of PDL1 expression in melanoma: Rationale for combination therapy", Cancer Research , 1 October 2014 (2014-10-01), XP002756556, Retrieved from the Internet: URL:http://cancerres.aacrjournals.org/cont ent/74/19_Supplement/4090.short [retrieved on 2016-04-14]
- KIM KIBEM ET AL: "Eradication of metastatic mouse cancers resistant to immune checkpoint blockade by suppression of myeloid-derived cells. (Includes Supporting Information)", PROCEEDINGS OF THE NATIONAL ACADEMY OF SCIENCES OF THE UNITED STATES OF AMERICA, vol. 111, no. 32, 12 August 2014 (2014-08-12), pages 11774-7, XP002756557, ISSN: 1091-6490
- OKI Y ET AL: "Immune regulatory effects of panobinostat in patients with Hodgkin lymphoma through modulation of serum cytokine levels and T-cell PD1 expression.", BLOOD CANCER JOURNAL, vol. 4, E236, 2014, pages 1-4, XP002756558, ISSN: 2044-5385
- KLEIN JAN M ET AL: "The histone deacetylase inhibitor LBH589 (panobinostat) modulates the crosstalk of lymphocytes with Hodgkin lymphoma cell lines.", PLOS ONE, vol. 8, no. 11, E79502, 2013, pages 1-6, XP002756559, ISSN: 1932-6203
- SONG W ET AL: "HDAC inhibition by LBH589 affects the phenotype and function of human myeloid dendritic cells.", LEUKEMIA JAN 2011, vol. 25, no. 1, January 2011 (2011-01), pages 161-168, XP002756560, ISSN: 1476-5551
- Song et al: "3681 Phenotypic and Functional Effects of Novel HDAC Inhibitor LBH589 On Human Lymphocyte Populations", 51st ASH Annual Meeting and Exposition , 3 December 2009 (2009-12-03), XP002756561, Retrieved from the Internet: URL:https://ash.confex.com/ash/2009/webpro gram/Paper22684.html [retrieved on 2016-04-14]
- WOODS DAVID M ET AL: "HDAC Inhibition Upregulates PD-1 Ligands in Melanoma and Augments Immunotherapy with PD-1 Blockade.", CANCER IMMUNOLOGY RESEARCH, vol. 3, no. 12, December 2015 (2015-12), pages 1375-1385, XP002756562, ISSN: 2326-6074
- WOODS DAVID M ET AL: "The antimelanoma activity of the histone deacetylase inhibitor panobinostat (LBH589) is mediated by direct tumor cytotoxicity and increased tumor immunogenicity.", MELANOMA RESEARCH, vol. 23, no. 5, October 2013 (2013-10), pages 341-348, XP002756563, ISSN: 1473-5636
- Bellucci et al: "JAK1 and JAK2 Modulate Tumor Cell Susceptibility To Natural Killer (NK) Cells Through Regulation Of PDL1 Expression", Blood , 15 November 2013 (2013-11-15), XP002756564, Retrieved from the Internet: URL:http://www.bloodjournal.org/content/12 2/21/3472.full.pdf [retrieved on 2016-04-14]
- QUINTARELLI CONCETTA ET AL: "Selective strong synergism of Ruxolitinib and second generation tyrosine kinase inhibitors to overcome bone marrow stroma related drug resistance in chronic myelogenous leukemia", LEUKEMIA RESEARCH, NEW YORK,NY, US, vol. 38, no. 2, 15 November 2013 (2013-11-15), pages 236-242, XP028812560, ISSN: 0145-2126, DOI: 10.1016/J.LEUKRES.2013.11.006
- CHRISTIANSSON LISA ET AL: "The tyrosine kinase inhibitors imatinib and dasatinib reduce myeloid suppressor cells and release effector lymphocyte responses.", MOLECULAR CANCER THERAPEUTICS, vol. 14, no. 5, May 2015 (2015-05), pages 1181-1191, XP002756565, ISSN: 1538-8514
- HU YI ET AL: "Essential role of AKT in tumor cells addicted to FGFR.", ANTI-CANCER DRUGS, vol. 25, no. 2, February 2014 (2014-02), pages 183-188, XP008179886, ISSN: 1473-5741
- J. ACQUAVIVA ET AL: "FGFR3 Translocations in Bladder Cancer: Differential Sensitivity to HSP90 Inhibition Based on Drug Metabolism", MOLECULAR CANCER RESEARCH, vol. 12, no. 7, 1 July 2014 (2014-07-01), pages 1042-1054, XP055182977, ISSN: 1541-7786, DOI: 10.1158/1541-7786.MCR-14-0004
- MITTENDORF ELIZABETH A ET AL: "PD-L1 expression in triple-negative breast cancer.", CANCER IMMUNOLOGY RESEARCH, vol. 2, no. 4, April 2014 (2014-04), pages 361-370, XP002756567, ISSN: 2326-6074
- GRYGIELEWICZ PAULINA ET AL: "Epithelial-mesenchymal transition confers resistance to selective FGFR inhibitors in SNU-16 gastric cancer cells", GASTRIC CANCER, SPRINGER JAPAN, TOKYO, vol. 19, no. 1, 19 November 2014 (2014-11-19), pages 53-62, XP035587475, ISSN: 1436-3291, DOI: 10.1007/S10120-014-0444-1 [retrieved on 2014-11-19]

## Description

### BACKGROUND

The ability of T cells to mediate an immune response against an antigen requires two distinct signaling interactions (Viglietta, V. et al. (2007) Neurotherapeutics 4:666-675; Korman, A. J. et al. (2007) Adv. Immunol. 90:297-339). First, an antigen that has been arrayed on the surface of antigen-presenting cells (APC) is presented to an antigen-specific naive CD4⁺ T cell. Such presentation delivers a signal via the T cell receptor (TCR) that directs the T cell to initiate an immune response specific to the presented antigen. Second, various co-stimulatory and inhibitory signals mediated through interactions between the APC and distinct T cell surface molecules trigger the activation and proliferation of the T cells and ultimately their inhibition.

The immune system is tightly controlled by a network of costimulatory and co-inhibitory ligands and receptors. These molecules provide the second signal for T cell activation and provide a balanced network of positive and negative signals to maximize immune responses against infection, while limiting immunity to self (Wang, L. *et al.* (Epub Mar. 7, 2011) J. Exp. Med. 208(3):577-92; Lepenies, B. et al. (2008) Endocrine, Metabolic & Immune Disorders-Drug Targets 8:279-288). Examples of costimulatory signals include the binding between the B7.1 (CD80) and B7.2 (CD86) ligands of the APC and the CD28 and CTLA-4 receptors of the CD4⁺ T-lymphocyte (Sharpe, A. H. et al. (2002) Nature Rev. Immunol. 2:116-126; Lindley, P. S. et al. (2009) Immunol. Rev. 229:307-321). Binding of B7.1 or B7.2 to CD28 stimulates T cell activation, whereas binding of B7.1 or B7.2 to CTLA-4 inhibits such activation (Dong, C. et al. (2003) Immunolog. Res. 28(1):39-48; Greenwald, R. J. et al. (2005) Ann. Rev. Immunol. 23:515-548). CD28 is constitutively expressed on the surface of T cells (Gross, J., et al. (1992) J. Immunol. 149:380-388), whereas CTLA-4 expression is rapidly up-regulated following T-cell activation (Linsley, P. et al. (1996) Immunity 4:535-543).

Other ligands of the CD28 receptor include a group of related B7 molecules, also known as the "B7 Superfamily" (Coyle, A. J. et al. (2001) Nature Immunol. 2(3):203-209; Sharpe, A. H. et al. (2002) Nature Rev. Immunol. 2:116-126; Collins, M. et al. (2005) Genome Biol. 6:223.1-223.7; Korman, A. J. et al. (2007) Adv. Immunol. 90:297-339). Several members of the B7 Superfamily are known, including B7.1 (CD80), B7.2 (CD86), the inducible co-stimulator ligand (ICOS-L), the programmed death-1 ligand (PD-L1; B7-H1), the programmed death-2 ligand (PD-L2; B7-DC), B7-H3, B7-H4 and B7-H6 (Collins, M. et al. (2005) Genome Biol. 6:223.1-223.7).

The Programmed Death 1 (PD-1) protein is an inhibitory member of the extended CD28/CTLA-4 family of T cell regulators (Okazaki et al. (2002) Curr Opin Immunol 14: 391779-82; Bennett et al. (2003) J. Immunol. 170:711-8). Other members of the CD28 family include CD28, CTLA-4, ICOS and BTLA. PD-1 is suggested to exist as a monomer, lacking the unpaired cysteine residue characteristic of other CD28 family members. PD-1 is expressed on activated B cells, T cells, and monocytes.

The PD-1 gene encodes a 55 kDa type I transmembrane protein (Agata et al. (1996) Int Immunol. 8:765-72). Although structurally similar to CTLA-4, PD-1 lacks the MYPPY motif (SEQ ID NO: 1) that is important for B7-1 and B7-2 binding. Two ligands for PD-1 have been identified, PD-L1 (B7-H1) and PD-L2 (B7-DC), that have been shown to downregulate T cell activation upon binding to PD-1 (Freeman et al. (2000) J. Exp. Med. 192:1027-34; Carter et al. (2002) Eur. J. Immunol. 32:634-43). Both PD-L1 and PD-L2 are B7 homologs that bind to PD-1, but do not bind to other CD28 family members. PD-L1 is abundant in a variety of human cancers (Dong et al. (2002) Nat. Med. 8:787-9).

PD-1 is known as an immunoinhibitory protein that negatively regulates TCR signals (Ishida, Y. et al. (1992) EMBO J. 11:3887-3895; Blank, C. *et al.* (Epub 2006 Dec. 29) Immunol. Immunother. 56(5):739-745). The interaction between PD-1 and PD-L1 can act as an immune checkpoint, which can lead to, *e.g.,* a decrease in tumor infiltrating lymphocytes, a decrease in T-cell receptor mediated proliferation, and/or immune evasion by cancerous cells (Dong et al. (2003) J. Mol. Med. 81:281-7; Blank et al. (2005) Cancer Immunol. Immunother. 54:307-314; Konishi et al. (2004) Clin. Cancer Res. 10:5094-100). Immune suppression can be reversed by inhibiting the local interaction of PD-1 with PD-L1 or PD-L2; the effect is additive when the interaction of PD-1 with PD-L2 is blocked as well (Iwai et al. (2002) Proc. Nat'l. Acad. Sci. USA 99:12293-7; Brown et al. (2003) J. Immunol. 170:1257-66).

WO-A-2015/112900 (publication date 30 July 2015; earliest claimed priority date 24 January 2014) describes antibody molecules that specifically bind to PD-1. The anti-PD-1 antibody molecules can be used to treat, prevent and/or diagnose cancerous or infectious conditions and disorders. Pharmaceutical compositions comprising the antibody molecules are also provided, with the anti-PD-1 antibody molecules used alone or in combination with other agents or therapeutic modalities.

Given the importance of immune checkpoint pathways in regulating an immune response, the need exists for developing novel combination therapies that activate the immune system.

### SUMMARY

The present invention is defined by the claims. The invention provides combination therapies and compositions comprising an anti-PD-1 antibody chosen from Nivolumab, Pembrolizumab or Pidilizumab, and LCL161, wherein LCL161 is (S)-N-((S)-1-cyclohexyl-2-((S)-2-(4-(4-fluorobenzoyl)thiazol-2-yl)pyrrolidin-1-yl)-2-oxoethyl)-2-(methylamino)propanamide or a pharmaceutically acceptable salt thereof. The combinations described herein can provide a beneficial effect, *e.g.,* in the treatment of a cancer, such as an enhanced anti-cancer effect, reduced toxicity and/or reduced side effects. For example, the immunomodulator, the second therapeutic agent, or both, can be administered at a lower dosage than would be required to achieve the same therapeutic effect compared to a monotherapy dose. Thus, compositions and methods for treating proliferative disorders, including cancer, using the aforesaid combination therapies are disclosed. The combination is also provided for use in a method of treating a hematopoiesis disorder in a subject.

Accordingly, in one aspect, the invention features the combination of the invention for use in a method of treating (*e.g*., inhibiting, reducing, ameliorating, or preventing) a proliferative condition or disorder (*e.g.*, a cancer) in a subject. The method includes administering to the subject the immunomodulator (Nivolumab, Pembrolizumab or Pidilizumab) and the second therapeutic agent, LCL161 wherein LCL161 is (S)-N-((S)-1-cyclohexyl-2-((S)-2-(4-(4-fluorobenzoyl)thiazol-2-yl)pyrrolidin-1-yl)-2-oxoethyl)-2-(methylamino)propanamide or a pharmaceutically acceptable salt thereof, thereby treating the proliferative condition or disorder (*e.g.*, the cancer). The immunomodulator is Nivolumab, Pembrolizumab or Pidilizumab, inhibitors of the immune checkpoint molecule PD-1. The second therapeutic agent is the IAP inhibitor LCL161, wherein LCL161 is (S)-N-((S)-1-cyclohexyl-2-((S)-2-(4-(4-fluorobenzoyl)thiazol-2-yl)pyrrolidin-1-yl)-2-oxoethyl)-2-(methylamino)propanamide or a pharmaceutically acceptable salt thereof. The combination of the immunomodulator and the second agent can be administered together in a single composition or administered separately in two or more different compositions, *e.g*., one or more compositions or dosage forms as described herein. The administration of the immunomodulator and the second agent can be in any order. For example, the immunomodulator can be administered concurrently with, prior to, or subsequent to, the second agent.

In another aspect, the disclosure features a method of reducing an activity (e.g., growth, survival, or viability, or all), of a proliferative (*e.g*., a cancer) cell. The method includes contacting the cell with the immunomodulator and the second therapeutic agent, thereby reducing an activity in the cell. The immunomodulator is Nivolumab, Pembrolizumab or Pidilizumab, inhibitors of the immune checkpoint molecule PD-1. The second therapeutic agent is the IAP inhibitor LCL161 wherein LCL161 is (S)-N-((S)-1-cyclohexyl-2-((S)-2-(4-(4-fluorobenzoyl)thiazol-2-yl)pyrrolidin-1-yl)-2-oxoethyl)-2-(methylamino)propanamide or a pharmaceutically acceptable salt thereof.

In some embodiments, the methods described herein can be used *in vitro.* For example, *in vitro* hPBMC-based assays can be used to screen for combination signals of immunomodulators and second therapeutic agents, as disclosed, *e.g.,* in Wang, C. et al. (2014) Cancer Immunology Research 2:846-856. In some embodiments, the methods described herein can be used *in vivo*, *e.g.*, in an animal subject or model or as part of a therapeutic protocol. The contacting of the cell with the immunomodulator and the second agent can be in any order. In certain embodiments, the cell is contacted with the immunomodulator concurrently, prior to, or subsequent to, the second agent. In some embodiments, the method described herein is used to measure tumor lymphocyte infiltration (TLI) *in vitro* or *in vivo*, as disclosed, *e.g.,* in Frederick, D.T. et al. (2013) Clinical Cancer Research 19:1225-31.

In some disclosed embodiments, the method includes contacting the cell with the immunomodulator and the second therapeutic agent, in an animal model. In some disclosed embodiments, the animal model has a mutation that inhibits or activates IAP, EGF receptor, cMET, ALK, CDK4/6, PI3K, BRAF, FGF receptor, MEK, and/or BCR-ABL. In one exemplary disclosed embodiment, an animal model is a mouse model implanted with MC38 murine colon carcinoma. In another exemplary embodiment, an animal model is a mouse model with an inactivated p110δ isoform of PI3 kinase (*e.g.*, p110δ^{D910A}) as disclosed, *e.g.,*in Ali,K.,et al., (2014) Nature 510:407-411.

In some embodiments, an immune phenotype is determined by measuring one or more of expression, activation, signalling, flow cytometry, mRNA analysis, cytokine levels and/or immunohistochemisty. In some embodiments, the immune phenotype is determined systemically, *e.g.,* in PBMCs. In some embodiments, the immune phenotype is determined *in situ, e.g,* in tumor cells. In some embodiments, one or more of the following parameters is characterized to determine an immune phenotype: checkpoint induction; level of M1 macrophages relative to level of M2 macrophages; level of effector T cells relative to level of regulatory T cells; and/or level of T_{H1} cells relative to T_{H2/H17} cells.

In another aspect, the invention features a composition, or a kit comprising one or more compositions, formulations or dosage formulations, comprising the immunomodulator and the second therapeutic agent. The immunomodulator is Nivolumab, Pembrolizumab or Pidilizumab, inhibitors of the immune checkpoint molecule PD-1. The second therapeutic agent is the IAP inhibitor LCL161 wherein LCL161 is (S)-N-((S)-1-cyclohexyl-2-((S)-2-(4-(4-fluorobenzoyl)thiazol-2-yl)pyrrolidin-1-yl)-2-oxoethyl)-2-(methylamino)propanamide or a pharmaceutically acceptable salt thereof. In one embodiment, the composition comprises a pharmaceutically acceptable carrier. The immunomodulator and the second agent can be present in a single composition or as two or more different compositions. The immunomodulator and the second agent can be administered via the same administration route or via different administration routes. In one embodiment, the pharmaceutical combination comprises the immunomodulator and the second agent separately or together.

In one embodiment, the composition, formulation or pharmaceutical combination is for use as a medicine, *e.g.,* for the treatment of a proliferative disease (*e.g.,* a cancer as described herein). In some embodiments, the immunomodulator and the second agent are administered concurrently, *e.g*., independently at the same time or within an overlapping time interval, or separately within time intervals. In certain embodiment, the time interval allows the immunomodulator and the second agent to be jointly active. In one embodiment, the composition, formulation or pharmaceutical combination includes an amount which is jointly therapeutically effective for the treatment of a proliferative disease, *e.g.,* a cancer as described herein.

Kits, *e.g*., therapeutic kits, that include the immunomodulator and the second therapeutic agent, and instructions for use, are also disclosed.

Additional features or embodiments of the methods, compositions, dosage formulations, and kits described herein include one or more of the following:
The immunomodulator is Nivolumab, Pembrolizumab or Pidilizumab, inhibitors of the immune checkpoint molecule PD-1.

This inhibitor of the inhibitory signal is an antibody that binds to the inhibitory molecule *e.g* is an antibody that binds to PD-1.

In one disclosed aspect, an antibody molecule is a full antibody or fragment thereof (*e.g.*, a Fab, F(ab')₂, Fv, or a single chain Fv fragment (scFv)). In yet other aspects, the antibody molecule has a heavy chain constant region (Fc) chosen from, *e.g.,* the heavy chain constant regions of IgG1, IgG2, IgG3, IgG4, IgM, IgA1, IgA2, IgD, and IgE; particularly, chosen from, *e.g.,* the heavy chain constant regions of IgG1, IgG2, IgG3, and IgG4, more particularly, the heavy chain constant region of IgG1 or IgG4 (e.g., human IgG1 or IgG4). In one embodiment, the heavy chain constant region is human IgG1 or human IgG4. In one embodiment, the constant region is altered, *e.g*., mutated, to modify the properties of the antibody molecule (*e.g.,* to increase or decrease one or more of: Fc receptor binding, antibody glycosylation, the number of cysteine residues, effector cell function, or complement function).

In certain disclosed aspects, the antibody molecule is in the form of a bispecific or multispecific antibody molecule. In one disclosed aspect, the bispecific antibody molecule has a first binding specificity to PD-1 or PD-L1 and a second binding specifity, *e.g.,* a second binding specificity to TIM-3, LAG-3, or PD-L2. In one the bispecific antibody molecule binds to PD-1 or PD-L1 and TIM-3. In another aspect, the bispecific antibody molecule binds to PD-1 or PD-L1 and LAG-3. In another aspect, the bispecific antibody molecule binds to PD-1 or PD-L1 and CEACAM (*e.g*., CEACAM-1, -3 and/or -5). In another aspect, the bispecific antibody molecule binds to PD-1 or PD-L1 and CEACAM-1. In still another aspect, the bispecific antibody molecule binds to PD-1 or PD-L1 and CEACAM-3. In yet another aspect, the bispecific antibody molecule binds to PD-1 or PD-L1 and CEACAM-1. In another aspect, the bispecific antibody molecule binds to PD-1 or PD-L1. In yet another aspect, the bispecific antibody molecule binds to PD-1 and PD-L2. In another aspect, the bispecific antibody molecule binds to TIM-3 and LAG-3. In another aspect, the bispecific antibody molecule binds to CEACAM (*e.g*., CEACAM-1, -3 and/or - 5) and LAG-3. In another aspect, the bispecific antibody molecule binds to CEACAM (e.g., CEACAM-1, -3 and/or -5) and TIM-3. Any combination of the aforesaid molecules can be made in a multispecific antibody molecule, *e.g.,* a trispecific antibody that includes a first binding specificity to PD-1 or PD-1, and a second and third binding specifities to two or more of: TIM-3, CEACAM (*e.g*., CEACAM-1, -3 and/or -5), LAG-3, or PD-L2.

In certain disclosed embodiments, the immunomodulator is an inhibitor of PD-1, *e.g.,* human PD-1. In another disclosed aspect, the immunomodulator is an inhibitor of PD-L1, *e.g*., human PD-L1. In one aspect, the inhibitor of PD-1 or PD-L1 is an antibody molecule to PD-1 or PD-L1. The PD-1 or PD-L1 inhibitor can be administered alone, or in combination with other immunomodulators, *e.g*., in combination with an inhibitor of LAG-3, TIM-3, CEACAM (*e.g*., CEACAM-1, -3 and/or -5) or CTLA-4. In an exemplary aspect of the disclosure, the inhibitor of PD-1 or PD-L1, *e.g.,* the anti-PD-1 or PD-L1 antibody molecule, is administered in combination with a LAG-3 inhibitor, *e.g*., an anti-LAG-3 antibody molecule. In another aspect, the inhibitor of PD-1 or PD-L1, *e.g.,* the anti-PD-1 or PD-L1 antibody molecule, is administered in combination with a TIM-3 inhibitor, *e.g*., an anti-TIM-3 antibody molecule. In yet other aspects, the inhibitor of PD-1 or PD-L1, *e.g.,* the anti-PD-1 antibody molecule, is administered in combination with a LAG-3 inhibitor, *e.g*., an anti-LAG-3 antibody molecule, and a TIM-3 inhibitor, *e.g*., an anti-TIM-3 antibody molecule. In another aspect, the inhibitor of PD-1 or PD-L1, *e.g.,* the anti-PD-1 or PD-L1 antibody molecule, is administered in combination with a CEACAM (*e.g.,* CEACAM-1, -3 and/or -5) inhibitor, *e.g.,* an anti-CEACAM antibody molecule. In another aspect, the inhibitor of PD-1 or PD-L1, *e.g.,* the anti-PD-1 or PD-L1 antibody molecule, is administered in combination with a CEACAM-1 inhibitor, *e.g*., an anti-CEACAM-1 antibody molecule. In another aspect, the inhibitor of PD-1 or PD-L1, *e.g.,* the anti-PD-1 or PD-L1 antibody molecule, is administered in combination with a CEACAM-3 inhibitor, *e.g*., an anti-CEACAM-3 antibody molecule. In another aspect, the inhibitor of PD-1 or PD-L1, *e.g.,* the anti-PD-1 or PD-L1 antibody molecule, is administered in combination with a CEACAM-5 inhibitor, *e.g*., an anti-CEACAM-5 antibody molecule. Other combinations of immunomodulators with a PD-1 inhibitor (*e.g*., one or more of PD-L2, CTLA-4, TIM-3, LAG-3, VISTA, BTLA, TIGIT, LAIR1, CD160, 2B4 and/or TGFR) are also within the present disclosure. Any of the antibody molecules known in the art or disclosed herein can be used in the aforesaid combinations of inhibitors of checkpoint molecule.

### Exemplary Inhibitors of Immune Checkpoint Molecules

According to the invention, the PD-1 inhibitor is an anti-PD-1 antibody chosen from Nivolumab, Pembrolizumab or Pidilizumab.

In some embodiments of the invention, the anti-PD-1 antibody is Nivolumab. Alternative names for Nivolumab include MDX- 1106, MDX-1106-04, ONO-4538, or BMS-936558. The anti-PD-1 antibody is Nivolumab (CAS Registry Number: 946414-94-4). Nivolumab is a fully human IgG4 monoclonal antibody which specifically blocks PD-1. Nivolumab (clone 5C4) and other human monoclonal antibodies that specifically bind to PD-1 are disclosed in US 8,008,449 and WO2006/121168.

In other embodiments of the invention, the anti-PD-1 antibody is Pembrolizumab. Pembrolizumab (Trade name KEYTRUDA formerly Lambrolizumab, also known as Merck 3745, MK-3475 or SCH-900475) is a humanized IgG4 monoclonal antibody that binds to PD-1. Pembrolizumab is disclosed, *e.g.,* in Hamid, O. et al. (2013) New England Journal of Medicine 369 (2): 134-44, WO2009/114335, and US 8,354,509.

In some embodiments of the invention, the anti-PD-1 antibody is Pidilizumab. Pidilizumab (CT-011; Cure Tech) is a humanized IgGlk monoclonal antibody that binds to PD-1. Pidilizumab and other humanized anti-PD-1 monoclonal antibodies are disclosed in WO2009/101611. Other anti-PD-1 antibodies are disclosed in US 8,609,089, US 2010028330, and/or US 20120114649. Other anti-PD-1 antibodies include AMP 514 (Amplimmune).

In some aspects of the disclosure, the PD-1 inhibitor is an immunoadhesin (*e.g*., an immunoadhesin comprising an extracellular or PD-1 binding portion of PD-L1 or PD-L2 fused to a constant region (*e.g.,* an Fc region of an immunoglobulin sequence)). In some aspects, the PD-1 inhibitor is AMP-224.

In some disclosed aspects, the PD-L1 inhibitor is anti-PD-L1 antibody. In some embodiments, the anti-PD-Ll inhibitor is chosen from YW243.55.S70, MPDL3280A, MEDI-4736, MSB-0010718C, or MDX-1105.

In one disclosed aspect, the PD-L1 inhibitor is MDX-1105. MDX-1105, also known as BMS-936559, is an anti-PD-L1 antibody described in WO2007/005874.

In one disclosed aspect, the PD-L1 inhibitor is YW243.55.S70. The YW243.55.S70 antibody is an anti-PD-Ll described in WO 2010/077634 (heavy and light chain variable region sequences shown in SEQ ID Nos. 20 and 21, respectively, of WO 2010/077634).

In one disclosed aspect, the PD-L1 inhibitor is MDPL3280A (Genentech / Roche). MDPL3280A is a human Fc optimized IgG1 monoclonal antibody that binds to PD-L1. MDPL3280A and other human monoclonal antibodies to PD-L1 are disclosed in U.S. Patent No.: 7,943,743 and U.S Publication No.: 20120039906.

In other disclosed aspects, the PD-L2 inhibitor is AMP-224. AMP-224 is a PD-L2 Fc fusion soluble receptor that blocks the interaction between PD-1 and B7-H1 (B7-DCIg; Amplimmune; *e.g*., disclosed in WO2010/027827 and WO2011/066342).

In one disclosed aspect, the LAG-3 inhibitor is an anti-LAG-3 antibody molecule. In one aspect, the LAG-3 inhibitor is BMS-986016, disclosed in more detail herein below.

In one disclosed aspect, the TIM-3 inhibitor is an anti-TIM-3 antibody molecule, *e.g.,* an anti-TIM-3 antibody molecule as described herein.

One or more of the aforesaid inhibitors of immune checkpoint molecules are disclosed in combination with one or more of the second agents disclosed in Table 1, as more specifically exemplified below. In aspects, the second agent is chosen from one or more of:
1) (S)-N-((S)-1-cyclohexyl-2-((S)-2-(4-(4-fluorobenzoyl)thiazol-2-yl)pyrrolidin-1-yl)-2-oxoethyl)-2-(methylamino)propanamide;
2) ((1R, 9S, 12S, 15R, 16E, 18R, 19R, 21R, 23S, 24E, 26E, 28E, 30S, 32S, 35R)-1,18-dihydroxy-12-{(1R)-2-[(1S, 3R, 4R)-4-(2-hydroxyethoxy)-3-methoxycyclohexyl]-1-methylethyl}-19,30-dimethoxy-15,17,21,23,29,35-hexamethyl-11,36-dioxa-4-aza-tricyclo[30.3.1.04,9] hexatriaconta-16,24,26,28-tetraene-2,3,10,14,20-pentaone);
3) (S)-1-(4-chlorophenyl)-7-isopropoxy-6-methoxy-2-(4-{methyl-[4-(4-methyl-3-oxo-piperazin-1-yl)-trans-cyclohexylmethyl]-amino}phenyl)-1,4-dihydro-2H-isoquinolin-3one;
4) N-(4-((1R,3S,5S)-3-amino-5-methylcyclohexyl)pyridin-3-yl)-6-(2,6-difluorophenyl)-5-fluoropicolinamide;
5) anti-HER3 monoclonal antibody or antigen binding fragment thereof, that comprises a VH of SEQ ID NO: 141 and VL of SEQ ID NO: 140, as described in U.S. 8,735,551;
6) (E)-N-hydroxy-3-(4-(((2-(2-methyl-1H-indol-3-yl)ethyl)amino)methyl)phenyl) acrylamide;
7) (3R)-3-cyclopentyl-3-[4-(7H-pyrrolo-[2,3-d]pyrimidin-4-yl)-1H-pyrazol-1-yl]propanenitrile; and/or
8) 8-(2,6-difluoro-3,5-dimethoxy-phenyl)-quinoxaline-5-carboxylic acid (4-dimethylaminomethyl-1H-imidazol-2-yl)-amide.

According to the invention, the second agent is (S)-N-((S)-1-cyclohexyl-2-((S)-2-(4-(4-fluorobenzoyl)thiazol-2-yl)pyrrolidin-1-yl)-2-oxoethyl)-2-(methylamino)propanamide or a pharmaceutically-acceptable salt thereof.

### Exemplary Combination Therapies

In one embodiment of the invention, the inhibitor of PD-1 is Nivolumab (CAS Registry No: 946414-94-4) disclosed in *e.g.,* US 8,008,449, and having a sequence disclosed herein, *e.g.,* a heavy chain sequence of SEQ ID NO: 2 and a light chain sequence of SEQ ID NO: 3 (or a sequence substantially identical or similar thereto, *e.g.,* a sequence at least 85%, 90%, 95% identical or higher to the sequence specified).

In another embodiment of the invention, the inhibitor of PD-1 is Pembrolizumab disclosed in, *e.g.,* US 8,354,509 and WO 2009/114335, and having a sequence disclosed herein, *e.g.,* a heavy cahin sequence of SEQ ID NO: 4 and a light chain sequence of SEQ ID NO: 5 (or a sequence substantially identical or similar thereto, *e.g.,* a sequence at least 85%, 90%, 95% identical or higher to the sequence specified).

In an aspect of the disclosure, the inhibitor of PD-L1 is MSB0010718C (also referred to as A09-246-2) disclosed in, *e.g.,* WO 2013/0179174, and having a sequence disclosed herein, *e.g.,* a heavy cahin sequence of SEQ ID NO: 6 and a light chain sequence of SEQ ID NO: 7 (or a sequence substantially identical or similar thereto, *e.g.,* a sequence at least 85%, 90%, 95% identical or higher to the sequence specified).

In certain embodiments, the PD-1 inhibitor, *e.g.,* the anti-PD-1 antibody (*e.g.,* Nivolumab) is used in a method or composition described herein. For example, the PD-1 antibody (Nivolumab or Pembrolizumab or Pidilizumab) is used in combination with the Inhibitor of Apoptosis (IAP) inhibitor inhibitor LCL161 wherein LCL161 is (S)-N-((S)-1-cyclohexyl-2-((S)-2-(4-(4-fluorobenzoyl)thiazol-2-yl)pyrrolidin-1-yl)-2-oxoethyl)-2-(methylamino)propanamide or a pharmaceutically acceptable salt thereof. In one aspect, one or more of the aforesaid combinations is used to treat a cancer, *e.g.,* a cancer described herein (*e.g.,* a cancer disclosed in Table 1). Each of these combinations is discussed in more detail below.

In one aspect, the inhibitor of the immune checkpoint molecule (alone or in combination with other immunomodulators) is used in combination with the IAP inhibitor to treat a cancer, *e.g.,* a cancer described herein (*e.g.,* a cancer disclosed in Table 1). The IAP inhibitor is LCL161 as disclosed herein, or in a publication recited in Table 1. In certain aspects, the IAP inhibitor is disclosed, *e.g.,* in U.S. Patent No. 8,546,336. LCL161 has the structure provided in Table 1, or as disclosed in the publication recited in Table 1. In one aspect, the inhibitor of the immune checkpoint molecule (Nivolumab, Pembrolizumab or Pidilizumab) is used in combination with LCL161 to treat a cancer or disorder described herein, *e.g.,* in Table 1, *e.g.,* a solid tumor, *e.g.,* a breast cancer, colon cancer, or a pancreatic cancer; or a hematological malignancy, *e.g*., multiple myeloma or a hematopoeisis disorder.

The inhibitor of the immune checkpoint molecule (alone or in combination with other immunomodulators) is used in combination with LCL161, wherein LCL161 is (S)-N-((S)-1-cyclohexyl-2-((S)-2-(4-(4-fluorobenzoyl)thiazol-2-yl)pyrrolidin-1-yl)-2-oxoethyl)-2-(methylamino)propanamide.

In an embodiment, LCL161 is administered at a dose (*e.g.,* oral dose) of about 10-3000 mg, *e.g*., about 20-2400 mg, about 50-1800 mg, about 100-1500 mg, about 200-1200 mg, about 300-900 mg, *e.g*., about 600 mg, about 900 mg, about 1200 mg, about 1500 mg, about 1800 mg, about 2100 mg, or about 2400 mg. In an embodiment, LCL161 is administered once a week or once every two weeks. In an embodiment, LCL161 is administered prior to administration of the immune checkpoint inhibitor (*e.g*., the anti-PD-1 antibody). For example, LCL161 can be administered one, two, three, four or five days or more before the anti-PD-1 antibody is administered. In another embodiment, LCL161 is administred concurrently or substantially concurrently (*e.g*., on the same day) with the anti-PD-1 antibody. In yet another embodiment, LCL161 is administered after administration of the immune checkpoint inhibitor (*e.g*., the anti-PD-1 antibody).

In some aspects, any of the aforesaid combinations can further include one or more of the second agents described herein below, *e.g.,* one or more of the additional compounds shown in Table 1 (*e.g.,* one or more of: an EGF receptor inhibitor, a c-MET inhibitor, an ALK inhibitor, a CDK4/6 inhibitor, a PI3K inhibitor, a BRAF inhibitor, a CAR T cell inhibitor, a MEK inhibitor or a BCR-ABL inhibitor as described herein).

### Cancers and Subjects

In certain embodiments of the compositions and methods described herein, the proliferative disorder or condition, *e.g*., the cancer, includes but is not limited to, a solid tumor, a soft tissue tumor (*e.g*., a hematological cancer, leukemia, lymphoma, or myeloma), and a metastatic lesion of any of the aforesaid cancers. In one embodiment, the cancer is a solid tumor. Examples of solid tumors include malignancies, *e.g*., sarcomas, adenocarcinomas, and carcinomas, of the various organ systems, such as those affecting the lung, breast, ovarian, lymphoid, gastrointestinal (*e.g.,* colon), anal, genitals and genitourinary tract (*e.g.*, renal, urothelial, bladder cells, prostate), pharynx, CNS (*e.g*., brain, neural or glial cells), head and neck, skin (*e.g*., melanoma), and pancreas, as well as adenocarcinomas which include malignancies such as colon cancers, rectal cancer, renal-cell carcinoma, liver cancer, non-small cell lung cancer, cancer of the small intestine and cancer of the esophagus. The cancer may be at an early, intermediate, late stage or metastatic cancer.

In one embodiment, the cancer is chosen from a cancer disclosed in Table 1. For example, the cancer can be chosen from a solid tumor, *e.g.,* a lung cancer (*e.g.,* a non-small cell lung cancer (NSCLC) (*e.g.,* a NSCLC with squamous and/or non-squamous histology)), a colorectal cancer, a melanoma (*e.g.,* an advanced melanoma), a head and neck cancer (*e.g.,* head and neck squamous cell carcinoma (HNSCC), a digestive/gastrointestinal cancer, a gastric cancer, a neurologic cancer, a glioblastoma (*e.g*., glioblastoma multiforme), an ovarian cancer, a renal cancer, a liver cancer, a pancreatic cancer, a prostate cancer, a liver cancer; a breast cancer, an anal cancer, a gastro-esophageal cancer, a thyroid cancer, a cervical cancer; or a hematological cancer (*e.g*., chosen from a Hogdkin lymphoma, a non-Hodgkin lymphoma, a lymphocytic leukemia, or a myeloid leukemia).

In one embodiment, the cancer is a colon cancer, *e.g.,* a colon cancer that expresses an IAP, *e.g.,* a human IAP. The human IAP family includes, *e.g.,* NAIP, XIAP, cIAP1, cIAP2, ILP2, BRUCE, surviving, and livin.

In one embodiment, the cancer is a non-small cell lung cancer (NSCLC), *e.g.,* an ALK+ NSCLC. As used herein, the term "ALK+ non-small cell lung cancer" or "ALK+ NSCLC" refers to an NSCLC that has an activated (*e.g*., constitutively activated) anaplastic lymphoma kinase activity or has a rearrangement or translocation of an Anaplastic Lymphoma Kinase (ALK) gene. Typically, compared with the general NSCLC population, patients with ALK+ NSCLC are generally younger, have light (*e.g.,* < 10 pack years) or no smoking history, present with lower Eastern Cooperative Oncology Group performance status, or may have more aggressive disease and, therefore, experience earlier disease progression (Shaw et al. J Clin Oncol. 2009; 27(26):4247-4253; Sasaki et al. Eur J Cancer. 2010; 46(10):1773-1780; Shaw et al. N Engl J Med. 2013;368(25):2385-2394; Socinski et al. J Clin Oncol. 2012; 30(17):2055-2062 ; Yang et al. J Thorac Oncol. 2012;7(1):90-97).

In one embodiment, the cancer, *e.g.,* an NSCLC, has a rearrangement or translocation of an ALK gene. In one embodiment, the rearrangement or translocation of the ALK gene leads to a fusion (*e.g*., fusion upstream of the ALK promoter region). In certain embodiments, the fusion results in constitutive activation of the kinase activity.

In one embodiment, the fusion is an EML4-ALK fusion. Exemplary EML4-ALK fusion proteins include, but are not limited to, E13;A20 (V1), E20;A20 (V2), E6a/b;A20 (V3a/b), E14;A20 (V4), E2a/b;A20 (V5a/b), E13b;A20 (V6), E14;A20(V7), E15;A20("V4"), or E18;A20 (V5) (Choi et al. Cancer Res. 2008; 68(13):4971-6; Horn et al. J Clin Oncol. 2009; 27(26):4232-5; Koivunen et al. Clin Cancer Res. 2008; 14(13):4275-83; Soda et al. Nature. 2007; 448(7153):561-6; Takeuchi et al. Clin Cancer Res. 2008; 14(20):6618-24; Takeuchi et al. Clin Cancer Res. 2009; 15(9):3143-9; Wong et al. Cancer. 2009 Apr 15;115(8):1723-33).

In certain embodiments, the ALK gene is fused to a non-EML4 partner. In one embodiment, the fusion is a KIF5B-ALK fusion. In another embodiment, the fusion is a TFG-ALK fusion. Exemplary KIF5B-ALK and TFG-ALK fusions are described, *e.g.,* in Takeuchi et al. Clin Cancer Res. 2009; 15(9):3143-9, Rikova et al. Cell. 2007; 131(6):1190-203.

ALK gene rearrangements or translocations, or cancer cells that has an ALK gene rearrangement or translocation, can be detected, *e.g.,* using fluorescence *in situ* hybridization (FISH), *e.g*., with an ALK break apart probe.

Methods and compositions disclosed herein are useful for treating metastatic lesions associated with the aforementioned cancers

In other embodiments, the subject is a mammal, *e.g*., a primate, preferably a higher primate, *e.g.,* a human (*e.g.,* a patient having, or at risk of having, a disorder described herein). In one embodiment, the subject is in need of enhancing an immune response. In one embodiment, the subject has, or is at risk of, having a disorder described herein, *e.g.,* a cancer as described herein. In certain embodiments, the subject is, or is at risk of being, immunocompromised. For example, the subject is undergoing or has undergone a chemotherapeutic treatment and/or radiation therapy. Alternatively, or in combination, the subject is, or is at risk of being, immunocompromised as a result of an infection.

In one embodiment, the subject (*e.g.,* a subject having a lung cancer (*e.g.,* a non-small cell lung cancer), a lymphoma (*e.g*., an anaplastic large-cell lymphoma or non-Hodgkin lymphoma), an inflammatory myofibroblastic tumor, or a neuroblastoma) is being treated, or has been treated, with another ALK inhibitor and/or a ROS1 inhibitor, *e.g*., crizotinib. For example, crizotinib can be administered at a daily oral dose of 750 mg or lower, *e.g.,* 600 mg or lower, *e.g.,* 450 mg or lower.

In another embodiment, the subject or cancer (*e.g.,* a lung cancer (*e.g.,* a non-small cell lung cancer), a lymphoma (*e.g*., an anaplastic large-cell lymphoma or non-Hodgkin lymphoma), an inflammatory myofibroblastic tumor, or a neuroblastoma) has progressed on, or is resistant or tolerant to, another ALK inhibitor and/or a ROS1 inhibitor, *e.g*., crizotinib.

In yet another embodiment, the subject or cancer (*e.g.,* a lung cancer (*e.g.,* a non-small cell lung cancer), a lymphoma (*e.g*., an anaplastic large-cell lymphoma or non-Hodgkin lymphoma), an inflammatory myofibroblastic tumor, or a neuroblastoma) is at risk of progression on, or developing resistance or tolerance to, another ALK inhibitor and/or a ROS1 inhibitor, *e.g*., crizotinib.

In other embodiments, the subject or cancer is resistant or tolerant, or is at risk of developing resistance or tolerance, to a tyrosine kinase inhibitor (TKI), *e.g.,* an EGFR tyrosine kinase inhibitor.

In some embodiments, the subject or cancer has no detectable EGFR mutation, KRAS mutation, or both.

In some embodiments, the subject has previously been treated with PD-1.

In some embodiments, the subject has or is identified as having a tumor that has one or more of high PD-L1 level or expression and/or Tumor Infiltrating Lymphocyte (TIL)+. In certain embodiments, the subject has or is identified as having a tumor that has high PD-L1 level or expression and TIL+. The methods described herein further describe identifying a subject based on having a tumor that has one or more of high PD-L1 level or expression and/or TIL+. The methods described herein further describe identifying a subject based on having a tumor that has high PD-L1 level or expression and TIL+. In some embodiments, tumors that are TIL+ are positive for CD8 and IFNγ. In some embodiments, the subject has or is identified as having a high percentage of cells that are positive for one or more of PD-L1, CD8, and/or IFNγ. In certain embodiments, the subject has or is identified as having a high percentage of cells that are positive for all of PD-L1, CD8, and IFNγ.

The methods described herein further describe identifying a subject based on having a high percentage of cells that are positive for one or more of PD-L1, CD8, and/or IFNγ. The methods described herein further describe identifying a subject based on having a high percentage of cells that are positive for all of PD-L1, CD8, and IFNγ. In some embodiments, the subject has or is identified as having one or more of PD-L1, CD8, and/or IFNγ, and one or more of a lung cancer, *e.g*., squamous cell lung cancer or lung adenocarcinoma; a head and neck cancer; a squamous cell cervical cancer; a stomach cancer; a thyroid cancer; and/or a melanoma. The methods described herein further describe identifying a subject based on having one or more of PD-L1, CD8, and/or IFNγ, and one or more of a lung cancer, *e.g*., squamous cell lung cancer or lung adenocarcinoma; a head and neck cancer; a squamous cell cervical cancer; a stomach cancer; a thyroid cancer; and/or a melanoma.

### Dosages and Administration

Dosages and therapeutic regimens of the agents described herein can be determined by a skilled artisan.

In certain embodiments, the anti-PD-1 antibody molecule is administered by injection (*e.g.,* subcutaneously or intravenously) at a dose of about 1 to 30 mg/kg, *e.g.,* about 5 to 25 mg/kg, about 10 to 20 mg/kg, about 1 to 5 mg/kg, or about 3 mg/kg. The dosing schedule can vary from *e.g.,* once a week to once every 2, 3, or 4 weeks. In one embodiment, the anti-PD-1 antibody molecule is administered at a dose from about 10 to 20 mg/kg every other week.

In one embodiment, the anti-PD-1 antibody molecule, *e.g*., Nivolumab, is administered intravenously at a dose from about 1 mg/kg to 3 mg/kg, *e.g*., about 1 mg/kg, 2 mg/kg or 3 mg/kg, every two weeks. In one embodiment, the anti-PD-1 antibody molecule, *e.g*., Nivolumab, is administered intravenously at a dose of about 2 mg/kg at 3-week intervals. In one embodiment, Nivolumab is administered in an amount from about 1 mg/kg to 5 mg/kg, *e.g.,* 3 mg/kg, and may be administered over a period of 60 minutes, ca. once a week to once every 2, 3 or 4 weeks.

The combination therapies described herein can be administered to the subject systemically (*e.g*., orally, parenterally, subcutaneously, intravenously, rectally, intramuscularly, intraperitoneally, intranasally, transdermally, or by inhalation or intracavitary installation), topically, or by application to mucous membranes, such as the nose, throat and bronchial tubes.

In one aspect, the anti-PD-1 antibody molecule is administered intravenously. In one embodiment, in a combination therapy, one or more of the agents listed in Table 1, *e.g.,* an IAP inhibitor or LCL161, is administered orally. In one aspect, the anti-PD-1 antibody molecule is administered, *e.g*., intravenously, at least one, two, three, four, five, six, or seven days, *e.g.,* three days, after an agent listed in Table 1, *e.g.,* an IAP inhibitor or LCL161, is administered, *e.g*., orally. In one aspect, the anti-PD-1 antibody molecule is administered, *e.g.,* intravenously, at least one, two, three, four, five, six, or seven days, *e.g.,* three days, before an agent listed in Table 1, *e.g.,* an IAP inhibitor or LCL161, is administered, *e.g*., orally. In yet another aspect, the anti-PD-1 antibody molecule is administered, *e.g*., intravenously, on the same day, as the one or more agents listed in Table 1, *e.g.,* an IAP inhibitor or LCL161, is administered, *e.g*., orally. In one aspect, the administration of the anti-PD-1 antibody molecule and one or more of the agents listed in Table 1, *e.g.,* an IAP inhibitor or LCL161, results in an enhanced reduction of a solid tumor, *e.g*., colon cancer, relative to administration of each of these agents as a monotherapy. In certain aspects, in a combination therapy, the concentration of an agent listed in Table 1, *e.g.,* an IAP inhibitor or LCL161, that is required to achieve inhibition, *e.g*., growth inhibition, is lower than the therapeutic dose of the agent as a monotherapy, *e.g*., 10-20%, 20-30%, 30-40%, 40-50%, 50-60%, 60-70%, 70-80%, or 80-90% lower. In other embodiments, in a combination therapy, the concentration of the anti-PD-1 antibody molecule that is required to achieve inhibition, *e.g*., growth inhibition, is lower than the therapeutic dose of the anti-PD-1 antibody molecule as a monotherapy, *e.g*., 10-20%, 20-30%, 30-40%, 40-50%, 50-60%, 60-70%, 70-80%, or 80-90% lower.

The methods and compositions described herein can be used in combination with further agents or therapeutic modalities. The combination therapies can be administered simultaneously or sequentially in any order. Any combination and sequence of the anti-PD-1 antibody molecules and other therapeutic agents, procedures or modalities (*e.g.*, as described herein) can be used. The combination therapies can be administered during periods of active disorder, or during a period of remission or less active disease. The combination therapies can be administered before the other treatment, concurrently with the treatment, post-treatment, or during remission of the disorder.

In certain embodiments, the methods and compositions described herein are administered in combination with one or more of other antibody molecules, chemotherapy, other anti-cancer therapy (*e.g*., targeted anti-cancer therapies, gene therapy, viral therapy, RNA therapy bone marrow transplantation, nanotherapy, or oncolytic drugs), cytotoxic agents, immune-based therapies (*e.g.,* cytokines or cell-based immune therapies), surgical procedures *(e.g.,* lumpectomy or mastectomy) or radiation procedures, or a combination of any of the foregoing. The additional therapy may be in the form of adjuvant or neoadjuvant therapy. In some embodiments, the additional therapy is an enzymatic inhibitor (*e.g*., a small molecule enzymatic inhibitor) or a metastatic inhibitor. Exemplary cytotoxic agents that can be administered in combination with include antimicrotubule agents, topoisomerase inhibitors, anti-metabolites, mitotic inhibitors, alkylating agents, anthracyclines, vinca alkaloids, intercalating agents, agents capable of interfering with a signal transduction pathway, agents that promote apoptosis, proteosome inhibitors, and radiation (*e.g.,* local or whole body irradiation (*e.g.,* gamma irradiation). In other embodiments, the additional therapy is surgery or radiation, or a combination thereof. In other embodiments, the additional therapy is a therapy targeting an mTOR pathway, an HSP90 inhibitor, or a tubulin inhibitor.

Alternatively, or in combination with the aforesaid combinations, the methods and compositions described herein can be administered in combination with one or more of: a vaccine, *e.g*., a therapeutic cancer vaccine; or other forms of cellular immunotherapy.

In another embodiment, the combination therapy is used in combination with one, two or all of oxaliplatin, leucovorin or 5-FU (*e.g.,* a FOLFOX co-treatment). Alternatively or in combination, combination further includes a VEGF inhibitor (*e.g*., a VEGF inhibitor as disclosed herein). In some embodiments, the cancer treated with the combination is chosen from a melanoma, a colorectal cancer, a non-small cell lung cancer, an ovarian cancer, a breast cancer, a prostate cancer, a pancreatic cancer, a hematological malignancy or a renal cell carcinoma. The cancer may be at an early, intermediate or late stage.

In other embodiments, the combination therapy is administered with a tyrosine kinase inhibitor (*e.g*., axitinib) to treat renal cell carcinoma and other solid tumors.

In other embodiments, the combination therapy is administered with a 4-1BB receptor targeting agent (*e.g.,* an antibody that stimulates signaling through 4-1BB (CD-137), *e.g.,* PF-2566). In one embodiment, the combination therapy is administered in combination with a tyrosine kinase inhibitor (*e.g*., axitinib) and a 4-1BB receptor targeting agent.

Other features, objects, and advantages of the invention will be apparent from the description and drawings, and from the claims.

### BRIEF DESCRIPTION OF THE DRAWINGS

**Figure 1** shows a graphical representation of flow cytometry of PD-L1 surface expression in EBC-1 cells *in vitro* with or without INC280 treatment. EBC-1 cells are non-small cell lung cancer cells with a cMET amplification.
**Figure 2** shows a graphical representation of PD-L1 mRNA expression in Hs.746.T cells in a tumor xenograft model with or without INC280 treatment. Hs.746.T cells are gastric cancer cells with a c-MET amplification and a c-MET mutation.
**Figure 3** shows a graphical representation of PD-L1 mRNA expression in H3122 cells *in vitro* with or without LDK378. H3122 cells are non-small cell lung cancer (NSCLC) cells with an ALK translocation.
**Figure 4** shows a graphical representation of PD-L1 mRNA expression in LOXIMV1 cells (BRAF mutant melanoma cells) in a tumor xenograft model with or without LGX818 treatment.
**Figure 5** shows a graphical representation of PD-L1 mRNA expression in HEYA8 cells (KRAS mutant ovarian cancer cells) in a tumor xenograft model with or without MEK162 treatment.

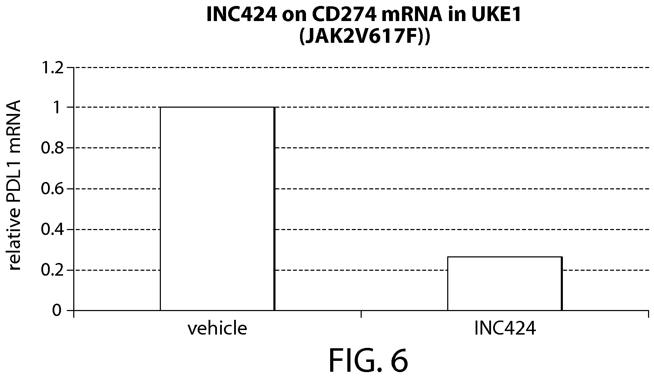

**Figure 6** shows a graphical representation of PD-L1 mRNA expression in UKE-1 cells (JAK2 V617F mutant myeloproliferative neoplasm cells) in a tumor xenograft model with or without INC424 treatment.
**Figure 7A** shows a graphical representation of IFN-γ production in unstimulated PBMCs or stimulated PBMCs treated with different concentrations of LCL161 or DMSO control.
**Figure 7B** shows a graphical representation of IL-10 production in unstimulated PBMCs or stimulated PBMCs treated with different concentrations of LCL161 or DMSO control.
**Figure 8A** shows a graphical representation of FACS analysis of CD4+ T cells from unstimulated PBMCs or PMBCs stimulated in the presence of different concentrations of LCL161 or DMSO control.
**Figure 8B** shows a graphical representation of FACS analysis of CD8+ T cells from unstimulated PBMCs or PMBCs stimulated in the presence of different concentrations of LCL161 or DMSO control.
**Figure 9** shows a graphical representation of CyTOF mass cytometry of unstimulated PBMCs or stimulated PBMCs treated with LCL161 or DMSO control.
**Figure 10A** shows a graphical representation of expression signatures related to T cells from mice implanted with MC38 cells. The mice were treated with LCL161, anti-mouse PD-1, or both. In the control group, mice were dosed with vehicle and isotype (mIgG1).
**Figure 10B** shows a graphical representation of expression signatures related to dendritic cells from mice implanted with MC38 cells. The mice were treated with LCL161, anti-mouse PD-1, or both. In the control group, mice were dosed with vehicle and isotype (mIgG1).
**Figure 10C** shows a graphical representation of expression signatures related to macrophages from mice implanted with MC38 cells. The mice were treated with LCL161, anti-mouse PD-1, or both. In the control group, mice were dosed with vehicle and isotype (mIgG1).
**Figure 10D** shows a graphical representation of chemokine expression signatures from mice implanted with MC38 cells. The mice were treated with LCL161, anti-mouse PD-1, or both. In the control group, mice were dosed with vehicle and isotype (mIgG1).
**Figure 11A** shows an exemplary treatment schedule and a graphical representation of tumor volumes in mice implanted with MC38 cells. The mice were treated with LCL161, anti-mouse PD-1, or both. In this treatment schedule, anti-mouse PD-1 was administered three days after LCL161 was administered. In the control group, mice were dosed with vehicle and isotype (mIgG1).
**Figure 11B** shows another exemplary treatment schedule and a graphical representation of tumor volumes in mice implanted with MC38 cells. The mice were treated with LCL161, anti-mouse PD-1, or both. In this treatment schedule, LCL161 and anti-mouse PD-1 were administered concurrently. In the control group, mice were dosed with vehicle and isotype (mIgG1).
**Figure 12** is a representation of the sequence of drug administration for patients enrolled in the Phase II trial that will be treated with EGF816 and Nivolumab.

### BRIEF DESCRIPTION OF THE TABLE

**Table 1** is a summary of selected therapeutic agents that can be administered in combination with the immunomodulators (*e.g*., one or more of: an activator of a costimulatory molecule and/or an inhibitor of an immune checkpoint molecule) described herein. **Table 1** provides from left to right the following: the Name and/or Designation of the second therapeutic agent, the Compound structure, a Patent publication disclosing the Compound, Exemplary Indications/Uses, and Generic structure.
**Table 2** shows the trial objectives and related endpoints in a phase II, multicenter, open-label study of EGF816 in combination with nivolumab in adult patients with EGFR mutated non-small cell lung cancer.
**Table 3** shows the dose and treatment schedule in a phase II, multicenter, open-label study of EGF816 in combination with nivolumab in adult patients with EGFR mutated non-small cell lung cancer.

### DETAILED DESCRIPTION

The invention is set out in the claims and provides combination therapies and compositions comprising an anti-PD-1 antibody chosen from Nivolumab, Pembrolizumab or Pidilizumab, and LCL161, wherein LCL161 is (S)-N-((S)-1-cyclohexyl-2-((S)-2-(4-(4-fluorobenzoyl)thiazol-2-yl)pyrrolidin-1-yl)-2-oxoethyl)-2-(methylamino)propanamide or a pharmaceutically acceptable salt thereof.

Methods and compositions are disclosed, which comprise an immunomodulator (*e.g*., one or more of: an activator of a costimulatory molecule and/or an inhibitor of an immune checkpoint molecule) in combination with a second therapeutic agent chosen from one or more of the agents listed in Table 1. Immune therapy alone can be effective in a number of indications (*e.g*., melanoma). However, for most patients, it is not a cure. In one aspect, an inhibitor of an immune checkpoint molecule (*e.g*., one or more of inhibitors to PD-1, PD-L1, LAG-3, TIM-3, CEACAM (*e.g*., CEACAM-1, -3 and/or -5) or CTLA-4) can be combined with a second therapeutic agent chosen from one or more of the agents listed in Table 1 *(e.g.,* chosen from one or more of: 1) an IAP inhibitor; 2) a TOR kinase inhibitor; 3) a HDM2 ligase inhibitor; 4) a PIM kinase inhibitor; 5) a HER3 kinase inhibitor; 6) a Histone Deacetylase (HDAC) inhibitor; 7) a Janus kinase inhibitor; 8) an FGF receptor inhibitor; 9) an EGF receptor inhibitor; 10) a c-MET inhibitor; 11) an ALK inhibitor; 12) a CDK4/6-inhibitor; 13) a PI3K inhibitor; 14) a BRAF inhibitor; 15) a CAR T cell (*e.g.,* a CAR T cell targeting CD19); 16) a MEK inhibitor; or 17) a BCR-ABL inhibitor),. The combinations described herein can provide a beneficial effect, *e.g.,* in the treatment of a cancer, such as an enhanced anti-cancer effect, reduced toxicity and/or reduced side effects. For example, the immunomodulator, the second therapeutic agent, or both, can be administered at a lower dosage than would be required to achieve the same therapeutic effect compared to a monotherapy dose.

The term "inhibition" or "inhibitor" includes a reduction in a certain parameter, *e.g*., an activity, of a given molecule, *e.g*., an immune checkpoint inhibitor. For example, inhibition of an activity, *e.g.,* an activity of, *e.g.,* PD-1, PD-L1, c-MET, ALK, CDK4/6, PI3K, BRAF, FGFR, MET or BCR-ABL, of at least 5%, 10%, 20%, 30%, 40% or more is included by this term. Thus, inhibition need not be 100%.

The term "Programmed Death 1" or "PD-1" include isoforms, mammalian, *e.g*., human PD-1, species homologs of human PD-1, and analogs comprising at least one common epitope with PD-1. The amino acid sequence of PD-1, *e.g.,* human PD-1, is known in the art, *e.g.,* Shinohara T et al. (1994) Genomics 23(3):704-6; Finger LR, et al. Gene (1997) 197(1-2):177-87.

The term or "PD-Ligand 1" or "PD-L1" include isoforms, mammalian, *e.g*., human PD-1, species homologs of human PD-L1, and analogs comprising at least one common epitope with PD-L1. The amino acid sequence of PD-L1, *e.g*., human PD-L1, is known in the art

The term "Lymphocyte Activation Gene-3" or "LAG-3" include all isoforms, mammalian, *e.g*., human LAG-3, species homologs of human LAG-3, and analogs comprising at least one common epitope with LAG-3. The amino acid and nucleotide sequences of LAG-3, *e.g.,* human LAG-3, is known in the art, *e.g.,* Triebel et al. (1990) J. Exp. Med. 171:1393-1405.

As used herein, "TIM-3" refers to a transmembrane receptor protein that is expressed on Th1 (T helper 1) cells. TIM-3 has a role in regulating immunity and tolerance *in vivo* (see Hastings et al., Eur J Immunol. 2009 Sep;39(9):2492-501).

The term "Carcinoembryonic Antigen-related Cell Adhesion Molecule" or "CEACAM" includes all family members (*e.g*., CEACAM-1, CEACAM-3, or CEACAM-5), isoforms, mammalian, *e.g*., human CEACAM, species homologs of human CEACAM, and analogs comprising at least one common epitope with CEACAM. The amino acid sequence of CEACAM, *e.g.,* human CEACAM, is known in the art, *e.g.,* Hinoda et al. (1988) Proc. Natl. Acad. Sci. U.S.A. 85 (18), 6959-6963; Zimmermann W. et al. (1987) Proc. Natl. Acad. Sci. U.S.A. 84 (9), 2960-2964; Thompson J. et al. (1989) Biochem. Biophys. Res. Commun. 158 (3), 996-1004.

Additional terms are defined below and throughout the application.

As used herein, the articles "a" and "an" refer to one or to more than one (*e.g.,* to at least one) of the grammatical object of the article.

The term "or" is used herein to mean, and is used interchangeably with, the term "and/or", unless context clearly indicates otherwise.

"About" and "approximately" shall generally mean an acceptable degree of error for the quantity measured given the nature or precision of the measurements. Exemplary degrees of error are within 20 percent (%), typically, within 10%, and more typically, within 5% of a given value or range of values.

The compositions and methods of the present disclosure encompass polypeptides and nucleic acids having the sequences specified, or sequences substantially identical or similar thereto, *e.g*., sequences at least 85%, 90%, 95% identical or higher to the sequence specified. In the context of an amino acid sequence, the term "substantially identical" is used herein to refer to a first amino acid that contains a sufficient or minimum number of amino acid residues that are i) identical to, or ii) conservative substitutions of aligned amino acid residues in a second amino acid sequence such that the first and second amino acid sequences can have a common structural domain and/or common functional activity. For example, amino acid sequences that contain a common structural domain having at least about 85%, 90%. 91%, 92%, 93%, 94%, 95%, 96%, 97%, 98% or 99% identity to a reference sequence, *e.g.,* a sequence provided herein.

In the context of nucleotide sequence, the term "substantially identical" is used herein to refer to a first nucleic acid sequence that contains a sufficient or minimum number of nucleotides that are identical to aligned nucleotides in a second nucleic acid sequence such that the first and second nucleotide sequences encode a polypeptide having common functional activity, or encode a common structural polypeptide domain or a common functional polypeptide activity. For example, nucleotide sequences having at least about 85%, 90%, 91%, 92%, 93%, 94%, 95%, 96%, 97%, 98% or 99% identity to a reference sequence, *e.g.,* a sequence provided herein.

The term "functional variant" refers polypeptides that have a substantially identical amino acid sequence to the naturally-occurring sequence, or are encoded by a substantially identical nucleotide sequence, and are capable of having one or more activities of the naturally-occurring sequence.

Calculations of homology or sequence identity between sequences (the terms are used interchangeably herein) are performed as follows.

To determine the percent identity of two amino acid sequences, or of two nucleic acid sequences, the sequences are aligned for optimal comparison purposes (*e.g*., gaps can be introduced in one or both of a first and a second amino acid or nucleic acid sequence for optimal alignment and non-homologous sequences can be disregarded for comparison purposes). In a preferred embodiment, the length of a reference sequence aligned for comparison purposes is at least 30%, preferably at least 40%, more preferably at least 50%, 60%, and even more preferably at least 70%, 80%, 90%, 100% of the length of the reference sequence. The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein amino acid or nucleic acid "identity" is equivalent to amino acid or nucleic acid "homology").

The percent identity between the two sequences is a function of the number of identical positions shared by the sequences, taking into account the number of gaps, and the length of each gap, which need to be introduced for optimal alignment of the two sequences.

The comparison of sequences and determination of percent identity between two sequences can be accomplished using a mathematical algorithm. In a preferred embodiment, the percent identity between two amino acid sequences is determined using the Needleman and Wunsch ((1970) J. Mol. Biol. 48:444-453) algorithm which has been incorporated into the GAP program in the GCG software package (available at www.gcg.com), using either a Blossum 62 matrix or a PAM250 matrix, and a gap weight of 16, 14, 12, 10, 8, 6, or 4 and a length weight of 1, 2, 3, 4, 5, or 6. In yet another preferred embodiment, the percent identity between two nucleotide sequences is determined using the GAP program in the GCG software package (available at http://www.gcg.com), using a NWSgapdna.CMP matrix and a gap weight of 40, 50, 60, 70, or 80 and a length weight of 1, 2, 3, 4, 5, or 6. A particularly preferred set of parameters (and the one that should be used unless otherwise specified) are a Blossum 62 scoring matrix with a gap penalty of 12, a gap extend penalty of 4, and a frameshift gap penalty of 5.

The percent identity between two amino acid or nucleotide sequences can be determined using the algorithm of E. Meyers and W. Miller ((1989) CABIOS, 4:11-17) which has been incorporated into the ALIGN program (version 2.0), using a PAM120 weight residue table, a gap length penalty of 12 and a gap penalty of 4.

The nucleic acid and protein sequences described herein can be used as a "query sequence" to perform a search against public databases to, for example, identify other family members or related sequences. Such searches can be performed using the NBLAST and XBLAST programs (version 2.0) of Altschul, et al. (1990) J. Mol. Biol. 215:403-10. BLAST nucleotide searches can be performed with the NBLAST program, score = 100, wordlength = 12 to obtain nucleotide sequences homologous to a nucleic acid molecules of the disclosure. BLAST protein searches can be performed with the XBLAST program, score = 50, wordlength = 3 to obtain amino acid sequences homologous to protein molecules of the disclosure. To obtain gapped alignments for comparison purposes, Gapped BLAST can be utilized as described in Altschul et al., (1997) Nucleic Acids Res. 25:3389-3402. When utilizing BLAST and Gapped BLAST programs, the default parameters of the respective programs (*e.g*., XBLAST and NBLAST) can be used. See http://www.ncbi.nlm.nih.gov.

As used herein, the term "hybridizes under low stringency, medium stringency, high stringency, or very high stringency conditions" describes conditions for hybridization and washing. Guidance for performing hybridization reactions can be found in Current Protocols in Molecular Biology, John Wiley & Sons, N.Y. (1989), 6.3.1-6.3.6. Aqueous and nonaqueous methods are described in that reference and either can be used. Specific hybridization conditions referred to herein are as follows: 1) low stringency hybridization conditions in 6X sodium chloride/sodium citrate (SSC) at about 45°C, followed by two washes in 0.2X SSC, 0.1% SDS at least at 50°C (the temperature of the washes can be increased to 55°C for low stringency conditions); 2) medium stringency hybridization conditions in 6X SSC at about 45°C, followed by one or more washes in 0.2X SSC, 0.1% SDS at 60°C; 3) high stringency hybridization conditions in 6X SSC at about 45°C, followed by one or more washes in 0.2X SSC, 0.1% SDS at 65°C; and preferably 4) very high stringency hybridization conditions are 0.5M sodium phosphate, 7% SDS at 65°C, followed by one or more washes at 0.2X SSC, 1% SDS at 65°C. Very high stringency conditions (4) are the preferred conditions and the ones that should be used unless otherwise specified.

It is understood that the molecules of the present disclosure may have additional conservative or non-essential amino acid substitutions, which do not have a substantial effect on their functions.

The term "amino acid" is intended to embrace all molecules, whether natural or synthetic, which include both an amino functionality and an acid functionality and capable of being included in a polymer of naturally-occurring amino acids. Exemplary amino acids include naturally-occurring amino acids; analogs, derivatives and congeners thereof; amino acid analogs having variant side chains; and all stereoisomers of any of any of the foregoing. As used herein the term "amino acid" includes both the D- or L- optical isomers and peptidomimetics.

A "conservative amino acid substitution" is one in which the amino acid residue is replaced with an amino acid residue having a similar side chain. Families of amino acid residues having similar side chains have been defined in the art. These families include amino acids with basic side chains (*e.g.,* lysine, arginine, histidine), acidic side chains (*e.g.,* aspartic acid, glutamic acid), uncharged polar side chains (*e.g*., glycine, asparagine, glutamine, serine, threonine, tyrosine, cysteine), nonpolar side chains (*e.g*., alanine, valine, leucine, isoleucine, proline, phenylalanine, methionine, tryptophan), beta-branched side chains (*e.g*., threonine, valine, isoleucine) and aromatic side chains (*e.g.*, tyrosine, phenylalanine, tryptophan, histidine).

The terms "polypeptide", "peptide" and "protein" (if single chain) are used interchangeably herein to refer to polymers of amino acids of any length. The polymer may be linear or branched, it may comprise modified amino acids, and it may be interrupted by non-amino acids. The terms also encompass an amino acid polymer that has been modified; for example, disulfide bond formation, glycosylation, lipidation, acetylation, phosphorylation, or any other manipulation, such as conjugation with a labeling component. The polypeptide can be isolated from natural sources, can be a produced by recombinant techniques from a eukaryotic or prokaryotic host, or can be a product of synthetic procedures.

The terms "nucleic acid," "nucleic acid sequence," "nucleotide sequence," or "polynucleotide sequence," and "polynucleotide" are used interchangeably. They refer to a polymeric form of nucleotides of any length, either deoxyribonucleotides or ribonucleotides, or analogs thereof. The polynucleotide may be either single-stranded or double-stranded, and if single-stranded may be the coding strand or non-coding (antisense) strand. A polynucleotide may comprise modified nucleotides, such as methylated nucleotides and nucleotide analogs. The sequence of nucleotides may be interrupted by non-nucleotide components. A polynucleotide may be further modified after polymerization, such as by conjugation with a labeling component. The nucleic acid may be a recombinant polynucleotide, or a polynucleotide of genomic, cDNA, semisynthetic, or synthetic origin which either does not occur in nature or is linked to another polynucleotide in a nonnatural arrangement.

The term "isolated," as used herein, refers to material that is removed from its original or native environment (*e.g*., the natural environment if it is naturally occurring). For example, a naturally-occurring polynucleotide or polypeptide present in a living animal is not isolated, but the same polynucleotide or polypeptide, separated by human intervention from some or all of the co-existing materials in the natural system, is isolated. Such polynucleotides could be part of a vector and/or such polynucleotides or polypeptides could be part of a composition, and still be isolated in that such vector or composition is not part of the environment in which it is found in nature.

Various aspects of the disclosure are described in further detail below. Additional definitions are set out throughout the specification.

### Antibody Molecules

In one aspect of the disclosure, the antibody molecule binds to a mammalian, *e.g*., human, checkpoint molecule, *e.g.,* PD-1, PD-L1, LAG-3, CEACAM (*e.g.,* CEACAM-1, -3 and/or -5) or TIM-3. For example, the antibody molecule binds specifically to an epitope, *e.g.,* linear or conformational epitope, (*e.g.,* an epitope as described herein) on PD-1, PD-L1, LAG-3, CEACAM (*e.g*., CEACAM-1, -3 and/or-5) or TIM-3.

As used herein, the term "antibody molecule" refers to a protein comprising at least one immunoglobulin variable domain sequence. The term antibody molecule includes, for example, full-length, mature antibodies and antigen-binding fragments of an antibody. For example, an antibody molecule can include a heavy (H) chain variable domain sequence (abbreviated herein as VH), and a light (L) chain variable domain sequence (abbreviated herein as VL). In another example, an antibody molecule includes two heavy (H) chain variable domain sequences and two light (L) chain variable domain sequence, thereby forming two antigen binding sites, such as Fab, Fab', F(ab')₂, Fc, Fd, Fd', Fv, single chain antibodies (scFv for example), single variable domain antibodies, diabodies (Dab) (bivalent and bispecific), and chimeric (*e.g*., humanized) antibodies, which may be produced by the modification of whole antibodies or those synthesized de novo using recombinant DNA technologies. These functional antibody fragments retain the ability to selectively bind with their respective antigen or receptor. Antibodies and antibody fragments can be from any class of antibodies including, but not limited to, IgG, IgA, IgM, IgD, and IgE, and from any subclass (*e.g*., IgG1, IgG2, IgG3, and IgG4) of antibodies. The antibodies of the present disclosure can be monoclonal or polyclonal. The antibody can also be a human, humanized, CDR-grafted, or *in vitro* generated antibody. The antibody can have a heavy chain constant region chosen from, *e.g.,* IgG1, IgG2, IgG3, or IgG4. The antibody can also have a light chain chosen from, *e.g*., kappa or lambda.

Examples of antigen-binding fragments include: (i) a Fab fragment, a monovalent fragment consisting of the VL, VH, CL and CH1 domains; (ii) a F(ab')2 fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a diabody (dAb) fragment, which consists of a VH domain; (vi) a camelid or camelized variable domain; (vii) a single chain Fv (scFv), *see e.g.,* Bird et al. (1988) Science 242:423-426; and Huston et al. (1988) Proc. Natl. Acad. Sci. USA 85:5879-5883); (viii) a single domain antibody. These antibody fragments are obtained using conventional techniques known to those with skill in the art, and the fragments are screened for utility in the same manner as are intact antibodies.

The term "antibody" includes intact molecules as well as functional fragments thereof. Constant regions of the antibodies can be altered, *e.g*., mutated, to modify the properties of the antibody (*e.g*., to increase or decrease one or more of: Fc receptor binding, antibody glycosylation, the number of cysteine residues, effector cell function, or complement function).

Antibody molecules can also be single domain antibodies. Single domain antibodies can include antibodies whose complementary determining regions are part of a single domain polypeptide. Examples include, but are not limited to, heavy chain antibodies, antibodies naturally devoid of light chains, single domain antibodies derived from conventional 4-chain antibodies, engineered antibodies and single domain scaffolds other than those derived from antibodies. Single domain antibodies may be any of the art, or any future single domain antibodies. Single domain antibodies may be derived from any species including, but not limited to mouse, human, camel, llama, fish, shark, goat, rabbit, and bovine. According to another aspect of the disclosure, a single domain antibody is a naturally occurring single domain antibody known as heavy chain antibody devoid of light chains. Such single domain antibodies are disclosed in WO 9404678, for example. For clarity reasons, this variable domain derived from a heavy chain antibody naturally devoid of light chain is known herein as a VHH or nanobody to distinguish it from the conventional VH of four chain immunoglobulins. Such a VHH molecule can be derived from antibodies raised in *Camelidae* species, for example in camel, llama, dromedary, alpaca and guanaco. Other species besides *Camelidae* may produce heavy chain antibodies naturally devoid of light chain; such VHHs are within the scope of the disclosure.

The VH and VL regions can be subdivided into regions of hypervariability, termed "complementarity determining regions" (CDR), interspersed with regions that are more conserved, termed "framework regions" (FR or FW).

The extent of the framework region and CDRs has been precisely defined by a number of methods (*see,* Kabat, E. A., et al. (1991) Sequences of Proteins of Immunological Interest, Fifth Edition, U.S. Department of Health and Human Services, NIH Publication No. 91-3242; Chothia, C. et al. (1987) J. Mol. Biol. 196:901-917; and the AbM definition used by Oxford Molecular's AbM antibody modeling software. *See*, generally, *e.g.,* Protein Sequence and Structure Analysis of Antibody Variable Domains. In: Antibody Engineering Lab Manual (Ed.: Duebel, S. and Kontermann, R., Springer-Verlag, Heidelberg).

The terms "complementarity determining region," and "CDR," as used herein refer to the sequences of amino acids within antibody variable regions which confer antigen specificity and binding affinity. In general, there are three CDRs in each heavy chain variable region (HCDR1, HCDR2, HCDR3) and three CDRs in each light chain variable region (LCDR1, LCDR2, LCDR3).

The precise amino acid sequence boundaries of a given CDR can be determined using any of a number of well-known schemes, including those described by Kabat et al. (1991), "Sequences of Proteins of Immunological Interest," 5th Ed. Public Health Service, National Institutes of Health, Bethesda, MD ("Kabat" numbering scheme), Al-Lazikani et al., (1997) JMB 273,927-948 ("Chothia" numbering scheme). As used herein, the CDRs defined according the "Chothia" number scheme are also sometimes referred to as "hypervariable loops."

For example, under Kabat, the CDR amino acid residues in the heavy chain variable domain (VH) are numbered 31-35 (HCDR1), 50-65 (HCDR2), and 95-102 (HCDR3); and the CDR amino acid residues in the light chain variable domain (VL) are numbered 24-34 (LCDR1), 50-56 (LCDR2), and 89-97 (LCDR3). Under Chothia the CDR amino acids in the VH are numbered 26-32 (HCDR1), 52-56 (HCDR2), and 95-102 (HCDR3); and the amino acid residues in VL are numbered 26-32 (LCDR1), 50-52 (LCDR2), and 91-96 (LCDR3). By combining the CDR definitions of both Kabat and Chothia, the CDRs consist of amino acid residues 26-35 (HCDR1), 50-65 (HCDR2), and 95-102 (HCDR3) in human VH and amino acid residues 24-34 (LCDR1), 50-56 (LCDR2), and 89-97 (LCDR3) in human VL.

As used herein, an "immunoglobulin variable domain sequence" refers to an amino acid sequence which can form the structure of an immunoglobulin variable domain. For example, the sequence may include all or part of the amino acid sequence of a naturally-occurring variable domain. For example, the sequence may or may not include one, two, or more N- or C-terminal amino acids, or may include other alterations that are compatible with formation of the protein structure.

The term "antigen-binding site" refers to the part of an antibody molecule that comprises determinants that form an interface that binds to the PD-1 polypeptide, or an epitope thereof. With respect to proteins (or protein mimetics), the antigen-binding site typically includes one or more loops (of at least four amino acids or amino acid mimics) that form an interface that binds to the PD-1 polypeptide. Typically, the antigen-binding site of an antibody molecule includes at least one or two CDRs and/or hypervariable loops, or more typically at least three, four, five or six CDRs and/or hypervariable loops.

The terms "monoclonal antibody" or "monoclonal antibody composition" as used herein refer to a preparation of antibody molecules of single molecular composition. A monoclonal antibody composition displays a single binding specificity and affinity for a particular epitope. A monoclonal antibody can be made by hybridoma technology or by methods that do not use hybridoma technology (*e.g*., recombinant methods).

An "effectively human" protein is a protein that does not evoke a neutralizing antibody response, *e.g.,* the human anti-murine antibody (HAMA) response. HAMA can be problematic in a number of circumstances, *e.g.,* if the antibody molecule is administered repeatedly, *e.g.,* in treatment of a chronic or recurrent disease condition. A HAMA response can make repeated antibody administration potentially ineffective because of an increased antibody clearance from the serum (*see, e.g.,* Saleh et al., Cancer Immunol. Immunother., 32:180-190 (1990)) and also because of potential allergic reactions (*see, e.g.,* LoBuglio et al., Hybridoma, 5:5117-5123 (1986)).

An antibody molecule can be a polyclonal or a monoclonal antibody. In other embodiments, the antibody can be recombinantly produced, *e.g*., produced by phage display or by combinatorial methods.

Phage display and combinatorial methods for generating antibodies are known in the art (as described in, *e.g.,* Ladner et al. U.S. Patent No. 5,223,409; Kang et al. International Publication No. WO 92/18619; Dower et al. International Publication No. WO 91/17271; Winter et al. International Publication WO 92/20791; Markland et al. International Publication No. WO 92/15679; Breitling et al. International Publication WO 93/01288; McCafferty et al. International Publication No. WO 92/01047; Garrard et al. International Publication No. WO 92/09690; Ladner et al. International Publication No. WO 90/02809; Fuchs et al. (1991) Bio/Technology 9:1370-1372; Hay et al. (1992) Hum Antibod Hybridomas 3:81-85; Huse et al. (1989) Science 246:1275-1281; Griffths et al. (1993) EMBO J 12:725-734; Hawkins et al. (1992) J Mol Biol 226:889-896; Clackson et al. (1991) Nature 352:624-628; Gram et al. (1992) PNAS 89:3576-3580; Garrad et al. (1991) Bio/Technology 9:1373-1377; Hoogenboom et al. (1991) Nuc Acid Res 19:4133-4137; and Barbas et al. (1991) PNAS 88:7978-7982).

In one embodiment, the antibody is a fully human antibody (e.g., an antibody made in a mouse which has been genetically engineered to produce an antibody from a human immunoglobulin sequence), or a non-human antibody, *e.g*., a rodent (mouse or rat), goat, primate (*e.g*., monkey), camel antibody. Preferably, the non-human antibody is a rodent (mouse or rat antibody). Methods of producing rodent antibodies are known in the art.

Human monoclonal antibodies can be generated using transgenic mice carrying the human immunoglobulin genes rather than the mouse system. Splenocytes from these transgenic mice immunized with the antigen of interest are used to produce hybridomas that secrete human mAbs with specific affinities for epitopes from a human protein (see, *e.g.,* Wood et al. International Application WO 91/00906, Kucherlapati et al. PCT publication WO 91/10741; Lonberg et al. International Application WO 92/03918; Kay et al. International Application 92/03917; Lonberg, N. et al. 1994 Nature 368:856-859; Green, L.L. et al. 1994 Nature Genet. 7:13-21; Morrison, S.L. et al. 1994 Proc. Natl. Acad. Sci. USA 81:6851-6855; Bruggeman et al. 1993 Year Immunol 7:33-40; Tuaillon et al. 1993 PNAS 90:3720-3724; Bruggeman et al. 1991 Eur J Immunol 21:1323-1326).

An antibody can be one in which the variable region, or a portion thereof, *e.g.,* the CDRs, are generated in a non-human organism, *e.g*., a rat or mouse. Chimeric, CDR-grafted, and humanized antibodies are within the disclosure. Antibodies generated in a non-human organism, *e.g.,* a rat or mouse, and then modified, *e.g.,* in the variable framework or constant region, to decrease antigenicity in a human are within the disclosure.

Chimeric antibodies can be produced by recombinant DNA techniques known in the art (see Robinson et al., International Patent Publication PCT/US86/02269; Akira, *et al.,* European Patent Application 184,187; Taniguchi, M., European Patent Application 171,496; Morrison *et al.,* European Patent Application 173,494; Neuberger et al., International Application WO 86/01533; Cabilly et al. U.S. Patent No. 4,816,567; Cabilly *et al.,* European Patent Application 125,023; Better et al. (1988 Science 240:1041-1043); Liu et al. (1987) PNAS 84:3439-3443; Liu et al., 1987, J. Immunol. 139:3521-3526; Sun et al. (1987) PNAS 84:214-218; Nishimura et al., 1987, Canc. Res. 47:999-1005; Wood et al. (1985) Nature 314:446-449; and Shaw et al., 1988, J. Natl Cancer Inst. 80:1553-1559).

A humanized or CDR-grafted antibody will have at least one or two but generally all three recipient CDRs (of heavy and or light immuoglobulin chains) replaced with a donor CDR. The antibody may be replaced with at least a portion of a non-human CDR or only some of the CDRs may be replaced with non-human CDRs. It is only necessary to replace the number of CDRs required for binding of the humanized antibody to PD-1. Preferably, the donor will be a rodent antibody, *e.g*., a rat or mouse antibody, and the recipient will be a human framework or a human consensus framework. Typically, the immunoglobulin providing the CDRs is called the "donor" and the immunoglobulin providing the framework is called the "acceptor." In one embodiment, the donor immunoglobulin is a non-human (*e.g*., rodent). The acceptor framework is a naturally-occurring (*e.g.,* a human) framework or a consensus framework, or a sequence about 85% or higher, preferably 90%, 95%, 99% or higher identical thereto.

As used herein, the term "consensus sequence" refers to the sequence formed from the most frequently occurring amino acids (or nucleotides) in a family of related sequences (See *e.g.,* Winnaker, From Genes to Clones (Verlagsgesellschaft, Weinheim, Germany 1987). In a family of proteins, each position in the consensus sequence is occupied by the amino acid occurring most frequently at that position in the family. If two amino acids occur equally frequently, either can be included in the consensus sequence. A "consensus framework" refers to the framework region in the consensus immunoglobulin sequence.

An antibody can be humanized by methods known in the art (*see e.g.,* Morrison, S. L., 1985, Science 229:1202-1207, by Oi et al., 1986, BioTechniques 4:214, and by Queen et al. US 5,585,089, US 5,693,761 and US 5,693,762).

Humanized or CDR-grafted antibodies can be produced by CDR-grafting or CDR substitution, wherein one, two, or all CDRs of an immunoglobulin chain can be replaced. *See e.g.,* U.S. Patent 5,225,539; Jones et al. 1986 Nature 321:552-525; Verhoeyan et al. 1988 Science 239:1534; Beidler et al. 1988 J. Immunol. 141:4053-4060; Winter US 5,225,539. Winter describes a CDR-grafting method which may be used to prepare the humanized antibodies of the present disclosure (UK Patent Application GB 2188638A, filed on March 26, 1987; Winter US 5,225,539).

Also within the scope of the disclosure are humanized antibodies in which specific amino acids have been substituted, deleted or added. Criteria for selecting amino acids from the donor are described in US 5,585,089, *e.g.,* columns 12-16 of US 5,585,089, *e.g.,* columns 12-16 of US 5,585,089. Other techniques for humanizing antibodies are described in Padlan et al. EP 519596 A1, published on December 23, 1992.

An antibody molecule can be a single chain antibody. A single-chain antibody (scFV) may be engineered (see, for example, Colcher, D. et al. (1999) Ann N Y Acad Sci 880:263-80; and Reiter, Y. (1996) Clin Cancer Res 2:245-52). The single chain antibody can be dimerized or multimerized to generate multivalent antibodies having specificities for different epitopes of the same target protein.

In yet other aspects, the antibody molecule has a heavy chain constant region chosen from, *e.g.,* the heavy chain constant regions of IgG1, IgG2, IgG3, IgG4, IgM, IgA1, IgA2, IgD, and IgE; particularly, chosen from, *e.g.,* the (*e.g.,* human) heavy chain constant regions of IgG1, IgG2, IgG3, and IgG4. In another aspect, the antibody molecule has a light chain constant region chosen from, *e.g*., the (*e.g*., human) light chain constant regions of kappa or lambda. The constant region can be altered, *e.g*., mutated, to modify the properties of the antibody (*e.g*., to increase or decrease one or more of: Fc receptor binding, antibody glycosylation, the number of cysteine residues, effector cell function, and/or complement function). In one embodiment the antibody has: effector function; and can fix complement. In other embodiments the antibody does not; recruit effector cells; or fix complement. In another embodiment, the antibody has reduced or no ability to bind an Fc receptor. For example, it is a isotype or subtype, fragment or other mutant, which does not support binding to an Fc receptor, *e.g.,* it has a mutagenized or deleted Fc receptor binding region.

Methods for altering an antibody constant region are known in the art. Antibodies with altered function, *e.g*. altered affinity for an effector ligand, such as FcR on a cell, or the C1 component of complement can be produced by replacing at least one amino acid residue in the constant portion of the antibody with a different residue (*see e.g.,* EP 388,151 A1, U.S. Pat. No. 5,624,821 and U.S. Pat. No. 5,648,260). Similar type of alterations could be described which if applied to the murine, or other species immunoglobulin would reduce or eliminate these functions.

An antibody molecule can be derivatized or linked to another functional molecule (*e.g*., another peptide or protein). As used herein, a "derivatized" antibody molecule is one that has been modified. Methods of derivatization include but are not limited to the addition of a fluorescent moiety, a radionucleotide, a toxin, an enzyme or an affinity ligand such as biotin. Accordingly, the antibody molecules of the invention are intended to include derivatized and otherwise modified forms of the antibodies described herein, including immunoadhesion molecules. For example, an antibody molecule can be functionally linked (by chemical coupling, genetic fusion, noncovalent association or otherwise) to one or more other molecular entities, such as another antibody (*e.g*., a bispecific antibody or a diabody), a detectable agent, a cytotoxic agent, a pharmaceutical agent, and/or a protein or peptide that can mediate association of the antibody or antibody portion with another molecule (such as a streptavidin core region or a polyhistidine tag).

One type of derivatized antibody molecule is produced by crosslinking two or more antibodies (of the same type or of different types, *e.g.,* to create bispecific antibodies). Suitable crosslinkers include those that are heterobifunctional, having two distinctly reactive groups separated by an appropriate spacer (*e.g*., m-maleimidobenzoyl-N-hydroxysuccinimide ester) or homobifunctional (*e.g*., disuccinimidyl suberate). Such linkers are available from Pierce Chemical Company, Rockford, Ill.

An antibody molecules may be conjugated to another molecular entity, typically a label or a therapeutic (*e.g*., a cytotoxic or cytostatic) agent or moiety. Radioactive isotopes can be used in diagnostic or therapeutic applications. Radioactive isotopes that can be coupled to the anti-PSMA antibodies include, but are not limited to α-, β-, or γ-emitters, or β-and γ-emitters. Such radioactive isotopes include, but are not limited to iodine (¹³¹I or ¹²⁵I), yttrium (⁹⁰Y), lutetium ( ¹⁷⁷Lu), actinium (²²⁵Ac), praseodymium, astatine (²¹¹At), rhenium (¹⁸⁶Re), bismuth (²¹²Bi or ²¹³Bi), indium (¹¹¹In), technetium (⁹⁹mTc), phosphorus (³²P), rhodium (¹⁸⁸Rh), sulfur (³⁵S), carbon (¹⁴C), tritium (³H), chromium (⁵¹Cr), chlorine (³⁶Cl), cobalt (⁵⁷Co or ⁵⁸Co), iron (⁵⁹Fe), selenium (⁷⁵Se), or gallium (⁶⁷Ga). Radioisotopes useful as therapeutic agents include yttrium (⁹⁰Y), lutetium (¹⁷⁷Lu), actinium (²²⁵Ac), praseodymium, astatine (²¹¹At), rhenium (¹⁸⁶Re), bismuth (²¹² Bi or ²¹³Bi), and rhodium (¹⁸⁸Rh). Radioisotopes useful as labels, *e.g.,* for use in diagnostics, include iodine (¹³¹I or ¹²⁵I), indium (¹¹¹In), technetium (⁹⁹mTc), phosphorus ( ³²P), carbon (¹⁴C), and tritium (³H), or one or more of the therapeutic isotopes listed above.

The disclosure provides radiolabeled antibody molecules and methods of labeling the same. A method of labeling an antibody molecule is disclosed. The method includes contacting an antibody molecule, with a chelating agent, to thereby produce a conjugated antibody. The conjugated antibody is radiolabeled with a radioisotope, *e.g*., ¹¹¹Indium, ⁹⁰Yttrium and ¹⁷⁷Lutetium, to thereby produce a labeled antibody molecule.

As is discussed above, the antibody molecule can be conjugated to a therapeutic agent. Therapeutically active radioisotopes have already been mentioned. Examples of other therapeutic agents include taxol, cytochalasin B, gramicidin D, ethidium bromide, emetine, mitomycin, etoposide, tenoposide, vincristine, vinblastine, colchicine, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, puromycin, maytansinoids, *e.g.,* maytansinol (*see* U.S. Pat. No. 5,208,020), CC-1065 (see U.S. Pat. Nos. 5,475,092, 5,585,499, 5,846, 545) and analogs or homologs thereof. Therapeutic agents include, but are not limited to, antimetabolites (*e.g*., methotrexate, 6-mercaptopurine, 6-thioguanine, cytarabine, 5-fluorouracil decarbazine), alkylating agents (*e.g.*, mechlorethamine, thioepa chlorambucil, CC-1065, melphalan, carmustine (BSNU) and lomustine (CCNU), cyclothosphamide, busulfan, dibromomannitol, streptozotocin, mitomycin C, and cis-dichlorodiamine platinum (II) (DDP) cisplatin), anthracyclinies (*e.g*., daunorubicin (formerly daunomycin) and doxorubicin), antibiotics (*e.g*., dactinomycin (formerly actinomycin), bleomycin, mithramycin, and anthramycin (AMC)), and anti-mitotic agents (*e.g*., vincristine, vinblastine, taxol and maytansinoids).

### Combination Therapies

The disclosed combination therapies (*e.g*., methods and compositions described herein) can include an immunomodulator (*e.g*., one or more of: an activator of a costimulatory molecule or an inhibitor of an immune checkpoint molecule) and a second therapeutic agent, *e.g.,* a second therapeutic agent chosen from one or more of the agents listed in Table 1. The invention provides the combination set out in the claims.

By "combination" or "in combination with," it is not intended to imply that the therapy or the therapeutic agents must be administered at the same time and/or formulated for delivery together (*e.g.,* in the same composition), although these methods and compositions are within the scope described herein. The immunomodulator and the second therapeutic agent can be administered concurrently with, prior to, or subsequent to, one or more other additional therapies or therapeutic agents. The agents in the combination can be administered in any order. In general, each agent will be administered at a dose and/or on a time schedule determined for that agent. In will further be appreciated that the additional therapeutic agent utilized in this combination may be administered together in a single composition or administered separately in different compositions. In general, it is expected that additional therapeutic agents utilized in combination be utilized at levels that do not exceed the levels at which they are utilized individually. In some embodiments, the levels utilized in combination will be lower than those utilized individually.

In some embodiments, a combination includes a formulation of the immunomodulator and the second therapeutic agent, with or without instructions for combined use or to combination products. The combined compounds can be manufactured and/or formulated by the same or different manufacturers. The combination partners may thus be entirely separate pharmaceutical dosage forms or pharmaceutical compositions that are also sold independently of each other. In embodiments, instructions for their combined use are provided: (i) prior to release to physicians (*e.g.* in the case of a "kit of part" comprising the compound of the disclosure and the other therapeutic agent); (ii) by the physicians themselves (or under the guidance of a physician) shortly before administration; (iii) the patient themselves by a physician or medical staff.

### Immunomodulators

The combination therapies disclosed herein can include an inhibitor of an inhibitory molecule of an immune checkpoint molecule. The term "immune checkpoints" refers to a group of molecules on the cell surface of CD4 and CD8 T cells. These molecules can effectively serve as "brakes" to down-modulate or inhibit an anti-tumor immune response. Inhibition of an inhibitory molecule can be performed by inhibition at the DNA, RNA or protein level. An inhibitory nucleic acid (*e.g.,* a dsRNA, siRNA or shRNA), can be used to inhibit expression of an inhibitory molecule. In other aspects, the inhibitor of an inhibitory signal is, a polypeptide *e.g.,* a soluble ligand, or an antibody or antigen-binding fragment thereof, that binds to the inhibitory molecule.

Immune checkpoint molecules useful in the methods and compositions of the present disclosure include, but are not limited to, Programmed Death 1 (PD-1), PD-1, PD-L1, PD-L2, Cytotoxic T-Lymphocyte Antigen 4 (CTLA-4), TIM-3, CEACAM (*e.g*., CEACAM-1, CEACAM-3 and/or CEACAM-5), LAG-3, VISTA, BTLA, TIGIT, LAIR1, CD160, 2B4, CD80, CD86, B7-H1, B7-H3 (CD276), B7-H4 (VTCN1), HVEM (TNFRSF14 or CD270), KIR, A2aR, MHC class I, MHC class II, GAL9, adenosine, TGFR (*e.g*., TGFR beta). In certain aspects, the immunomodulator is an inhibitor of an immune checkpoint molecule (*e.g*., an inhibitor of PD-1, PD-L1, LAG-3, TIM-3, CEACAM (*e.g.*, CEACAM-1, -3 and/or -5) or CTLA-4, or any combination thereof).

In other embodiments, the PD-1 inhibitor is an anti-PD-1 antibody chosen from Nivolumab, Pembrolizumab or Pidilizumab.

In some embodiments, the anti-PD-1 antibody is Nivolumab. Alternative names for Nivolumab include MDX-1106, MDX-1106-04, ONO-4538, or BMS-936558. In some embodiments, the anti-PD-1 antibody is Nivolumab (CAS Registry Number: 946414-94-4). Nivolumab is a fully human IgG4 monoclonal antibody which specifically blocks PD-1. Nivolumab (clone 5C4) and other human monoclonal antibodies that specifically bind to PD-1 are disclosed in US 8,008,449 and WO2006/121168. In one embodiment, the inhibitor of PD-1 is Nivolumab, and having a sequence disclosed herein.

The heavy and light chain amino acid sequences of Nivolumab are as follows:
*Heavy chain* (SEQ ID NO: 2)
*Light chain* (SEQ ID NO: 3)

In some embodiments, the anti-PD-1 antibody is Pembrolizumab. Pembrolizumab (also referred to as Lambrolizumab, MK-3475, MK03475, SCH-900475 or KEYTRUDA®; Merck) is a humanized IgG4 monoclonal antibody that binds to PD-1. Pembrolizumab and other humanized anti-PD-1 antibodies are disclosed in Hamid, O. et al. (2013) New England Journal of Medicine 369 (2): 134-44, US 8,354,509 and WO2009/114335. In one embodiment, the inhibitor of PD-1 is Pembrolizumab disclosed in, *e.g.,* US 8,354,509 and WO 2009/114335, and having a sequence disclosed herein.

The heavy and light chain amino acid sequences of Pembrolizumab are as follows:
*Heavy chain* (SEQ ID NO: 4)
*Light chain* (SEQ ID NO: 5)

In some embodiments, the anti-PD-1 antibody is Pidilizumab. Pidilizumab (CT-011; Cure Tech) is a humanized IgGlk monoclonal antibody that binds to PD-1. Pidilizumab and other humanized anti-PD-1 monoclonal antibodies are disclosed in WO2009/101611.

Other anti-PD-1 antibodies include AMP 514 (Amplimmune), among others, *e.g*., anti-PD-1 antibodies disclosed in US 8,609,089, US 2010028330, and/or US 20120114649.

### Exemplary PD-L1 or PD-L2 Inhibitors

In some disclosed aspects, the PD-L1 inhibitor is an antibody molecule. In some aspects, the anti-PD-L1 inhibitor is chosen from YW243.55.S70, MPDL3280A, MEDI-4736, MSB-0010718C, or MDX-1105.

In some disclosed aspects, the anti-PD-L1 antibody is MSB0010718C. MSB0010718C (also referred to as A09-246-2; Merck Serono) is a monoclonal antibody that binds to PD-L1. Pembrolizumab and other humanized anti-PD-Ll antibodies are disclosed in WO2013/079174, and having a sequence disclosed herein (or a sequence substantially identical or similar thereto, *e.g.,* a sequence at least 85%, 90%, 95% identical or higher to the sequence specified). The heavy and light chain amino acid sequences of MSB0010718C include at least the following:
Heavy chain variable region (SEQ ID NO: 24 as disclosed in WO2013/079174) (SEQ ID NO: 6)
*Light chain variable region (SEQ ID NO: 25 as disclosed in* WO2013/079174*)* (SEQ ID NO: 7)

In one disclosed aspects, the PD-L1 inhibitor is YW243.55.S70. The YW243.55.S70 antibody is an anti-PD-Ll described in WO 2010/077634 (heavy and light chain variable region sequences shown in SEQ ID Nos. 20 and 21, respectively, of WO 2010/077634), and having a sequence disclosed therein (or a sequence substantially identical or similar thereto, *e.g.,* a sequence at least 85%, 90%, 95% identical or higher to the sequence specified).

In one disclosed aspect, the PD-L1 inhibitor is MDX-1105. MDX-1105, also known as BMS-936559, is an anti-PD-Ll antibody described in WO2007/005874, and having a sequence disclosed therein (or a sequence substantially identical or similar thereto, *e.g.,* a sequence at least 85%, 90%, 95% identical or higher to the sequence specified).

In one disclosed aspect, the PD-L1 inhibitor is MDPL3280A (Genentech / Roche). MDPL3280A is a human Fc optimized IgG1 monoclonal antibody that binds to PD-L1. MDPL3280A and other human monoclonal antibodies to PD-L1 are disclosed in U.S. Patent No.: 7,943,743 and U.S Publication No.: 20120039906.

In other disclosed aspects, the PD-L2 inhibitor is AMP-224. AMP-224 is a PD-L2 Fc fusion soluble receptor that blocks the interaction between PD-1 and B7-H1 (B7-DCIg; Amplimmune; *e.g*., disclosed in WO2010/027827 and WO2011/066342).

### Exemplary TIM-3 Inhibitors

In one disclosed aspect, a combination described herein includes a TIM-3 inhibitor. In some embodiments, the combination is used to treat a cancer, *e.g.,* a cancer described herein, *e.g.,* a solid tumor or a hematologic malignancy.

Exemplary anti-TIM-3 antibodies are disclosed in U.S. Patent No.: 8,552,156, WO 2011/155607, EP 2581113 and U.S Publication No.: 2014/044728.

### Exemplary LAG-3 Inhibitors

In one disclosed aspect, a combination described herein includes a LAG-3 inhibitor. In some aspects, the combination is used to treat a cancer, *e.g.,* a cancer described herein, *e.g.,* a solid tumor or a hematologic malignancy.

In some disclosed aspects, the anti-LAG-3 antibody is BMS-986016. BMS-986016 (also referred to as BMS986016; Bristol-Myers Squibb) is a monoclonal antibody that binds to LAG-3. BMS-986016 and other humanized anti-LAG-3 antibodies are disclosed in US 2011/0150892, WO2010/019570, and WO2014/008218.

### Exemplary CTLA-4 Inhibitors

In one disclosed aspect, a combination described herein includes a CTLA-4 inhibitor. In some aspects, the combination is used to treat a cancer, *e.g.,* a cancer described herein, *e.g.,* a solid tumor or a hematologic malignancy.

Exemplary disclosed anti-CTLA-4 antibodies include Tremelimumab (IgG2 monoclonal antibody available from Pfizer, formerly known as ticilimumab, CP-675,206); and Ipilimumab (CTLA-4 antibody, also known as MDX-010, CAS No. 477202-00-9).

In one disclosed aspect, the combination includes an anti-PD-1 antibody molecule, *e.g.,* as described herein, and an anti-CTLA-4 antibody, *e.g.,* ipilimumab. Exemplary doses that can be use include a dose of anti-PD-1 antibody molecule of about 1 to 10 mg/kg, *e.g.,* 3 mg/kg, and a dose of an anti-CTLA-4 antibody, *e.g*., ipilimumab, of about 3 mg/kg. In one aspect, the anti-PD-1 antibody molecule is administered after treatment, *e.g.,* after treatment of a melanoma, with an anti-CTLA-4 antibody (*e.g*., ipilimumab) with or without a BRAF inhibitor (*e.g*., vemurafenib or dabrafenib).

Other exemplary disclosed anti-CTLA-4 antibodies are disclosed, *e.g.,* in U.S. Pat. No. 5,811,097.

In one aspect, the inhibitor is a soluble ligand (*e.g.,* a CTLA-4-Ig), or an antibody or antibody fragment that binds to PD-L1, PD-L2 or CTLA-4. For example, the anti-PD-1 antibody molecule can be administered in combination with an anti-CTLA-4 antibody, *e.g.,* ipilimumab, for example, to treat a cancer (*e.g.,* a cancer chosen from: a melanoma, *e.g.,* a metastatic melanoma; a lung cancer, *e.g.,* a non-small cell lung carcinoma; or a prostate cancer).

### Additional Combinations of Inhibitors

In certain embodiments, the anti-PD-1 molecules described herein are administered in combination with one or more other inhibitors of PD-1, PD-L1 and/or PD-L2, *e.g.,* as described herein. The antagonist may be an antibody, an antigen binding fragment thereof, an immunoadhesin, a fusion protein, or oligopeptide.

In one aspect, the anti-PD-1 or PD-L1 antibody molecule is administered in combination with an anti-LAG-3 antibody or an antigen-binding fragment thereof. In another aspect, the anti-PD-1 or PD-L1 antibody molecule is administered in combination with an anti-TIM-3 antibody or antigen-binding fragment thereof. In yet other aspects, the anti-PD-1 or PD-L1 antibody molecule is administered in combination with an anti-LAG-3 antibody and an anti-TIM-3 antibody, or antigen-binding fragments thereof. The combination of antibodies recited herein can be administered separately, *e.g*., as separate antibodies, or linked, *e.g.,* as a bispecific or trispecific antibody molecule. In one aspect, a bispecific antibody that includes an anti-PD-1 or PD-L1 antibody molecule and an anti-TIM-3 or anti-LAG-3 antibody, or antigen-binding fragment thereof, is administered. In certain aspects, the combination of antibodies recited herein is used to treat a cancer, *e.g.,* a cancer as described herein (*e.g.,* a solid tumor). The efficacy of the aforesaid combinations can be tested in animal models known in the art. For example, the animal models to test the synergistic effect of anti-PD-1 and anti-LAG-3 are described, *e.g.,* in Woo et al. (2012) Cancer Res. 72(4):917-27).

In another aspect, the anti-PD-1 or PD-L1 antibody molecule is administered in combination with an inhibitor of CEACAM (*e.g*., CEACAM-1, -3 and/or -5). In one aspect, the inhibitor of CEACAM (*e.g*., CEACAM-1, -3 and/or -5) is an anti-CEACAM antibody molecule. Without wishing to be bound by theory, carcinoembryonic antigen cell adhesion molecules (CEACAM), such as CEACAM-1 and CEACAM-5, are believed to mediate, at least in part, inhibition of an anti-tumor immune response (*see e.g.,* Markel et al. J Immunol. 2002 Mar 15;168(6):2803-10; Markel et al. J Immunol. 2006 Nov 1;177(9):6062-71; Markel et al. Immunology. 2009 Feb;126(2):186-200; Markel et al. Cancer Immunol Immunother. 2010 Feb;59(2):215-30; Ortenberg et al. Mol Cancer Ther. 2012 Jun;11(6):1300-10; Stern et al. J Immunol. 2005 Jun 1;174(11):6692-701; Zheng et al. PLoS One. 2010 Sep 2;5(9). pii: e12529). For example, CEACAM-1 has been described as a heterophilic ligand for TIM-3 and as playing a role in TIM-3-mediated T cell tolerance and exhaustion (*see e.g.,* WO 2014/022332; Huang, et al. (2014) Nature doi:10.1038/nature13848). In aspects, co-blockade of CEACAM-1 and TIM-3 has been shown to enhance an anti-tumor immune response in xenograft colorectal cancer models (*see e.g.,* WO 2014/022332; Huang, *et al.* (2014), *supra*). In other embodiments, co-blockade of CEACAM-1 and PD-1 reduce T cell tolerance as described, *e.g.,* in WO 2014/059251. Thus, CEACAM inhibitors can be used with the other immunomodulators described herein (*e.g*., anti-PD-1 and/or anti-TIM-3 inhibitors) to enhance an immune response against a cancer, *e.g.,* a melanoma, a lung cancer (*e.g.,* NSCLC), a bladder cancer, a colon cancer an ovarian cancer, and other cancers as described herein.

Accordingly, in some embodiments, the anti-PD-1 antibody molecule is administered in combination with a CEACAM inhibitor (*e.g*., CEACAM-1, CEACAM-3, and/or CEACAM-5 inhibitor). In one embodiment, the inhibitor of CEACAM is an anti-CEACAM antibody molecule. In one embodiment, the anti-PD-1 antibody molecule is administered in combination with a CEACAM-1 inhibitor, *e.g.,* an anti- CEACAM-1 antibody molecule. In another embodiment, the anti-PD-1 antibody molecule is administered in combination with a CEACAM-3 inhibitor, *e.g.,* an anti- CEACAM-3 antibody molecule. In another embodiment, the anti-PD-1 antibody molecule is administered in combination with a CEACAM-5 inhibitor, *e.g.,* an anti-CEACAM-5 antibody molecule. Exemplary anti-CEACAM-1 antibodies are described in WO 2010/125571, WO 2013/082366 and WO 2014/022332, *e.g.,* a monoclonal antibody 34B1, 26H7, and 5F4; or a recombinant form thereof, as described in, *e.g.,* US 2004/0047858, US 7,132,255 and WO 99/052552. In other embodiments, the anti-CEACAM antibody binds to CEACAM-5 as described in, *e.g.,* Zheng et al. PLoS One. 2010 Sep 2;5(9). pii: e12529 (DOI:10:1371/journal.pone.0021146), or crossreacts with CEACAM-1 and CEACAM-5 as described in, *e.g.,* WO 2013/054331 and US 2014/0271618.

### Costimulatory Modulators

In certain aspects, the combination therapies disclosed herein include a modulator of a costimulatory molecule. In one aspect, the costimulatory modulator, *e.g*., agonist, of a costimulatory molecule is chosen from an agonist (*e.g*., an agonistic antibody or antigen-binding fragment thereof, or soluble fusion) of an MHC class I molecule, a TNF receptor protein, an Immunoglobulin-like proteins, a cytokine receptor, an integrin, a signaling lymphocytic activation molecules (SLAM proteins), an activating NK cell receptor, BTLA, a Toll ligand receptor, OX40, CD2, CD7, CD27, CD28, CD30, CD40, CDS, ICAM-1, LFA-1 (CD11a/CD18), 4-1BB (CD137), B7-H3, ICOS (CD278), GITR, BAFFR, LIGHT, HVEM (LIGHTR), KIRDS2, SLAMF7, NKp80 (KLRF1), NKp44, NKp30, NKp46, CD19, CD4, CD8alpha, CD8beta, IL2R beta, IL2R gamma, IL7R alpha, ITGA4, VLA1, CD49a, ITGA4, IA4, CD49D, ITGA6, VLA-6, CD49f, ITGAD, CD11d, ITGAE, CD103, ITGAL, ITGAM, CD11b, ITGAX, CD11c, ITGB1, CD29, ITGB2, CD18, ITGB7, NKG2D, NKG2C, TNFR2, TRANCE/RANKL, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), SLAM7, BLAME (SLAMF8), SELPLG (CD 162), LTBR, LAT, GADS, SLP-76, PAG/Cbp, CD19a, and a ligand that specifically binds with CD83.

In one aspect, the combination therapies disclosed herein include a costimulatory molecule, *e.g.,* an agonist associated with a positive signal that includes a costimulatory domain of CD28, CD27, ICOS and GITR.

### Exemplary GITR agonist

In one disclosed aspect, a combination described herein includes a GITR agonist. In some aspects, the combination is used to treat a cancer, *e.g.,* a cancer described herein, *e.g.,* a solid tumor or a hematologic malignancy.

Exemplary GITR agonists include, *e.g.,* GITR fusion proteins and anti-GITR antibodies (*e.g*., bivalent anti-GITR antibodies), such as, a GITR fusion protein described in U.S. Patent No.: 6,111,090, European Patent No.: 0920505B1, U.S Patent No.: 8,586,023, PCT Publication Nos.: WO 2010/003118 and 2011/090754, or an anti-GITR antibody described, *e.g.,* in U.S. Patent No.: 7,025,962, European Patent No.: 1947183B1, U.S. Patent No.: 7,812,135, U.S. Patent No.: 8,388,967, U.S. Patent No.: 8,591,886, European Patent No.: EP 1866339, PCT Publication No.: WO 2011/028683, U.S. Patent No.: 8,709,424, PCT Publication No.:WO 2013/039954, International Publication No.: WO2013/039954, U.S. Publication No.: US2014/0072566, International Publication NO.: WO2015/026684, PCT Publication No.: WO2005/007190, PCT Publication No.: WO 2007/133822, PCT Publication No.: WO2005/055808, PCT Publication No.: WO 99/40196, PCT Publication No.: WO 2001/03720, PCT Publication No.: WO99/20758, U.S. Patent No.: 6,689,607, PCT Publication No.: WO2006/083289, PCT Publication No.: WO 2005/115451, U.S. Patent No.: 7,618,632, PCT Publication No.: WO 2011/051726, International Publication No.: WO2004060319, and International Publication No.: WO2014012479.

In one aspect, the GITR agonist is used in combination with a PD-1 inhibitor, *e.g*., as described in WO2015/026684.

In another aspect, the GITR agonist is used in combination with a TLR agonist, *e.g.,* as described in WO2004060319, and International Publication No.: WO2014012479.

### Additional Combinations

In another embodiment, the combination therapies include a modified T-cell, *e.g.,* in combination with an adoptive T-cell immunotherapy using chimeric antigen receptor (CAR) T cells (*e.g.,* as described by John LB, et al. (2013) Clin. Cancer Res. 19(20): 5636-46).

In other embodiments, the combination therapies disclosed herein can also include a cytokine, *e.g*., interleukin-21 or interleukin-2. In certain embodiments, the combination described herein is used to treat a cancer, *e.g.,* a cancer as described herein (*e.g.,* a solid tumor or melanoma).

Exemplary immunomodulators that can be used in the combination therapies include, but are not limited to, *e.g*., afutuzumab (available from Roche®); pegfilgrastim (Neulasta®); lenalidomide (CC-5013, Revlimid®); thalidomide (Thalomid®), actimid (CC4047); and cytokines, *e.g*., IL-21 or IRX-2 (mixture of human cytokines including interleukin 1, interleukin 2, and interferon γ, CAS 951209-71-5, available from IRX Therapeutics).

In other embodiments, the combination therapies can be administered to a subject in conjunction with (*e.g*., before, simultaneously or following) one or more of: bone marrow transplantation, T cell ablative therapy using chemotherapy agents such as, fludarabine, external-beam radiation therapy (XRT), cyclophosphamide, and/or antibodies such as OKT3 or CAMPATH. In one aspect, the anti-PD-1 or PD-L1 antibody molecules are administered following B-cell ablative therapy such as agents that react with CD20, e.g., Rituxan. For example, subjects may undergo standard treatment with high dose chemotherapy followed by peripheral blood stem cell transplantation. In certain aspects, following the transplant, subjects receive the anti-PD-1 or PD-L1 antibody molecules. In an additional aspect, the anti-PD-1 or PD-L1 antibody molecules are administered before or following surgery.

Another example of a further combination therapy includes decarbazine for the treatment of melanoma. Without being bound by theory, the combined use of PD-1 blockade and chemotherapy is believed to be facilitated by cell death, that is a consequence of the cytotoxic action of most chemotherapeutic compounds, which can result in increased levels of tumor antigen in the antigen presentation pathway. Other combination therapies that may result in synergy with PD-1 blockade through cell death are radiation, surgery, and hormone deprivation. Each of these protocols creates a source of tumor antigen in the host. Angiogenesis inhibitors may also be combined with PD-1 blockade. Inhibition of angiogenesis leads to tumor cell death which may feed tumor antigen into host antigen presentation pathways.

Combination therapies can also be used in combination with bispecific antibodies. Bispecific antibodies can be used to target two separate antigens. For example anti-Fc receptor/anti tumor antigen (*e.g*., Her-2/neu) bispecific antibodies have been used to target macrophages to sites of tumor. This targeting may more effectively activate tumor specific responses. The T cell arm of these responses would by augmented by the use of PD-1 blockade. Alternatively, antigen may be delivered directly to DCs by the use of bispecific antibodies which bind to tumor antigen and a dendritic cell specific cell surface marker.

Tumors evade host immune surveillance by a large variety of mechanisms. Many of these mechanisms may be overcome by the inactivation of proteins which are expressed by the tumors and which are immunosuppressive. These include among others TGF-beta (Kehrl, J. et al. (1986) J. Exp. Med. 163: 1037-1050), IL-10 (Howard, M. & O'Garra, A. (1992) Immunology Today 13: 198-200), and Fas ligand (Hahne, M. et al. (1996) Science 274: 1363-1365). Antibodies or antigen-binding fragments thereof to each of these entities may be used in combination with anti-PD-1 to counteract the effects of the immunosuppressive agent and favor tumor immune responses by the host.

Other antibodies which may be used to activate host immune responsiveness can be used in combination with the combination therapies described herein. These include molecules on the surface of dendritic cells which activate DC function and antigen presentation. Anti-CD40 antibodies are able to substitute effectively for T cell helper activity (Ridge, J. et al. (1998) Nature 393: 474-478) and can be used in conjunction with PD-1 antibodies (Ito, N. et al. (2000) Immunobiology 201 (5) 527-40). Antibodies to T cell costimulatory molecules such as CTLA-4 *(e.g.,* U.S. Pat. No. 5,811,097), OX-40 (Weinberg, A. et al. (2000) Immunol 164: 2160-2169), 4-1BB (Melero, I. et al. (1997) Nature Medicine 3: 682-685 (1997), and ICOS (Hutloff, A. et al. (1999) Nature 397: 262-266) may also provide for increased levels of T cell activation.

In all of the methods described herein, PD-1 blockade can be combined with other forms of immunotherapy such as cytokine treatment (*e.g*., interferons, GM-CSF, G-CSF, IL-2, IL-21), or bispecific antibody therapy, which provides for enhanced presentation of tumor antigens (*see e.g.,* Holliger (1993) Proc. Natl. Acad. Sci. USA 90:6444-6448; Poljak (1994) Structure 2:1121-1123).

The combination therapies disclosed herein can be further combined with an immunogenic agent, such as cancerous cells, purified tumor antigens (including recombinant proteins, peptides, and carbohydrate molecules), cells, and cells transfected with genes encoding immune stimulating cytokines (He et al. (2004) J. Immunol. 173:4919-28). Non-limiting examples of tumor vaccines that can be used include peptides of melanoma antigens, such as peptides of gp100, MAGE antigens, Trp-2, MART1 and/or tyrosinase, or tumor cells transfected to express the cytokine GM-CSF.

PD-1 blockade can be combined with a vaccination protocol. Many experimental strategies for vaccination against tumors have been devised (*see* Rosenberg, S., 2000, Development of Cancer Vaccines, ASCO Educational Book Spring: 60-62; Logothetis, C., 2000, ASCO Educational Book Spring: 300-302; Khayat, D. 2000, ASCO Educational Book Spring: 414-428; Foon, K. 2000, ASCO Educational Book Spring: 730-738; *see* also Restifo, N. and Sznol, M., Cancer Vaccines, Ch. 61, pp. 3023-3043 in DeVita, V. et al. (eds.), 1997, Cancer: Principles and Practice of Oncology. Fifth Edition). In one of these strategies, a vaccine is prepared using autologous or allogeneic tumor cells. These cellular vaccines have been shown to be most effective when the tumor cells are transduced to express GM-CSF. GM-CSF has been shown to be a potent activator of antigen presentation for tumor vaccination (Dranoff et al. (1993) Proc. Natl. Acad. Sci. U.S.A. 90: 3539-43).

PD-1 blockade can be used in conjunction with a collection of recombinant proteins and/or peptides expressed in a tumor in order to generate an immune response to these proteins. These proteins are normally viewed by the immune system as self antigens and are therefore tolerant to them. The tumor antigen may also include the protein telomerase, which is required for the synthesis of telomeres of chromosomes and which is expressed in more than 85% of human cancers and in only a limited number of somatic tissues (Kim, N et al. (1994) Science 266: 2011-2013). (These somatic tissues may be protected from immune attack by various means). Tumor antigen may also be "neo-antigens" expressed in cancer cells because of somatic mutations that alter protein sequence or create fusion proteins between two unrelated sequences (ie. bcr-abl in the Philadelphia chromosome), or idiotype from B cell tumors.

Other tumor vaccines may include the proteins from viruses implicated in human cancers such a Human Papilloma Viruses (HPV), Hepatitis Viruses (HBV and HCV) and Kaposi's Herpes Sarcoma Virus (KHSV). Another form of tumor specific antigen which may be used in conjunction with PD-1 blockade is purified heat shock proteins (HSP) isolated from the tumor tissue itself. These heat shock proteins contain fragments of proteins from the tumor cells and these HSPs are highly efficient at delivery to antigen presenting cells for eliciting tumor immunity (Suot, R & Srivastava, P (1995) Science 269:1585-1588; Tamura, Y. et al. (1997) Science 278:117-120).

Dendritic cells (DC) are potent antigen presenting cells that can be used to prime antigen-specific responses. DC's can be produced *ex vivo* and loaded with various protein and peptide antigens as well as tumor cell extracts (Nestle, F. et al. (1998) Nature Medicine 4: 328-332). DCs may also be transduced by genetic means to express these tumor antigens as well. DCs have also been fused directly to tumor cells for the purposes of immunization (Kugler, A. et al. (2000) Nature Medicine 6:332-336). As a method of vaccination, DC immunization may be effectively combined with PD-1 blockade to activate more potent anti-tumor responses.

### Second Therapeutic Agents

The second therapeutic agent of the invention is LCL161, wherein LCL161 is (S)-N-((S)-1-cyclohexyl-2-((S)-2-(4-(4-fluorobenzoyl)thiazol-2-yl)pyrrolidin-1-yl)-2-oxoethyl)-2-(methylamino)propanamide or a pharmaceutically acceptable salt thereof.

According to the disclosure, the second therapeutic agent can be chosen from one or more of: 1) an IAP inhibitor; 2) a TOR kinase inhibitor; 3) a HDM2 ligase inhibitor; 4) a PIM kinase inhibitor; 5) a HER3 kinase inhibitor; 6) a Histone Deacetylase (HDAC) inhibitor; 7) a Janus kinase inhibitor; 8) an FGF receptor inhibitor; 9) an EGF receptor inhibitor; 10) a c-MET inhibitor; 11) an ALK inhibitor; 12) a CDK4/6-inhibitor; 13) a PI3K inhibitor; 14) a BRAF inhibitor; 15) a CAR T cell (*e.g.,* a CAR T cell targeting CD19); 16) a MEK inhibitor; or 17) a BCR-ABL inhibitor; *e.g.,* chosen from one or more of the agents listed in Table 1.

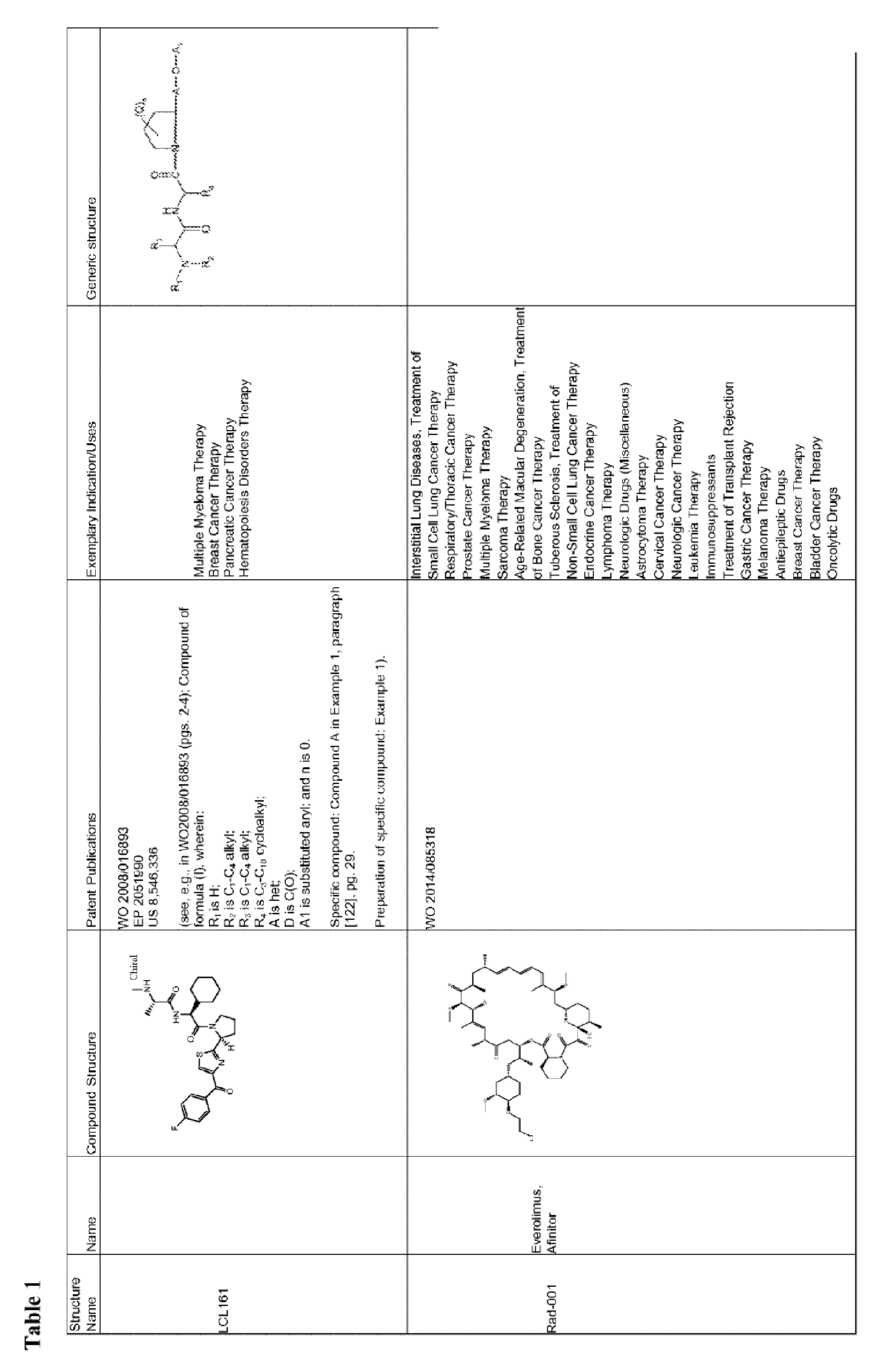

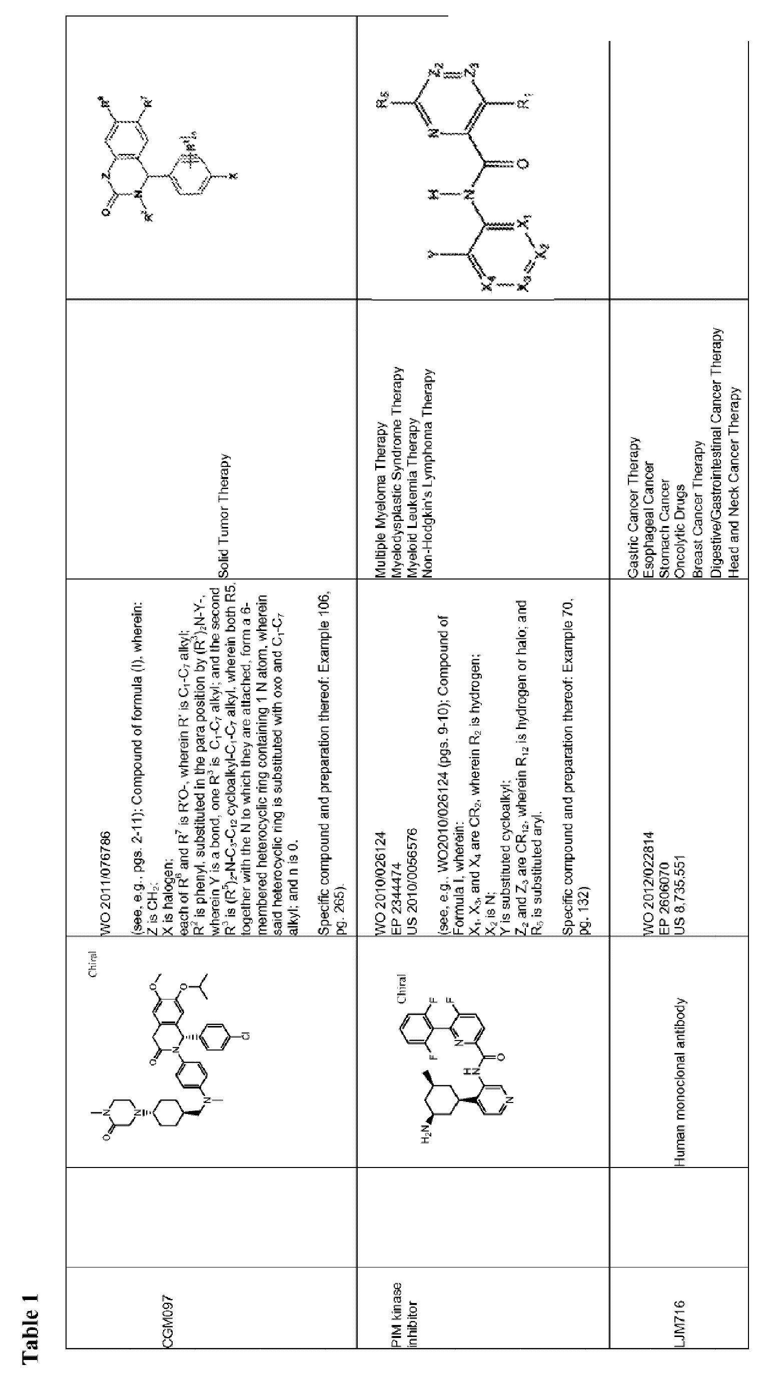

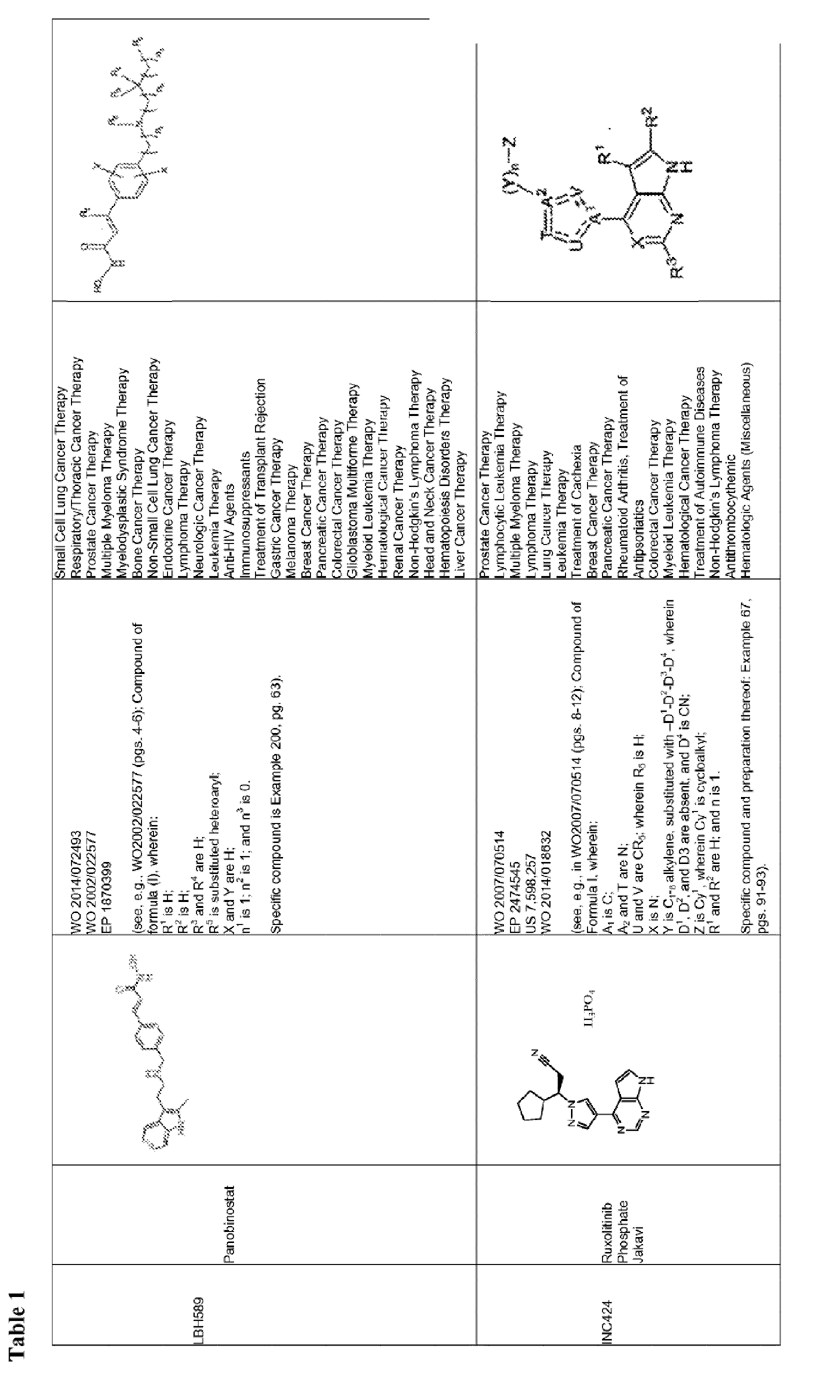

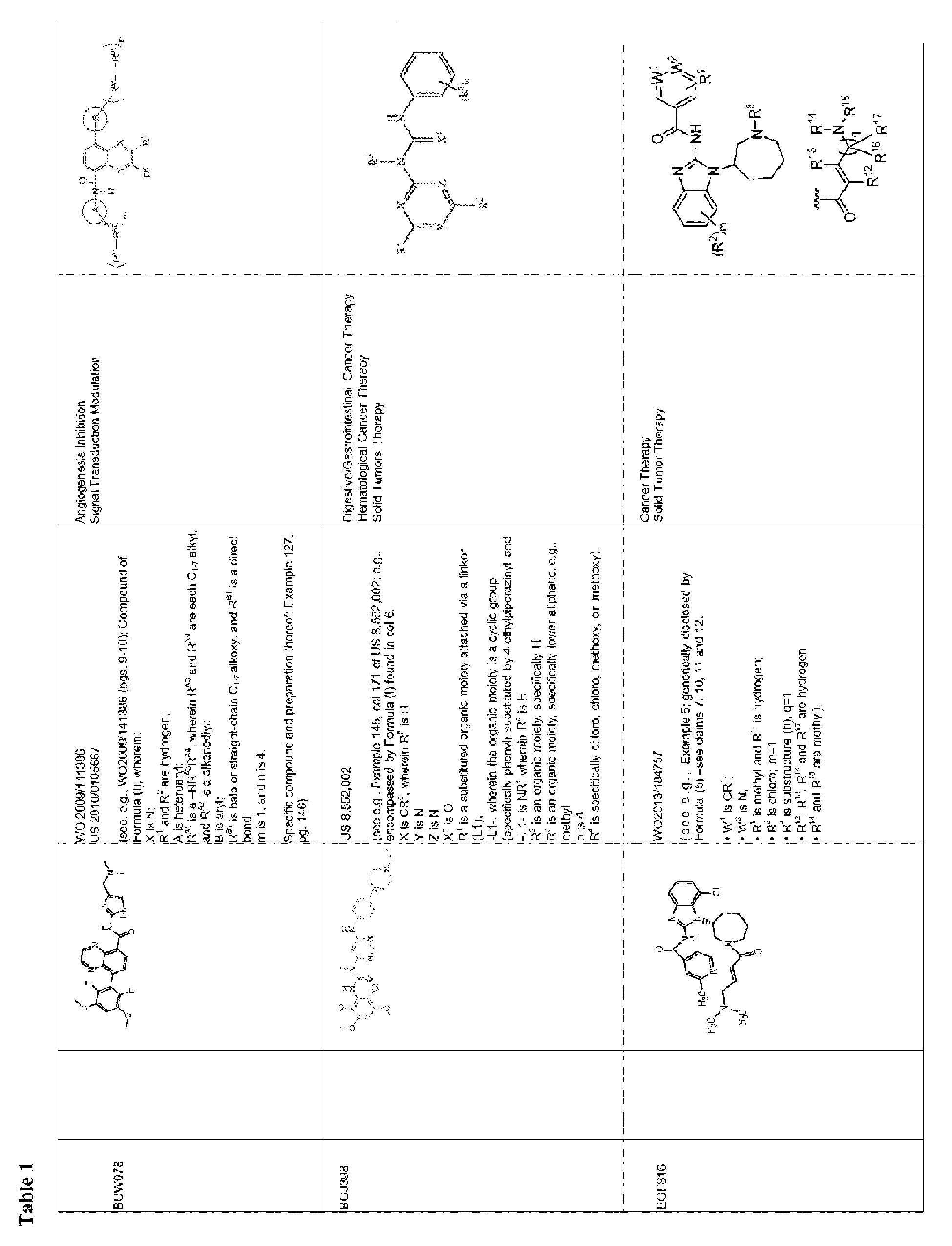

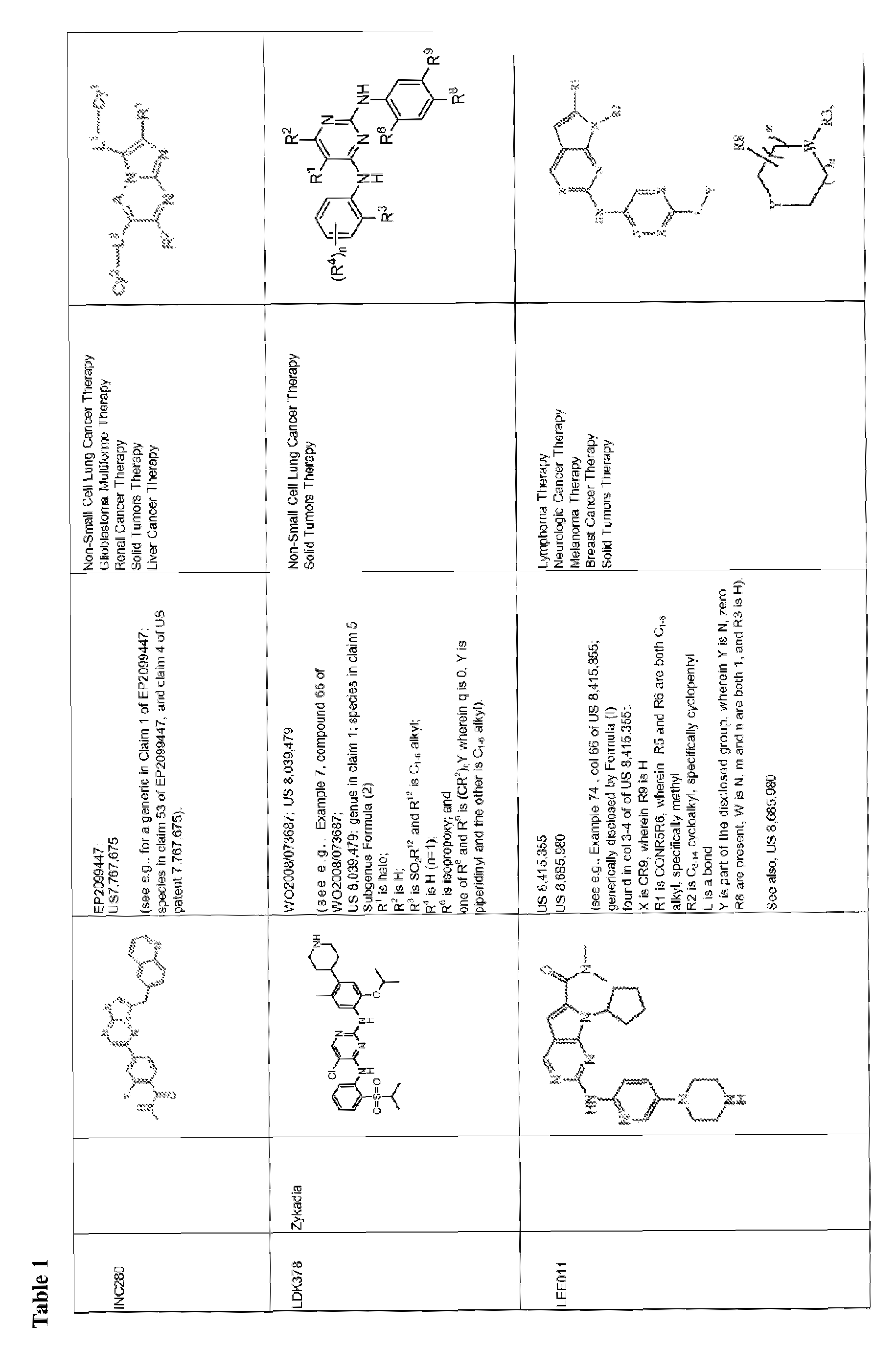

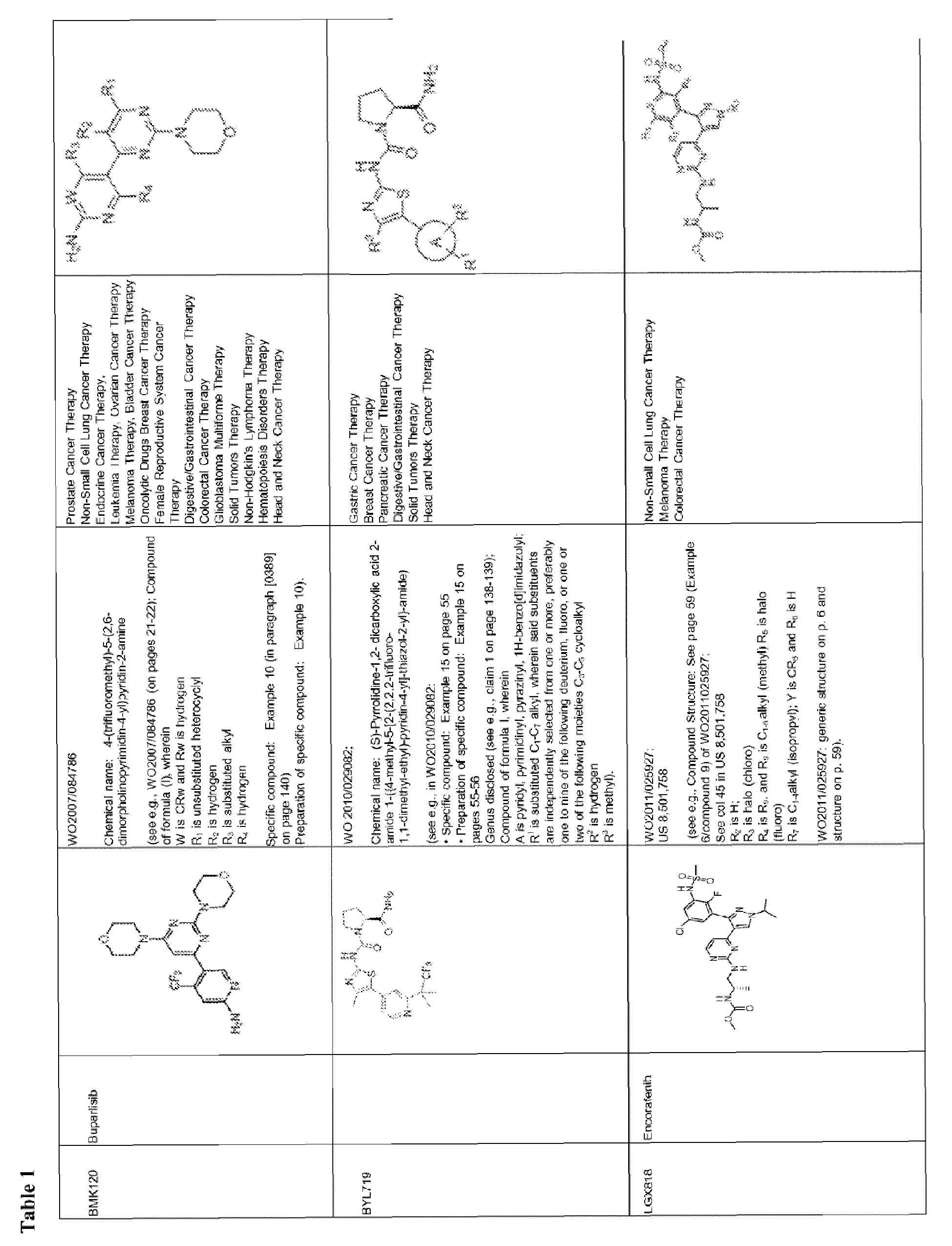

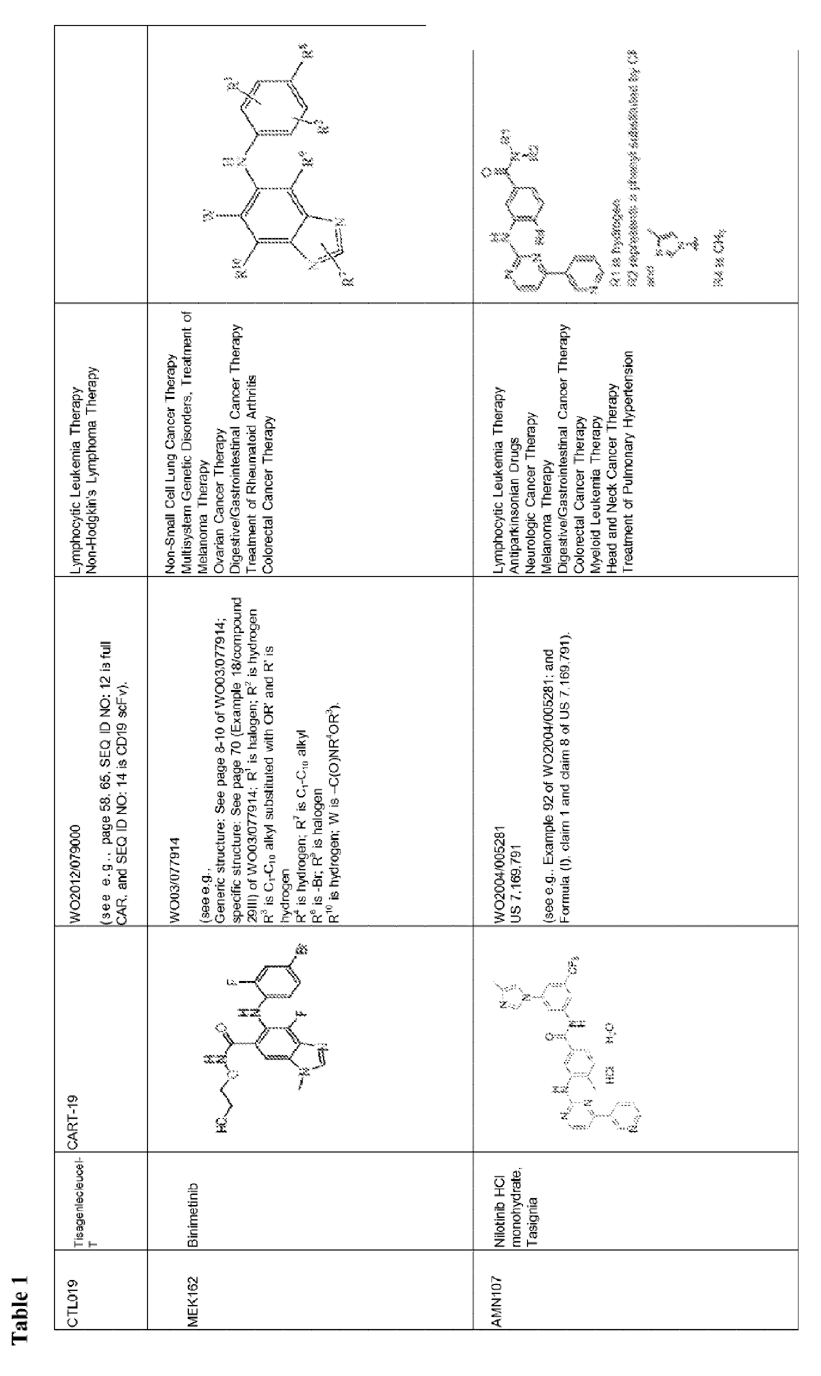

### Exemplary Combination Therapies

In certain embodiments, the inhibitor of the immune checkpoint molecule is used in a method or composition described herein. The inhibitor of the immune checkpoint molecule is the anti-PD-1 antibody Nivolumab, Pembrolizumab or Pidilizumab (alone or in combination with other immunomodulators) and is used in combination with the Inhibitor of Apoptosis (IAP) LCL161. In one embodiment, one or more of the aforesaid combinations is used to treat a disorder, *e.g.,* a disorder described herein (*e.g.*, a disorder disclosed in Table 1). In one embodiment, one or more of the aforesaid combinations is used to treat a cancer, *e.g.*, a cancer described herein (*e.g.*, a cancer disclosed in Table 1).

In some aspects of the disclosure, one or more of the immunomodulators described herein are used in combination with:
1) (S)-N-((S)-1-cyclohexyl-2-((S)-2-(4-(4-fluorobenzoyl)thiazol-2-yl)pyrrolidin-1-yl)-2-oxoethyl)-2-(methylamino)propanamide;
2) ((1R, 9S, 12S, 15R, 16E, 18R, 19R, 21R, 23S, 24E, 26E, 28E, 30S, 32S, 35R)-1,18-dihydroxy-12-{(1R)-2-[(1S,3R, 4R)-4-(2-hydroxyethoxy)-3-methoxycyclohexyl]-1-methylethyl}-19,30-dimethoxy-15,17,21,23,29,35-hexamethyl-11,36-dioxa-4-azatricyclo[30.3.1.04,9] hexatriaconta-16,24,26,28-tetraene-2,3,10,14,20-pentaone);
3) (S)-1-(4-chlorophenyl)-7-isopropoxy-6-methoxy-2-(4-{methyl-[4-(4-methyl-3-oxopiperazin-1-yl)-trans-cyclohexylmethyl]-amino}phenyl)-1,4-dihydro-2H-isoquinolin-3one;
4) N-(4-((1R,3S,5S)-3-amino-5-methylcyclohexyl)pyridin-3-yl)-6-(2,6-difluorophenyl)-5-fluoropicolinamide;
5) anti-HER3 monoclonal antibody or antigen binding fragment thereof, that comprises a VH of SEQ ID NO: 141 and VL of SEQ ID NO: 140, as described in U.S. 8,735,551;
6) (E)-N-hydroxy-3-(4-(((2-(2-methyl-1H-indol-3-yl)ethyl)amino)methyl)phenyl) acrylamide;
7) (3R)-3-cyclopentyl-3-[4-(7H-pyrrolo-[2,3-d]pyrimidin-4-yl)-1H-pyrazol-1-yl]propanenitrile; and/or
8) 8-(2,6-difluoro-3,5-dimethoxy-phenyl)-quinoxaline-5-carboxylic acid (4-dimethylaminomethyl-1H-imidazol-2-yl)-amide.

Each of these combinations is discussed in more detail below.

In one aspect, the inhibitor of the immune checkpoint molecule (alone or in combination with other immunomodulators) is used in combination with an IAP inhibitor to treat a cancer, *e.g.*, a cancer described herein (*e.g.*, a cancer disclosed in Table 1). The IAP inhibitor is disclosed in Table 1 as LCL161, or in a publication recited in Table 1, *e.g.*, International Patent Publication No. WO2008/016893 (*e.g.*, Formula (I), Example 1, and Compound A), European Patent No. 2051990, and U.S. Patent No. 8,546,336. In certain aspects, the IAP inhibitor is disclosed, *e.g.*, in International Patent Publication No. WO2008/016893 (*e*.*g*., Formula (I), Example 1, and Compound A), European Patent No. 2051990, and U.S. Patent No. 8,546,336. In one aspect, the IAP inhibitor, *e.g.*, LCL161, has the structure (compound or generic) provided in Table 1, or as disclosed in the publication recited in Table 1, *e.g.,* International Patent Publication No. WO2008/016893 (*e.g.*, Formula (I), Example 1, and Compound A), European Patent No. 2051990, and U.S. Patent No. 8,546,336. In one aspect, the inhibitor of the immune checkpoint molecule (*e.g.*, one of Nivolumab, Pembrolizumab or MSB0010718C) is used in combination with LCL161 to treat a cancer or disorder described in Table 1, *e.g.*, a solid tumor, e.g., a breast cancer or a pancreatic cancer; or a hematological malignancy, *e.g.*, multiple myeloma or a hematopoeisis disorder.

In one disclosed aspect, the IAP inhibitor is a compound of Formula (I): 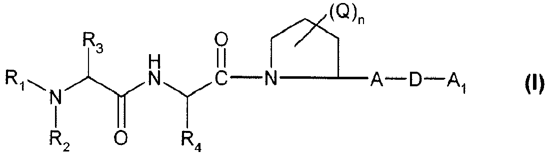 or a pharmaceutically acceptable salt thereof, wherein
R₁ is H, C₁-C₄ alkyl, C₂-C₄ alkenyl, C₂-C₄ alkynyl or C₃-C₁₀ cycloalkyl, which R₁ may be unsubstituted or substituted;
R₂ is H, C₁-C₄ alkyl, C₂-C₄ alkenyl, C₂-C₄ alkynyl, C₃-C₁₀ cycloalkyl which R₂ may be unsubstituted or substituted;
R₃ is H, CF₃, C₂F₆, C₁-C₄ alkyl, C₂-C₄ alkenyl, C₂-C₄ alkynyl, CH₂-Z, or
R₂ and R₃, taken together with the nitrogen atom to which they are attached, form a heterocyclic ring, which alkyl, alkenyl, alkynyl or het ring may be unsubstituted or substituted;
Z is H, OH, F, Cl, CH₃, CH₂CI, CH₂F or CH₂OH;
R₄ is C₀₋₁₀ alkyl, C₀₋₁₀ alkenyl, C₀₋₁₀ alkynyl, C₃-C₁₀ cycloalkyl, wherein the C₀₋₁₀ alkyl, or cycloalkyl group is unsubstituted or substituted;
A is het, which may be substituted or unsubstituted;
D is C₁-C₇ alkylene or C₂-C₉ alkenylene, C(O), O, NR₇, S(O)r, C(O)-C₁-C₁₀ alkyl, 0-C₁-C₁₀ alkyl, S(O)r-CᵣC₁₀ alkyl, C(O)C₀-C₁₀ arylalkyl, OC₀-C₁₀ arylalkyl, or S(O)r C₀-C₁₀ arylalkyl, which alkyl and aryl groups may be unsubstituted or substituted;
r is O, 1 or 2;
A₁ is a substituted or unsubstituted aryl or unsubstituted or substituted het which substituents on aryl and het are halo, alkyl, lower alkoxy, NR₅R₆, CN, NO₂ or SR₅;
each Q is independently H, C₁-C₁₀ alkyl, C₁-C₁₀ alkoxy, aryl C₁-C₁₀ alkoxy, OH, O-C₁-C₁₀ alkyl, (CH₂)₀-₆-C₃-C₇ cycloalkyl, aryl, aryl C₁-C₁₀ alkyl, O-(CH₂)₀-₆ aryl, (CH₂)₁-₆ het, het, O-(CH₂)₁-₆ het, -OR₁₁, C(O)R₁₁, -C(O)N(R₁₁)(R₁₂), N(R₁₁)(R₁₂J₁SR₁₁, S(O)R₁₁₁S(O)₂R₁₁, S(O)₂-N(R₁₁)(R₁₂), or NR₁₁-S(O)₂-(R₁₂), wherein alkyl, cycloalkyl and aryl are unsubstituted or substituted;
n is 0, 1 , 2 or 3, 4, 5, 6 or 7;
het is a 5- to 7-membered monocyclic heterocyclic ring containing 1-4 heteroring atoms selected from N, O and S or an 8- to 12-membered fused ring system that includes one 5- to 7-membered monocyclic heterocyclic ring containing 1 , 2 or 3 heteroring atoms selected from N, O and S, which het is unsubstituted or substituted;
R₁₁ and R₁₂ are independently H, C₁-C₁₀ alkyl, (CH₂)₀-₆-C₃-C₇ cycloalkyl, (CH₂)₀-₆-(CH)₀-₁(aryl)_{1·2},C(O)-C₁-C₁₀ alkyl, -C(O)-(CH₂)₁₋₆-C₃-C₇ cycloalkyl, -C(O)-O-(CH₂)₀₋₆-aryl, - C(O)-(CH₂)₀₋₆-O-fluorenyl, C(O)-NH-(CH₂)₀₋₆-aryl, C(O)-(CH₂)₀₋₆-aryl, C(O)-(CH₂)₁₋₆-het, - C(S)-CᵣC₁₀alkyl, -C(S)-(CH₂)_{L6}-C₃-C₇ cycloalkyl, -C(S)-O-(CH₂W aryl, -C(S)-(CH₂)₀₋₆-O-fluorenyl, C(S)-NH-(CH₂)₀₋₆-aryl, -C(S)-(CH₂)₀₋₆-aryl or C(S)-(CH₂)₁₋₆-het, C(O)R₁₁, C(O)NR₁₁R₁₂, C(O)OR₁₁, S(O)ₙR₁₁, S(O)₁ₙNR₁₁R₁₂, m = 1 or 2, C(S)R₁₁, C(S)NR₁₁R₁₂, C(S)OR₁₁, wherein alkyl, cycloalkyl and aryl are unsubstituted or substituted; or R₁₁ and R₁₂ are a substituent that facilitates transport of the molecule across a cell membrane,
or R₁₁ and R₁₂ together with the nitrogen atom form het,
wherein the alkyl substituents of R₁₁ and R₁₂ may be unsubstituted or substituted by one or more substituents selected from C₁-C₁₀ alkyl, halogen, OH, O-C₁-C₆ alkyl, -S-C₁-C₆ alkyl, CF₃ or NR₁₁R₁₂;
substituted cycloalkyl substituents of R₁₁ and R₁₂ are substituted by one or more substituents selected from a C₂-C₁₀ alkene; C₁-C₆ alkyl; halogen; OH; 0-C₁-C₆ alkyl; S-C₁-C₆ alkyl,CF₃; or NR₁₁R₁₂;
substituted het or substituted aryl of R₁₁ and R₁₂ are substituted by one or more substituents selected from halogen, hydroxy, C₁-C₄ alkyl, C₁-C₄ alkoxy, nitro, CNO-C(O)-CrC4alkyl and C(O)-O-CᵣC₄-alkyl;
R₅, R₆ and R₇ are independently hydrogen, lower alkyl, aryl, aryl lower alkyl, cycloalkyl, or cycloalkyl lower alkyl, C(O)R₅; S(O)R₅ C(O)OR₅ C(O)N R₅R₆, and
the substituents on R₁, R₂, R₃, R₄, Q, and A and A₁ groups are independently halo, hydroxy, lower alkyl, lower alkenyl, lower alkynyl, lower alkanoyl, lower alkoxy, aryl, aryl lower alkyl, amino, amino lower alkyl, diloweralkylamino, lower alkanoyl, amino lower alkoxy, nitro, cyano, cyano lower alkyl, carboxy, lower carbalkoxy, lower alkanoyl, aryloyl, lower arylalkanoyl, carbamoyl, N-mono- or N,N-di lower alkyl carbamoyl, lower alkyl carbamic acid ester, amidino, guanidine, ureido, mercapto, sulfo, lower alkylthio, sulfoamino, sulfonamide, benzosulfonamide, sulfonate, sulfanyl lower alkyl, aryl sulfonamide, halogen substituted aryl sulfonate, lower alkylsulfinyl, arylsulfinyl; aryl-lower alkylsulfinyl, lower alkylarylsulfinyl, lower alkylsulfonyl, arylsulfonyl, aryl-lower alkylsulfonyl, lower aryl alkyl lower alkylarylsulfonyl, halogen- lower alkylmercapto, halogen-lower alkylsulfonyl, phosphono (-P(=O)(OH)₂), hydroxy-lower alkoxy phosphoryl or di-lower alkoxyphosphoryl, (R₉)NC(O)-NR₁₀R₁₃, lower alkyl carbamic acid ester or carbamates or -NR₈R₁₄, wherein
R₈ and R₁₄ can be the same or different and are independently H or lower alkyl, or
R₈ and R₁₄, together with the N atom, form a 3- to 8-membered heterocyclic ring containing a nitrogen heteroring atoms and may optionally contain one or two additional heteroring atoms selected from nitrogen, oxygen and sulfur, which heterocyclic ring may be unsubstituted or substituted with lower alkyl, halo, lower alkenyl, lower alkynyl, hydroxy, lower alkoxy, nitro, amino, lower alkyl, amino, diloweralkyl amino, cyano, carboxy, lower carbalkoxy, formyl, lower alkanoyl, oxo, carbarmoyl, Λ/-lower or Λ/,Λ/- dilower alkyl carbamoyl, mercapto, or lower alkylthio; and
R₉, R₁₀ and R₁₃ are independently hydrogen, lower alkyl, halogen substituted lower alkyl, aryl, aryl lower alkyl, halogen substituted aryl, halogen substituted aryl lower alkyl.

In one embodiment, LCL161 has the following structure:

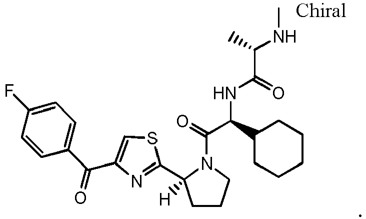

According to the invention, LCL161 is (S)-N-((S)-1-cyclohexyl-2-((S)-2-(4-(4-fluorobenzoyl)thiazol-2-yl)pyrrolidin-1-yl)-2-oxoethyl)-2-(methylamino)propanamide.

### LCL161 and Immunomodulators

LCL161, also known as SMAC mimetic LCL161 is an orally bioavailable second mitochondrial-derived activator of caspases (SMAC) mimetic and inhibitor of IAP (Inhibitor of Apoptosis Protein) family of proteins, with antineoplastic activity. SMAC mimetic LCL161 binds to IAPs, such as X chromosome-linked IAP (XIAP) and cellular IAPs 1 and 2. Since IAPs shield cancer cells from the apoptosis process, this agent can be used to restore and promote the induction of apoptosis through apoptotic signaling pathways in cancer cells. IAPs are overexpressed by many cancer cell types and suppress apoptosis by binding and inhibiting active caspases-3, -7 and -9, which play essential roles in apoptosis (programmed cell death), necrosis and inflammation.

In one embodiment, LCL161 has the structure provided in Table 1, or as disclosed in the publication recited in Table 1, *e.g.*, International Patent Publication No. WO2008/016893 (*e.g*., Formula (I), Example 1, and Compound A), European Patent No. 2051990, and U.S. Patent No. 8,546,336.

In one embodiment, LCL161 has the following structure:

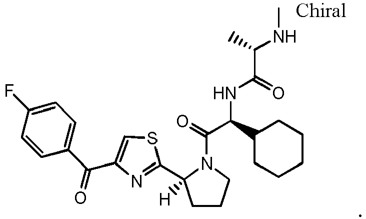

LCL161 is (S)-N-((S)-1-cyclohexyl-2-((S)-2-(4-(4-fluorobenzoyl)thiazol-2-yl)pyrrolidin-1-yl)-2-oxoethyl)-2-(methylamino)propanamide.

In one embodiment, the inhibitor of the immune checkpoint molecule (i.e. the PD-1 inhibitor Nivolumab, Pidilizumab or Pembrolizumab) is used in combination with LCL161 to treat a cancer or disorder described in Table 1, *e.g.*, a solid tumor, *e.g.*, a breast cancer or a pancreatic cancer; or a hematological malignancy, *e.g.*, multiple myeloma or a hematopoeisis disorder.

In one embodiment, the anti-PD-1 antibody molecule is administered intravenously. In one embodiment, in a combination therapy, LCL161 is administered orally. In one embodiment, the anti-PD-1 antibody molecule is administered, *e.g.*, intravenously, at least one, two, three, four, five, six, or seven days, *e.g.*, three days, after LCL161 is administered, *e.g.*, orally. In one embodiment, the anti-PD-1 antibody molecule is administered, *e.g.*, intravenously, at least one, two, three, four, five, six, or seven days, *e.g.*, three days, before LCL161 is administered, *e.g.*, orally. In yet another embodiment, the anti-PD-1 antibody molecule is administered, *e.g.*, intravenously, on the same day, as LCL161 is administered, *e.g.*, orally.

In one embodiment, the administration of the inhibitor of the immune checkpoint molecule (the anti-PD-1 antibody molecule) and LCL161 results in a synergistic effect. In certain embodiments, in a combination therapy, the concentration LCL161 that is required to achieve inhibition, *e.g.*, growth inhibition, is lower than the therapeutic dose of LCL161 as a monotherapy, *e.g.*, 10-20%, 20-30%, 30-40%, 40-50%, 50-60%, 60-70%, 70-80%, or 80-90% lower. In other embodiments, in a combination therapy, the concentration of the inhibitor of the immune checkpoint molecule (the anti-PD-1 antibody molecule) that is required to achieve inhibition, *e.g.*, growth inhibition, is lower than the therapeutic dose of the inhibitor of the immune checkpoint molecule (the anti-PD-1 antibody molecule) as a monotherapy, *e.g.*, 10-20%, 20-30%, 30-40%, 40-50%, 50-60%, 60-70%, 70-80%, or 80-90% lower. In one embodiment, administration of LCL161, alone or in combination with the anti-PD-1 antibody molecule, increases the expression of an immune-active cytokine, *e.g.*, IFN-gamma, in the cancer or the subject. In another embodiment, administration of LCL161, alone or in combination with the anti-PD-1 antibody molecule, reduces the expression of an immune-suppressive cytokine, *e.g.*, IL-10, in the cancer or the subject.

In an embodiment, the LCL161 is administered at a dose (*e.g.*, oral dose) of about 10-3000 mg, *e.g.*, about 20-2400 mg, about 50-1800 mg, about 100-1500 mg, about 200-1200 mg, about 300-900 mg, *e.g.*, about 600 mg, about 900 mg, about 1200 mg, about 1500 mg, about 1800 mg, about 2100 mg, or about 2400 mg. In an embodiment, LCL161 is administered once a week or once every two weeks.

In one aspect, the present disclosure relates to a pharmaceutical combination, especially a pharmaceutical combination product, comprising the combination of an immunomodulator and an agent disclosed herein.

The term "pharmaceutical combination" as used herein refers to a product obtained from mixing or combining in a non-fixed combination the active ingredients.

The term "non-fixed combination" means that the active ingredients, are both administered separately or together, independently at the same time or separately within time intervals, wherein such administration provides therapeutically effective levels of the active ingredient in the subject in need. The latter also applies to cocktail therapy, *e.g.* the administration of three or more active ingredients.

The term "jointly therapeutically effective" means that the compounds show synergistic interaction when administered separately or together, independently at the same time or separately within time intervals, to treat a subject in need, such as a warm-blooded animal in particular a human.

It was shown that the combination of the present disclosure possesses beneficial therapeutic properties, *e.g.* synergistic interaction, strong *in-vivo* and *in-vitro* antitumor response, which can be used as a medicine. Its characteristics render it particularly useful for the treatment of cancer.

Suitable cancers that can be treated with the combination of the present disclosure include but are not limited to anaplastic large cell lymphoma (ALCL), neuroblastoma, lung cancer, non-small cell lung cancer (NSCLC). In a preferred embodiment, the cancer is NSCLC.

The combination according to the present disclosure can besides or in addition be administered especially for cancer therapy in combination with chemotherapy, radiotherapy, immunotherapy, surgical intervention, or in combination of these. Long-term therapy is equally possible as is adjuvant therapy in the context of other treatment strategies, as described above. Other possible treatments are therapy to maintain the patient's status after tumor regression, or even chemo-preventive therapy, for example in patients at risk.

For example, the term "jointly (therapeutically) active" may mean that the compounds may be given separately or sequentially (in a chronically staggered manner, especially a sequence specific manner) in such time intervals that they preferably, in the warm-blooded animal, especially human, to be treated, and still show a (preferably synergistic) interaction (joint therapeutic effect). A joint therapeutic effect can, *inter alia,* be determined by following the blood levels, showing that both compounds are present in the blood of the human to be treated at least during certain time intervals, but this is not to exclude the case where the compounds are jointly active although they are not present in blood simultaneously.

The present disclosure also describes the method for the treatment of an ALK mediated disease, wherein the combination of (i) LDK378, or a pharmaceutically acceptable salt thereof, and (ii) Nivolumab or a pharmaceutically acceptable salt thereof separately or together.

The present disclosure relates to a pharmaceutical composition comprising effective amounts of (i) LDK378, or a pharmaceutically acceptable salt thereof, and (ii) Nivolumab, or a pharmaceutically acceptable salt thereof.

The present disclosure also describes the pharmaceutical combination according to the present disclosure in the form of a "kit of parts" for the combined administration. The combination can refer to either a fixed combination in one dosage unit form, or a kit of parts for the combined administration where (i) LDK378, or a pharmaceutically acceptable salt thereof, and (ii) Nivolumab, or a pharmaceutically acceptable salt thereof, may be administered independently at the same time or separately within time intervals, especially where these time intervals allow that the combination partners show a cooperative (= joint) effect. The independent formulations or the parts of the formulation, product, or composition, can then, *e.g.* be administered simultaneously or chronologically staggered, that is at different time points and with equal or different time intervals for any part of the kit of parts. In the combination therapies of the disclosure, the compounds useful according to the disclosure may be manufactured and/or formulated by the same or different manufacturers. Moreover, the combination partners may be brought together into a combination therapy: (i) prior to release of the combination product to physicians (e.g. in the case of a kit comprising LDK378 and the Nivolumab); (ii) by the physician themselves (or under the guidance of a physician) shortly before administration; (iii) in the patient themselves, *e.g.* during sequential administration of the compound of the disclosure and the other therapeutic agent. In one embodiment the effect of the combination is synergistic.

The therapeutically effective dosage of the combination of the disclosure, or pharmaceutical composition, is dependent on the species of the subject, the body weight, age and individual condition, the disorder or disease or the severity thereof being treated, and can be determined by standard clinical techniques. In addition, *in vitro* or *in vivo* assays can optionally be employed to help identify optimal dosage ranges. The precise dose to be employed can also depend on the route of administration, and the seriousness of the condition being treated and can be decided according to the judgment of the practitioner and each subject's circumstances in view of, *e.g.*, published clinical studies.

### Pharmaceutical Compositions and Kits

In another aspect, the present invention as defined in the claims provides compositions, *e.g.*, pharmaceutically acceptable compositions, which include an antibody molecule described herein, formulated together with a pharmaceutically acceptable carrier. The term "pharmaceutically acceptable" refers to those compounds, materials, compositions, and/or dosage forms which are suitable for use in contact with the tissues of human beings and animals without excessive toxicity, irritation, allergic response, or other problem or complication, commensurate with a reasonable benefit/risk ratio.

As used herein, "pharmaceutically acceptable carrier" includes any and all solvents, dispersion media, isotonic and absorption delaying agents, and the like that are physiologically compatible. The carrier can be suitable for intravenous, intramuscular, subcutaneous, parenteral, rectal, spinal or epidermal administration (*e.g.* by injection or infusion).

Pharmaceutically acceptable salts can be formed, for example, as acid addition salts, preferably with organic or inorganic acids. Suitable inorganic acids are, for example, halogen acids, such as hydrochloric acid. Suitable organic acids are, *e.g.*, carboxylic acids or sulfonic acids, such as fumaric acid or methanesulfonic acid. For isolation or purification purposes it is also possible to use pharmaceutically unacceptable salts, for example picrates or perchlorates. For therapeutic use, only pharmaceutically acceptable salts or free compounds are employed (where applicable in the form of pharmaceutical preparations), and these are therefore preferred. In view of the close relationship between the novel compounds in free form and those in the form of their salts, including those salts that can be used as intermediates, for example in the purification or identification of the novel compounds, any reference to the free compounds hereinbefore and hereinafter is to be understood as referring also to the corresponding salts, as appropriate and expedient. The salts of compounds described herein are preferably pharmaceutically acceptable salts; suitable counter-ions forming pharmaceutically acceptable salts are known in the field.

The compositions of this invention may be in a variety of forms. These include, for example, liquid, semi-solid and solid dosage forms, such as liquid solutions (*e.g.*, injectable and infusible solutions), dispersions or suspensions, liposomes and suppositories. The preferred form depends on the intended mode of administration and therapeutic application. Typical preferred compositions are in the form of injectable or infusible solutions. The preferred mode of administration is parenteral (*e.g.*, intravenous, subcutaneous, intraperitoneal, intramuscular). In a preferred embodiment, the antibody is administered by intravenous infusion or injection. In another preferred embodiment, the antibody is administered by intramuscular or subcutaneous injection.

The pharmaceutical composition can be prepared with a pharmaceutically acceptable carrier, which can be for example any suitable pharmaceutical excipient. The carrier includes any and all binders, fillers, solvents, dispersion media, coatings, surfactants, antioxidants, preservatives (*e.g.*, antibacterial agents, antifungal agents), isotonic agents, absorption delaying agents, salts, drug stabilizers, disintegration agents, lubricants, sweetening agents, flavoring agents, dyes, and the like and combinations thereof, as would be known to those skilled in the art (*see*, for example, Remington's Pharmaceutical Sciences, 18th Ed. Mack Printing Company, 1990, pp. 1289- 1329; Remington: The Science and Practice of Pharmacy, 21st Ed. Pharmaceutical Press 2011; and subsequent versions thereof). Except insofar as any conventional carrier is incompatible with the active ingredient, its use in the therapeutic or pharmaceutical compositions is contemplated. Other disclosure herein relating to the pharmaceutical composition can also be followed.

In accordance with the present disclosure, the combination partners can be administered independently at the same time or separately within time intervals in separate unit dosage forms. The two therapeutic partners may be prepared in a manner known per se and are suitable for enteral, such as oral or rectal, topical and parenteral administration to subject in need thereof, including warm-blooded animal, in particular a human being. Suitable pharmaceutical compositions contain, e.g., from about 0.1% to about 99.9% of active ingredient.

The pharmaceutical composition can be processed to prepare a final dosage form - a tablet or a capsule. This can be achieved by compressing the final blend of the combination, optionally together with one or more excipients. The compression can be achieved for example with a rotary tablet press. Tablet of different shapes can be prepared (round, ovaloid, or other suitable shape). The tablet can be coated or uncoated by known techniques to delay disintegration and absorption in the gastrointestinal tract and thereby provide a sustained action over a longer period. If not indicated otherwise, these are prepared in a manner known per se, *e.g.* by means of mixing, granulating, sugar-coating processes. Formulation for oral use can be presented as hard gelatin capsules wherein the active ingredient is mixed with an inert solid diluent, for example, calcium carbonate, calcium phosphate or cellulose-based excipient, or as soft gelatin capsules wherein the active ingredient is mixed with water or an oil medium, for example, olive oil, liquid paraffin or peanut oil.

The phrases "parenteral administration" and "administered parenterally" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion.

Therapeutic compositions typically should be sterile and stable under the conditions of manufacture and storage. The composition can be formulated as a solution, microemulsion, dispersion, liposome, or other ordered structure suitable to high antibody concentration. Sterile injectable solutions can be prepared by incorporating the active compound (*i.e.*, antibody or antibody portion) in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying that yields a powder of the active ingredient plus any additional desired ingredient from a previously sterile-filtered solution thereof. The proper fluidity of a solution can be maintained, for example, by the use of a coating such as lecithin, by the maintenance of the required particle size in the case of dispersion and by the use of surfactants. Prolonged absorption of injectable compositions can be brought about by including in the composition an agent that delays absorption, for example, monostearate salts and gelatin.

The antibody molecules can be administered by a variety of methods known in the art, although for many therapeutic applications, the preferred route/mode of administration is intravenous injection or infusion. For example, the antibody molecules can be administered by intravenous infusion at a rate of less than 10 mg/min; preferably less than or equal to 5 mg/min to reach a dose of about 1 to 100 mg/m², preferably about 5 to 50 mg/m², about 7 to 25 mg/m² and more preferably, about 10 mg/m². As will be appreciated by the skilled artisan, the route and/or mode of administration will vary depending upon the desired results. In certain embodiments, the active compound may be prepared with a carrier that will protect the compound against rapid release, such as a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are patented or generally known to those skilled in the art. *See, e.g.,* Sustained and Controlled Release Drug Delivery Systems, J. R. Robinson, ed., Marcel Dekker, Inc., New York, 1978.

In certain embodiments, an antibody molecule can be orally administered, for example, with an inert diluent or an assimilable edible carrier. The compound (and other ingredients, if desired) may also be enclosed in a hard or soft shell gelatin capsule, compressed into tablets, or incorporated directly into the subject's diet. For oral therapeutic administration, the compounds may be incorporated with excipients and used in the form of ingestible tablets, buccal tablets, troches, capsules, elixirs, suspensions, syrups, wafers, and the like. To administer a compound of the invention by other than parenteral administration, it may be necessary to coat the compound with, or co-administer the compound with, a material to prevent its inactivation. Therapeutic compositions can also be administered with medical devices known in the art.

Dosage regimens are adjusted to provide the optimum desired response *(e.g.,* a therapeutic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic effect in association with the required pharmaceutical carrier. The specification for the dosage unit forms of the invention are dictated by and directly dependent on (a) the unique characteristics of the active compound and the particular therapeutic effect to be achieved, and (b) the limitations inherent in the art of compounding such an active compound for the treatment of sensitivity in individuals.

The term "effective amount" refers to the amount of the subject compound that can engender a biological or medical response in a cell, tissue, organ, system, animal or human that is being sought by the researcher, veterinarian, medical doctor or other clinician. The effective dosage of each combination partner agents employed in the combinations disclosed herein may vary depending on the particular compound or pharmaceutical composition employed, the mode of administration, the condition being treated, the severity the condition being treated. A physician, clinician or veterinarian of ordinary skill can readily determine and prescribe the effective amount of the drug required to prevent, counter or arrest the progress of the condition. Optimal precision in achieving concentration of drug within the range that yields efficacy requires a regimen based on the kinetics of the combination's drugs availability to target sites. This involves a consideration of the distribution, equilibrium and elimination of a drug.

A "therapeutically effective amount" refers to an amount effective, at dosages and for periods of time necessary, to achieve the desired therapeutic result. A therapeutically effective amount of the modified antibody or antibody fragment may vary according to factors such as the disease state, age, sex, and weight of the individual, and the ability of the antibody or antibody portion to elicit a desired response in the individual. A therapeutically effective amount is also one in which any toxic or detrimental effects of the modified antibody or antibody fragment is outweighed by the therapeutically beneficial effects. A "therapeutically effective dosage" preferably inhibits a measurable parameter, e.g., tumor growth rate by at least about 20%, more preferably by at least about 40%, even more preferably by at least about 60%, and still more preferably by at least about 80% relative to untreated subjects. The ability of a compound to inhibit a measurable parameter, *e.g.*, cancer, can be evaluated in an animal model system predictive of efficacy in human tumors. Alternatively, this property of a composition can be evaluated by examining the ability of the compound to inhibit, such inhibition *in vitro* by assays known to the skilled practitioner.

A "prophylactically effective amount" refers to an amount effective, at dosages and for periods of time necessary, to achieve the desired prophylactic result. Typically, since a prophylactic dose is used in subjects prior to or at an earlier stage of disease, the prophylactically effective amount will be less than the therapeutically effective amount.

Methods of administering the antibody molecules are known in the art and are described below. Suitable dosages of the molecules used will depend on the age and weight of the subject and the particular drug used. Dosages and therapeutic regimens of the anti-PD-1 antibody molecule can be determined by a skilled artisan. In certain embodiments, the anti-PD-1 antibody molecule is administered by injection (*e.g.*, subcutaneously or intravenously) at a dose of about 1 to 30 mg/kg, *e.g.*, about 5 to 25 mg/kg, about 10 to 20 mg/kg, about 1 to 5 mg/kg, or about 3 mg/kg. The dosing schedule can vary from *e.g.,* once a week to once every 2, 3, or 4 weeks. In one embodiment, the anti-PD-1 antibody molecule is administered at a dose from about 10 to 20 mg/kg every other week.

An exemplary, non-limiting range for a therapeutically or prophylactically effective amount of an antibody molecule is 0.1-30 mg/kg, more preferably 1-25 mg/kg. Dosages and therapeutic regimens of the anti-PD-1 antibody molecule can be determined by a skilled artisan. In certain embodiments, the anti-PD-1 antibody molecule is administered by injection (*e.g.*, subcutaneously or intravenously) at a dose of about 1 to 30 mg/kg, *e.g.*, about 5 to 25 mg/kg, about 10 to 20 mg/kg, about 1 to 5 mg/kg, 1 to 10 mg/kg, 5 to 15 mg/kg, 10 to 20 mg/kg, 15 to 25 mg/kg, or about 3 mg/kg. The dosing schedule can vary from *e.g.,* once a week to once every 2, 3, or 4 weeks. In one embodiment, the anti-PD-1 antibody molecule is administered at a dose from about 10 to 20 mg/kg every other week. The antibody molecule can be administered by intravenous infusion at a rate of less than 10 mg/min, preferably less than or equal to 5 mg/min to reach a dose of about 1 to 100 mg/m², preferably about 5 to 50 mg/m², about 7 to 25 mg/m², and more preferably, about 10 mg/m². It is to be noted that dosage values may vary with the type and severity of the condition to be alleviated. It is to be further understood that for any particular subject, specific dosage regimens should be adjusted over time according to the individual need and the professional judgment of the person administering or supervising the administration of the compositions, and that dosage ranges set forth herein are exemplary only and are not intended to limit the scope or practice of the claimed composition.

The antibody molecules can be used by themselves or conjugated to a second agent, *e.g.*, a cytotoxic drug, radioisotope, or a protein, *e.g.*, a protein toxin or a viral protein. This method includes: administering the antibody molecule, alone or conjugated to a cytotoxic drug, to a subject requiring such treatment. The antibody molecules can be used to deliver a variety of therapeutic agents, *e.g.*, a cytotoxic moiety, *e.g.*, a therapeutic drug, a radioisotope, molecules of plant, fungal, or bacterial origin, or biological proteins (*e.g.*, protein toxins) or particles (*e.g.*, a recombinant viral particles, *e.g.*; via a viral coat protein), or mixtures thereof.

Also within the scope of the invention is a kit that includes a combination therapy described herein. The kit can include one or more other elements including: instructions for use; other reagents, *e.g.*, a label, a therapeutic agent, or an agent useful for chelating, or otherwise coupling, an antibody to a label or therapeutic agent, or a radioprotective composition; devices or other materials for preparing the antibody for administration; pharmaceutically acceptable carriers; and devices or other materials for administration to a subject.

### Uses of Combination Therapies

The combination therapies disclosed herein have *in vitro and in vivo* therapeutic and prophylactic utilities. For example, these molecules can be administered to cells in culture, *in vitro* or *ex vivo,* or to a subject, *e.g.,* a human subject, to treat, prevent, and/or diagnose a variety of disorders, such as cancers.

As used herein, the terms "treat", "treatment" and "treating" refer to the reduction or amelioration of the progression, severity and/or duration of a disorder, *e.g.,* a proliferative disorder (*e.g.*, a cancer), or the amelioration of one or more symptoms (preferably, one or more discernible symptoms) of a disorder, *e.g.*, a proliferative disorder, resulting from the administration of one or more therapies (*e.g.*, one or more therapeutic agents such as the combination therapies disclosed herein). In specific embodiments, the terms "treat", "treatment" and "treating" refer to the amelioration of at least one measurable physical parameter of a proliferative disorder (*e.g.*, a cancer), such as growth of a tumor, not necessarily discernible by the subject, *e.g.*, a patient. In other embodiments the terms "treat", "treatment" and "treating" refer to the inhibition of the progression of a proliferative disorder, either physically by, *e.g.,* stabilization of a discernible symptom, physiologically by, *e.g.*, stabilization of a physical parameter, or both. In other embodiments the terms "treat", "treatment" and "treating" refer to the reduction or stabilization of tumor size or cancerous cell count.

In some embodiments, ameliorating the disorder includes one or more of: slowing or arresting or reducing the development of the disease or at least one of the clinical symptoms thereof), to preventing or delaying the onset or development or progression of the disease or disorder. In addition those terms refers to alleviating or ameliorating at least one physical parameter including those which may not be discernible by the patient and also to modulating the disease or disorder, either physically (*e.g.* stabilization of a discernible symptom), physiologically (*e.g.* stabilization of a physical parameter), or both.

The term "treatment" comprises, for example, the therapeutic administration of one or more combination therapies disclosed herein to a subject, *e.g.*, warm-blooded animal, in particular a human being, in need of such treatment. In embodiment, the treatment aims to cure the disease or to have an effect on disease regression or on the delay of progression of a disease.

As used herein, the term "subject" is intended to include human and non-human animals. In one embodiment, the subject is a human subject, *e.g.*, a human patient having a disorder or condition characterized by abnormal cell proliferation and/or immune functioning. The term "non-human animals" includes mammals and non-mammals, such as non-human primates. In one embodiment, the subject is a human. In one embodiment, the subject is a human patient in need of enhancement of an immune response. The term "subject in need" refers to a warm-blooded animal, in particular a human being that would benefit biologically, medically or in quality of life from the treatment. In one embodiment, the subject is immunocompromised, *e.g.*, the subject is undergoing, or has undergone a chemotherapeutic or radiation therapy. Alternatively, or in combination, the subject is, or is at risk of being, immunocompromised as a result of an infection. The methods and compositions described herein are suitable for treating human patients having a disorder that can be treated by augmenting the T-cell mediated immune response. For example, the methods and compositions described herein can enhance a number of immune activities. In one embodiment, the subject has increased number or activity of tumour-infiltrating T lymphocytes (TILs). In another embodiment, the subject has increased expression or activity of interferon-gamma (IFN-γ). In yet another embodiment, the subject has decreased PD-L1 expression or activity.

Accordingly, in one aspect, the disclosure provides a method of modifying an immune response in a subject comprising administering to the subject the antibody molecule described herein, such that the immune response in the subject is modified. In one embodiment, the immune response is enhanced, stimulated or up-regulated. In one aspect, the antibody molecules enhance an immune response in a subject by blockade of a checkpoint inhibitor (*e.g.*, PD-1, PD-L1, LAG-3 or TIM-3).

### Cancer

Blockade of checkpoint inhibitors, *e.g.*, PD-1, can enhance an immune response to cancerous cells in a subject. The ligand for PD-1, PD-L1, is not expressed in normal human cells, but is abundant in a variety of human cancers (Dong et al. (2002) Nat Med 8:787-9). The interaction between PD-1 and PD-L1 can result in a decrease in tumor infiltrating lymphocytes, a decrease in T-cell receptor mediated proliferation, and/or immune evasion by the cancerous cells (Dong et al. (2003) J Mol Med 81:281-7; Blank et al. (2005) Cancer Immunol. Immunother. 54:307-314; Konishi et al. (2004) Clin. Cancer Res. 10:5094-100).

In one aspect, the disclosure relates to treatment of a subject *in vivo* using an immunomodulatory, *e.g.*, anti-PD-1 or anti-PD-L1 antibody molecule, alone or in combination with a second agent described herein, such that growth of cancerous tumors is inhibited or reduced. An immunomodulator may be used alone to inhibit the growth of cancerous tumors. Alternatively, an anti-PD-1 or anti-PD-L1 antibody may be used in combination with one or more of: an agent disclosed in Table 1, a standard of care treatment (*e.g.*, for cancers), another antibody or antigen-binding fragment thereof, another immunomodulator (*e.g.*, an activator of a costimulatory molecule or an inhibitor of an inhibitory molecule); a vaccine, *e.g.*, a therapeutic cancer vaccine; or other forms of cellular immunotherapy, as described below.

Accordingly, in one aspect, the disclosure provides a method of inhibiting growth of tumor cells in a subject, comprising administering to the subject a therapeutically effective amount of a combination therapy disclosed herein. In one aspect, the methods are suitable for the treatment of cancer *in vivo.* When antibodies to PD-1 are administered in combination with one or more agents, the combination can be administered in either order or simultaneously.

In another aspect, a method of treating a subject, *e.g.*, reducing or ameliorating, a proliferative condition or disorder (*e.g.*, a cancer), *e.g.*, solid tumor, a soft tissue tumor, or a metastatic lesion, in a subject is provided. The method includes administering to the subject one or more immunomodulators, *e.g.*, anti-PD-1 or PD-L1 antibody molecules described herein, alone or in combination with other agents or therapeutic modalities (*e.g.*, one or more agents from Table 1).

As used herein, the term "cancer" is meant to include all types of cancerous growths or oncogenic processes, metastatic tissues or malignantly transformed cells, tissues, or organs, irrespective of histopathologic type or stage of invasiveness. Examples of cancerous disorders include, but are not limited to, solid tumors, soft tissue tumors, and metastatic lesions. Examples of solid tumors include malignancies, *e.g.*, sarcomas, adenocarcinomas, and carcinomas, of the various organ systems, such as those affecting liver, lung, breast, lymphoid, gastrointestinal (*e.g.*, colon), genitourinary tract (*e.g.*, renal, urothelial cells), prostate and pharynx. Adenocarcinomas include malignancies such as most colon cancers, rectal cancer, renal-cell carcinoma, liver cancer, non-small cell carcinoma of the lung, cancer of the small intestine and cancer of the esophagus. In one embodiment, the cancer is a melanoma, *e.g.*, an advanced stage melanoma. Metastatic lesions of the aforementioned cancers can also be treated or prevented using the methods and compositions of the disclosure.

Exemplary cancers whose growth can be inhibited using the antibodies molecules disclosed herein include cancers typically responsive to immunotherapy. Non-limiting examples of preferred cancers for treatment include melanoma (*e.g.*, metastatic malignant melanoma), renal cancer (*e.g.*, clear cell carcinoma), prostate cancer (*e.g.*, hormone refractory prostate adenocarcinoma), breast cancer, colon cancer and lung cancer (*e.g.*, non-small cell lung cancer). Additionally, refractory or recurrent malignancies can be treated using the antibody molecules described herein.

Examples of other cancers that can be treated include bone cancer, pancreatic cancer, skin cancer, cancer of the head or neck, cutaneous or intraocular malignant melanoma, uterine cancer, ovarian cancer, rectal cancer, anal cancer, gastro-esophageal, stomach cancer, testicular cancer, uterine cancer, carcinoma of the fallopian tubes, carcinoma of the endometrium, carcinoma of the cervix, carcinoma of the vagina, carcinoma of the vulva, Hodgkin Disease, non-Hodgkin lymphoma, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, chronic or acute leukemias including acute myeloid leukemia, chronic myeloid leukemia, acute lymphoblastic leukemia, chronic lymphocytic leukemia, solid tumors of childhood, lymphocytic lymphoma, cancer of the bladder, cancer of the kidney or ureter, carcinoma of the renal pelvis, neoplasm of the central nervous system (CNS), primary CNS lymphoma, tumor angiogenesis, spinal axis tumor, brain stem glioma, pituitary adenoma, Kaposi's sarcoma, epidermoid cancer, squamous cell cancer, T-cell lymphoma, environmentally induced cancers including those induced by asbestos, and combinations of said cancers.

Treatment of metastatic cancers, *e.g.,* metastatic cancers that express PD-L1 (Iwai et al. (2005) Int. Immunol. 17:133-144) can be effected using the antibody molecules described herein. In one embodiment, the cancer expresses an elevated level of PD-L1, IFNγ and /or CD8.

Hematological cancer conditions are the types of cancer such as leukemia and malignant lymphoproliferative conditions that affect blood, bone marrow and the lymphatic system. Leukemia can be classified as acute leukemia and chronic leukemia. Acute leukemia can be further classified as acute myelogenous leukemia (AML) and acute lymphoid leukemia (ALL). Chronic leukemia includes chronic myelogenous leukemia (CML) and chronic lymphoid leukemia (CLL). Other related conditions include myelodysplastic syndromes (MDS, formerly known as "preleukemia") which are a diverse collection of hematological conditions united by ineffective production (or dysplasia) of myeloid blood cells and risk of transformation to AML.

In other embodiments, the cancer is a hematological malignancy or cancer including but is not limited to a leukemia or a lymphoma. For example, the combination therapy can be used to treat cancers and malignancies including, but not limited to, *e.g.,* acute leukemias including but not limited to, *e.g.*, B-cell acute lymphoid leukemia ("BALL"), T-cell acute lymphoid leukemia ("TALL"), acute lymphoid leukemia (ALL); one or more chronic leukemias including but not limited to, *e.g.*, chronic myelogenous leukemia (CML), chronic lymphocytic leukemia (CLL); additional hematologic cancers or hematologic conditions including, but not limited to, *e.g.*, B cell prolymphocytic leukemia, blastic plasmacytoid dendritic cell neoplasm, Burkitt's lymphoma, diffuse large B cell lymphoma, Follicular lymphoma, Hairy cell leukemia, small cell-or a large cell-follicular lymphoma, malignant lymphoproliferative conditions, MALT lymphoma, mantle cell lymphoma, Marginal zone lymphoma, multiple myeloma, myelodysplasia and myelodysplastic syndrome, non-Hodgkin lymphoma, plasmablastic lymphoma, plasmacytoid dendritic cell neoplasm, Waldenstrom macroglobulinemia, and "preleukemia" which are a diverse collection of hematological conditions united by ineffective production (or dysplasia) of myeloid blood cells, and the like. In some embodiments, the lymphoma (*e.g.,* an anaplastic large-cell lymphoma or non-Hodgkin lymphoma) has, or is identified as having, an ALK translocation, *e.g.*, an EML4-ALK fusion.

In one embodiment, the cancer is chosen from a lung cancer (*e.g.*, a non-small cell lung cancer (NSCLC) (*e.g.*, a NSCLC with squamous and/or non-squamous histology)), a melanoma (*e.g.*, an advanced melanoma), a renal cancer (*e.g.*, a renal cell carcinoma, *e.g.*, clear cell renal cell carcinoma), a liver cancer, a myeloma (*e.g.*, a multiple myeloma), a prostate cancer, a breast cancer (*e.g.*, a breast cancer that does not express one, two or all of estrogen receptor, progesterone receptor, or Her2/neu, *e.g.*, a triple negative breast cancer), a colorectal cancer, a pancreatic cancer, a head and neck cancer (*e.g.*, head and neck squamous cell carcinoma (HNSCC), anal cancer, gastro-esophageal cancer, thyroid cancer, cervical cancer, a lymphoproliferative disease (*e.g.*, a post-transplant lymphoproliferative disease) or a hematological cancer, T-cell lymphoma, a non-Hogdkin lymphoma, or a leukemia (*e.g.*, a myeloid leukemia).

In another embodiment, the cancer is chosen form a carcinoma (*e.g.*, advanced or metastatic carcinoma), melanoma or a lung carcinoma, e.g., a non-small cell lung carcinoma.

In one embodiment, the cancer is a lung cancer, *e.g.*, a non-small cell lung cancer (NSCLC). In certain embodiments, the lung cancer, *e.g.*, the non-small cell lung cancer, has, or is identified as having, an ALK rearrangement or translocation, *e.g.*, an ALK fusion, *e.g.*, an EML4-ALK fusion.

In another embodiment, the cancer is an inflammatory myofibroblastic tumor (IMT). In certain embodiments, the inflammatory myofibroblastic tumor has, or is identified as having, an ALK rearrangement or translocation, *e.g.*, an ALK fusion, *e.g.*, an EML4-ALK fusion.

In other embodiments, the cancer is NSCLC wherein the NSCLC is characterized by one or more of: aberrant activation, amplification, or a mutation of epidermal growth factor receptor (EGFR). In certain embodiments the cancer is NSCLC wherein the NSCLC is characterized by harbouring an EGFR exon 20 insertion, an EGFR exon 19 deletion, EGFR L858R mutation, EGFR T790M, or any combination thereof. In some embodiments, the NSCLC is characterized by harboring L858R and T790M mutations of EGFR. In some embodiments, the NSCLC is characterized by harboring an EGFR exon 20 insertion and T790M mutations of EGFR. In some embodiments, the NSCLC is characterized by harboring an EGFR exon 19 deletion and T790M mutations of EGFR. In some embodiments, the NSCLC is characterized by harboring EGFR mutation selected from the group consisting of an exon 20 insertion, an exon 19 deletion, L858R mutation, T790M mutation, and any combination thereof.

In yet another embodiment, the cancer is a neuroblastoma.

In certain embodiments, the neuroblastoma has, or is identified as having, an ALK rearrangement or translocation, *e.g.*, an ALK fusion, *e.g.*, an EML4-ALK fusion. Methods and compositions disclosed herein are useful for treating metastatic lesions associated with the aforementioned cancers.

In another embodiment, the cancer is a hepatocarcinoma, *e.g.*, an advanced hepatocarcinoma, with or without a viral infection, *e.g.*, a chronic viral hepatitis.

In another embodiment, the cancer is a prostate cancer, *e.g.*, an advanced prostate cancer.

In yet another embodiment, the cancer is a myeloma, *e.g.*, multiple myeloma.

In yet another embodiment, the cancer is a renal cancer, *e.g.*, a renal cell carcinoma (RCC) (*e.g.*, a metastatic RCC or clear cell renal cell carcinoma).

In one embodiment, the cancer is a melanoma, *e.g.*, an advanced melanoma. In one embodiment, the cancer is an advanced or unresectable melanoma that does not respond to other therapies. In other embodiments, the cancer is a melanoma with a BRAF mutation (*e.g.*, a BRAF V600 mutation). In yet other embodiments, the anti-PD-1 or PD-L1 antibody molecule is administered after treatment with an anti-CTLA-4 antibody (*e.g.*, ipilimumab) with or without a BRAF inhibitor (*e.g.*, vemurafenib or dabrafenib).

In another embodiment, the cancer is an inflammatory myofibroblastic tumor (IMT). In certain embodiments, the inflammatory myofibroblastic tumor has, or is identified as having, an ALK rearrangement or translocation, *e.g.*, an ALK fusion, *e.g.,* an EML4-ALK fusion.

Methods and compositions disclosed herein are useful for treating metastatic lesions associated with the aforementioned cancers.

### Additional Combination Therapies

The combination therapy disclosed herein can be further co-formulated with, and/or co-administered with, one or more additional therapeutic agents, *e.g.,* one or more anti-cancer agents, cytotoxic or cytostatic agents, hormone treatment, vaccines, and/or other immunotherapies. In other embodiments, the antibody molecules are administered in combination with other therapeutic treatment modalities, including surgery, radiation, cryosurgery, and/or thermotherapy. Such combination therapies may advantageously utilize lower dosages of the administered therapeutic agents, thus avoiding possible toxicities or complications associated with the various monotherapies.

For example, the combination therapies disclosed herein can also be combined with a standard cancer treatment. For example, PD-1 blockade may be effectively combined with chemotherapeutic regimes. In these instances, it may be possible to reduce the dose of chemotherapeutic reagent administered (Mokyr, M. et al. (1998) Cancer Research 58: 5301-5304). In certain embodiments, the methods and compositions described herein are administered in combination with one or more of other antibody molecules, chemotherapy, other anti-cancer therapy (*e.g.*, targeted anti-cancer therapies, or oncolytic drugs), cytotoxic agents, immune-based therapies (*e.g.*, cytokines), surgical and/or radiation procedures. Exemplary cytotoxic agents that can be administered in combination with include antimicrotubule agents, topoisomerase inhibitors, anti-metabolites, mitotic inhibitors, alkylating agents, anthracyclines, vinca alkaloids, intercalating agents, agents capable of interfering with a signal transduction pathway, agents that promote apoptosis, proteosome inhibitors, and radiation (*e.g.*, local or whole body irradiation).

Exemplary combinations of with the standard of care for cancer, include at least the following.

In certain embodiments, the combination therapy, is used in combination with a standard of cancer care chemotherapeutic agent including, but not limited to, anastrozole (Arimidex®), bicalutamide (Casodex®), bleomycin sulfate (Blenoxane®), busulfan (Myleran®), busulfan injection (Busulfex®), capecitabine (Xeloda®), N4-pentoxycarbonyl-5-deoxy-5-fluorocytidine, carboplatin (Paraplatin®), carmustine (BiCNU®), chlorambucil (Leukeran®), cisplatin (Platinol®), cladribine (Leustatin®), cyclophosphamide (Cytoxan® or Neosar®), cytarabine, cytosine arabinoside (Cytosar-U®), cytarabine liposome injection (DepoCyt®), dacarbazine (DTIC-Dome®), dactinomycin (Actinomycin D, Cosmegan), daunorubicin hydrochloride (Cerubidine®), daunorubicin citrate liposome injection (DaunoXome®), dexamethasone, docetaxel (Taxotere®), doxorubicin hydrochloride (Adriamycin®, Rubex®), etoposide (Vepesid®), fludarabine phosphate (Fludara®), 5-fluorouracil (Adrucil®, Efudex®), flutamide (Eulexin®), tezacitibine, Gemcitabine (difluorodeoxycitidine), hydroxyurea (Hydrea®), Idarubicin (Idamycin®), ifosfamide (IFEX®), irinotecan (Camptosar®), L-asparaginase (ELSPAR®), leucovorin calcium, melphalan (Alkeran®), 6-mercaptopurine (Purinethol®), methotrexate (Folex®), mitoxantrone (Novantrone®), mylotarg, paclitaxel (Taxol®), nabpaclitaxel (Abraxane®), phoenix (Yttrium90/MX-DTPA), pentostatin, polifeprosan 20 with carmustine implant (Gliadel®), tamoxifen citrate (Nolvadex®), teniposide (Vumon®), 6-thioguanine, thiotepa, tirapazamine (Tirazone®), topotecan hydrochloride for injection (Hycamptin®), vinblastine (Velban®), vincristine (Oncovin®), and vinorelbine (Navelbine®).

Exemplary alkylating agents include, without limitation, nitrogen mustards, ethylenimine derivatives, alkyl sulfonates, nitrosoureas and triazenes): uracil mustard (Aminouracil Mustard®, Chlorethaminacil®, Demethyldopan®, Desmethyldopan®, Haemanthamine®, Nordopan®, Uracil nitrogen mustard®, Uracillost®, Uracilmostaza®, Uramustin®, Uramustine®), chlormethine (Mustargen®), cyclophosphamide (Cytoxan®, Neosar®, Clafen®, Endoxan®, Procytox®, Revimmune™), ifosfamide (Mitoxana®), melphalan (Alkeran®), Chlorambucil (Leukeran®), pipobroman (Amedel®, Vercyte®), triethylenemelamine (Hemel®, Hexalen®, Hexastat®), triethylenethiophosphoramine, Temozolomide (Temodar®), thiotepa (Thioplex®), busulfan (Busilvex®, Myleran®), carmustine (BiCNU®), lomustine (CeeNU®), streptozocin (Zanosar®), and Dacarbazine (DTIC-Dome®). Additional exemplary alkylating agents include, without limitation, Oxaliplatin (Eloxatin®); Temozolomide (Temodar® and Temodal®); Dactinomycin (also known as actinomycin-D, Cosmegen®); Melphalan (also known as L-PAM, L-sarcolysin, and phenylalanine mustard, Alkeran®); Altretamine (also known as hexamethylmelamine (HMM), Hexalen®); Carmustine (BiCNU®); Bendamustine (Treanda®); Busulfan (Busulfex® and Myleran®); Carboplatin (Paraplatin®); Lomustine (also known as CCNU, CeeNU®); Cisplatin (also known as CDDP, Platinol® and Platinol®-AQ); Chlorambucil (Leukeran®); Cyclophosphamide (Cytoxan® and Neosar®); Dacarbazine (also known as DTIC, DIC and imidazole carboxamide, DTIC-Dome®); Altretamine (also known as hexamethylmelamine (HMM), Hexalen®); Ifosfamide (Ifex®); Prednumustine; Procarbazine (Matulane®); Mechlorethamine (also known as nitrogen mustard, mustine and mechloroethamine hydrochloride, Mustargen®); Streptozocin (Zanosar®); Thiotepa (also known as thiophosphoamide, TESPA and TSPA, Thioplex®); Cyclophosphamide (Endoxan®, Cytoxan®, Neosar®, Procytox®, Revimmune®); and Bendamustine HCl (Treanda®).

Exemplary anthracyclines include, e.g., doxorubicin (Adriamycin® and Rubex®); bleomycin (lenoxane®); daunorubicin (dauorubicin hydrochloride, daunomycin, and rubidomycin hydrochloride, Cerubidine®); daunorubicin liposomal (daunorubicin citrate liposome, DaunoXome®); mitoxantrone (DHAD, Novantrone®); epirubicin (Ellence™); idarubicin (Idamycin®, Idamycin PFS®); mitomycin C (Mutamycin®); geldanamycin; herbimycin; ravidomycin; and desacetylravidomycin.

Exemplary vinca alkaloids that can be used in combination with a combination therapy disclosed herein *(e.g.,* an anti-PD-1 or PD-L1 antibody molecule, alone or in combination with another immunomodulator (*e.g.*, an anti-LAG-3, or anti-TIM-3 antibody molecule) and a compound of Table 1), include, but are not limited to, vinorelbine tartrate (Navelbine®), Vincristine (Oncovin®), and Vindesine (Eldisine®)); vinblastine (also known as vinblastine sulfate, vincaleukoblastine and VLB, Alkaban-AQ® and Velban®); and vinorelbine (Navelbine®).

Exemplary proteosome inhibitors that can be used in combination with combination therapy disclosed herein (*e.g.,* an anti-PD-1 or PD-L1 antibody molecule, alone or in combination with another immunomodulator (*e.g.*, an anti-LAG-3, or anti-TIM-3 antibody molecule) and a compound of Table 1), include, but are not limited to, bortezomib (Velcade®); carfilzomib (PX-171-007, (*S*)-4-Methyl-*N*-((*S*)-1-(((*S*)-4-methyl-1-((*R*)-2-methyloxiran-2-yl)-1-oxopentan-2-yl)amino)-1-oxo-3-phenylpropan-2-yl)-2-((*S*)-2-(2-morpholinoacetamido)-4-phenylbutanamido)-pentanamide); marizomib (NPI-0052); ixazomib citrate (MLN-9708); delanzomib (CEP-18770); O-Methyl-*N*-[(2-methyl-5-thiazolyl)carbonyl]-L-seryl-O-methyl-*N-*[(1*S*)-2-[(2*R*)-2-methyl-2-oxiranyl]-2-oxo-1-(phenylmethyl)ethyl]- L-serinamide (ONX-0912); danoprevir (RG7227, CAS 850876-88-9); ixazomib (MLN2238, CAS 1072833-77-2); and (S)-N-[(phenylmethoxy)carbonyl]-L-leucyl-N-(1-formyl-3-methylbutyl)- L-Leucinamide (MG-132, CAS 133407-82-6).

In some aspects, the combination therapy disclosed herein (*e.g.*, an anti-PD-1 or PD-L1 antibody molecule, alone or in combination with another immunomodulator (*e.g.*, an anti-LAG-3, or anti-TIM-3 antibody molecule) and a compound of Table 1), in combination with a tyrosine kinase inhibitor (*e.g.*, a receptor tyrosine kinase (RTK) inhibitor). Exemplary tyrosine kinase inhibitor include, but are not limited to, an epidermal growth factor (EGF) pathway inhibitor (*e.g.*, an epidermal growth factor receptor (EGFR) inhibitor), a vascular endothelial growth factor (VEGF) pathway inhibitor (*e.g.*, a vascular endothelial growth factor receptor (VEGFR) inhibitor (*e.g.*, a VEGFR-1 inhibitor, a VEGFR-2 inhibitor, a VEGFR-3 inhibitor)), a platelet derived growth factor (PDGF) pathway inhibitor (*e.g.*, a platelet derived growth factor receptor (PDGFR) inhibitor (*e*.*g*., a PDGFR-β inhibitor)), a RAF-1 inhibitor, a KIT inhibitor and a RET inhibitor. In some embodiments, the anti-cancer agent used in combination with the hedgehog inhibitor is selected from the group consisting of: axitinib (AG013736), bosutinib (SKI-606), cediranib (RECENTIN™, AZD2171), dasatinib (SPRYCEL®, BMS-354825), erlotinib (TARCEVA®), gefitinib (IRESSA®), imatinib (Gleevec®, CGP57148B, STI-571), lapatinib (TYKERB®, TYVERB®), lestaurtinib (CEP-701), neratinib (HKI-272), nilotinib (TASIGNA®), semaxanib (semaxinib, SU5416), sunitinib (SUTENT®, SU11248), toceranib (PALLADIA®), vandetanib (ZACTIMA®, ZD6474), vatalanib (PTK787, PTK/ZK), trastuzumab (HERCEPTIN®), bevacizumab (AVASTIN®), rituximab (RITUXAN®), cetuximab (ERBITUX®), panitumumab (VECTIBIX®), ranibizumab (Lucentis®), nilotinib (TASIGNA®), sorafenib (NEXAVAR®), alemtuzumab (CAMPATH®), gemtuzumab ozogamicin (MYLOTARG®), ENMD-2076, PCI-32765, AC220, dovitinib lactate (TKI258, CHIR-258), BIBW 2992 (TOVOK™), SGX523, PF-04217903, PF-02341066, PF-299804, BMS-777607, ABT-869, MP470, BIBF 1120 (VARGATEF®), AP24534, JNJ-26483327, MGCD265, DCC-2036, BMS-690154, CEP-11981, tivozanib (AV-951), OSI-930, MM-121, XL-184, XL-647, XL228, AEE788, AG-490, AST-6, BMS-599626, CUDC-101, PD153035, pelitinib (EKB-569), vandetanib (zactima), WZ3146, WZ4002, WZ8040, ABT-869 (linifanib), AEE788, AP24534 (ponatinib), AV-951(tivozanib), axitinib, BAY 73-4506 (regorafenib), brivanib alaninate (BMS-582664), brivanib (BMS-540215), cediranib (AZD2171), CHIR-258 (dovitinib), CP 673451, CYC116, E7080, Ki8751, masitinib (AB1010), MGCD-265, motesanib diphosphate (AMG-706), MP-470, OSI-930, Pazopanib Hydrochloride, PD173074, Sorafenib Tosylate(Bay 43-9006), SU 5402, TSU-68(SU6668), vatalanib, XL880 (GSK1363089, EXEL-2880). Further examples of hedgehog inhibitors include, but are not limited to,vismodegib (2-chloro-N-[4-chloro-3-(2-pyridinyl)phenyl]-4-(methylsulfonyl)- benzamide, GDC-0449, described in PCT Publication No. WO 06/028958); 1-(4-Chloro-3-(trifluoromethyl)phenyl)-3-((3-(4-fluorophenyl)-3,4-dihydro-4-oxo-2-quinazolinyl)methyl)-urea (CAS 330796-24-2); N-[(2S,3R,3'R,3aS,4'aR,6S,6'aR,6'bS,7aR,12'aS,12'bS)-2',3',3a,4,4',4'a,5,5',6,6',6'a,6'b,7,7',7a,8',10',12',12'a,12'b-Eicosahydro-3,6,11',12'b-tetramethylspiro[furo[3,2-b]pyridine-2(3H),9'(1'H)-naphth[2,1-a]azulen]-3'-yl]-methanesulfonamide (IPI926, CAS 1037210-93-7); and 4-Fluoro-N-methyl-N-[1-[4-(1-methyl-1H-pyrazol-5-yl)-1-phthalazinyl]-4-piperidinyl]-2-(trifluoromethyl)-benzamide (LY2940680, CAS 1258861-20-9); and Erismodegib (LDE225). Selected tyrosine kinase inhibitors are chosen from sunitinib, erlotinib, gefitinib;, or sorafenib erlotinib hydrochloride (Tarceva®); linifanib (N-[4-(3-amino-1H-indazol-4-yl)phenyl]-N'-(2-fluoro-5-methylphenyl)urea, also known as ABT 869, available from Genentech); sunitinib malate (Sutent®); bosutinib (4-[(2,4-dichloro-5-methoxyphenyl)amino]-6-methoxy-7-[3-(4-methylpiperazin-1-yl)propoxy]quinoline-3-carbonitrile, also known as SKI-606, described in US Patent No. 6,780,996); dasatinib (Sprycel®); pazopanib (Votrient®); sorafenib (Nexavar®); zactima (ZD6474); and imatinib or imatinib mesylate (Gilvec® and Gleevec®).

In certain aspects, the combination therapy disclosed herein (*e.g.*, an anti-PD-1 or PD-L1 antibody molecule, alone or in combination with another immunomodulator (*e.g.*, an anti-LAG-3, or anti-TIM-3 antibody molecule) and a compound of Table 1), in combination with a Vascular Endothelial Growth Factor (VEGF) receptor inhibitors, including but not limited to, Bevacizumab (Avastin®), axitinib (Inlyta®); Brivanib alaninate (BMS-582664, (*S*)*-*((*R*)-1-(4-(4-Fluoro-2-methyl-1*H*-indol-5-yloxy)-5-methylpyrrolo[2,1-*f*][1,2,4]triazin-6-yloxy)propan-2-yl)2-aminopropanoate); Sorafenib (Nexavar®); Pazopanib (Votrient®); Sunitinib malate (Sutent®); Cediranib (AZD2171, CAS 288383-20-1); Vargatef (BIBF1120, CAS 928326-83-4); Foretinib (GSK1363089); Telatinib (BAY57-9352, CAS 332012-40-5); Apatinib (YN968D1, CAS 811803-05-1); Imatinib (Gleevec®); Ponatinib (AP24534, CAS 943319-70-8); Tivozanib (AV951, CAS 475108-18-0); Regorafenib (BAY73-4506, CAS 755037-03-7); Vatalanib dihydrochloride (PTK787, CAS 212141-51-0); Brivanib (BMS-540215, CAS 649735-46-6); Vandetanib (Caprelsa® or AZD6474); Motesanib diphosphate (AMG706, CAS 857876-30-3, N-(2,3-dihydro-3,3-dimethyl-1H-indol-6-yl)-2-[(4-pyridinylmethyl)amino]-3-pyridinecarboxamide, described in PCT Publication No. WO 02/066470); Dovitinib dilactic acid (TKI258, CAS 852433-84-2); Linfanib (ABT869, CAS 796967-16-3); Cabozantinib (XL184, CAS 849217-68-1); Lestaurtinib (CAS 111358-88-4); N-[5-[[[5-(1,1-Dimethylethyl)-2-oxazolyl]methyl]thio]-2-thiazolyl]-4-piperidinecarboxamide (BMS38703, CAS 345627-80-7); (3R,4R)-4-Amino-1-((4-((3-methoxyphenyl)amino)pyrrolo[2,1-f][1,2,4]triazin-5-yl)methyl)piperidin-3-ol (BMS690514); *N*-(3,4-Dichloro-2-fluorophenyl)-6-methoxy-7-[[(3aα,5β,6aα)-octahydro-2-methylcyclopenta[*c*]pyrrol-5-yl]methoxy]-4-quinazolinamine (XL647, CAS 781613-23-8); 4-Methyl-3-[[1-methyl-6-(3-pyridinyl)-1*H*-pyrazolo[3,4-*d*]pyrimidin-4-yl]amino]-*N*-[3-(trifluoromethyl)phenyl]-benzamide (BHG712, CAS 940310-85-0); and Aflibercept (Eylea®).

Exemplary anti-VEGF antibodies include, but are not limited to, a monoclonal antibody that binds to the same epitope as the monoclonal anti-VEGF antibody A4.6.1 produced by hybridoma ATCC HB 10709; a recombinant humanized anti-VEGF monoclonal antibody generated according to Presta et al. (1997) Cancer Res. 57:4593-4599. In one embodiment, the anti-VEGF antibody is Bevacizumab (BV), also known as rhuMAb VEGF or AVASTIN®. It comprises mutated human IgGl framework regions and antigen-binding complementarity-determining regions from the murine anti-hVEGF monoclonal antibody A.4.6.1 that blocks binding of human VEGF to its receptors. Bevacizumab and other humanized anti-VEGF antibodies are further described in U.S. Pat. No. 6,884,879 issued Feb. 26, 2005. Additional antibodies include the G6 or B20 series antibodies (*e.g.*, G6-31, B20-4.1), as described in PCT Publication No. WO2005/012359, PCT Publication No. WO2005/044853. For additional antibodies see U.S. Pat. Nos. 7,060,269, 6,582,959, 6,703,020, 6,054,297, WO98/45332, WO 96/30046, WO94/10202, EP 0666868B1, U.S. Patent Application Publication Nos. 2006009360, 20050186208, 20030206899, 20030190317, 20030203409, and 20050112126; and Popkov et al, Journal of Immunological Methods 288: 149-164 (2004). Other antibodies include those that bind to a functional epitope on human VEGF comprising of residues F17, M18, D19, Y21, Y25, Q89, 191 , Kl 01, El 03, and C104 or, alternatively, comprising residues F17, Y21, Q22, Y25, D63, 183 and Q89.

In some aspects, the combination therapy disclosed herein (*e.g.*, an anti-PD-1 or PD-L1 antibody molecule, alone or in combination with another immunomodulator (*e.g.*, an anti-LAG-3, or anti-TIM-3 antibody molecule) and a compound of Table 1), in combination with a PI3K inhibitor. In one embodiment, the PI3K inhibitor is an inhibitor of delta and gamma isoforms of PI3K. Exemplary PI3K inhibitors that can be used in combination are described in, *e.g*., WO 2010/036380, WO 2010/006086, WO 09/114870, WO 05/113556, GSK 2126458, GDC-0980, GDC-0941, Sanofi XL147, XL756, XL147, PF-46915032, BKM 120, CAL-101, CAL 263, SF1126, PX-886, and a dual PI3K inhibitor (*e.g.*, Novartis BEZ235). Further examples of PI3K inhibitors include, but are not limited to, 4-[2-(1H-Indazol-4-yl)-6-[[4-(methylsulfonyl)piperazin-1-yl]methyl]thieno[3,2-d]pyrimidin-4-yl]morpholine (also known as GDC 0941, described in PCT Publication Nos. WO 09/036082 and WO 09/055730); 2-Methyl-2-[4-[3-methyl-2-oxo-8-(quinolin-3-yl)-2,3-dihydroimidazo[4,5-c]quinolin-1-yl]phenyl]propionitrile (also known as BEZ235 or NVP-BEZ 235, described in PCT Publication No. WO 06/122806); 4-(trifluoromethyl)-5-(2,6-dimorpholinopyrimidin-4-yl)pyridin-2-amine (also known as BKM120 or NVP-BKM120, described in PCT Publication No. WO2007/084786); Tozasertib (VX680 or MK-0457, CAS 639089-54-6); (5Z)-5-[[4-(4-Pyridinyl)-6-quinolinyl]methylene]-2,4-thiazolidinedione (GSK1059615, CAS 958852-01-2); (1E,4S,4aR,5R,6aS,9aR)-5-(Acetyloxy)-1-[(di-2-propenylamino)methylene]-4,4a,5,6,6a,8,9,9a-octahydro-11-hydroxy-4-(methoxymethyl)-4a,6a-dimethyl-cyclopenta[5,6]naphtho[1,2-c]pyran-2,7,10(1H)-trione (PX866, CAS 502632-66-8); 8-Phenyl-2-(morpholin-4-yl)-chromen-4-one (LY294002, CAS 154447-36-6); 2-Amino-8-ethyl-4-methyl-6-(1H-pyrazol-5-yl)pyrido[2,3-d]pyrimidin-7(8H)-one (SAR 245409 or XL 765); 1,3-Dihydro-8-(6-methoxy-3-pyridinyl)-3-methyl-1-[4-(1-piperazinyl)-3-(trifluoromethyl)phenyl]-2H-imidazo[4,5-c]quinolin-2-one,, (2Z)-2-butenedioate (1:1) (BGT 226); 5-Fluoro-3-phenyl-2-[(1S)-1-(9H-purin-6-ylamino)ethyl]-4(3H)-quinazolinone (CAL101); 2-Amino-N-[3-[N-[3-[(2-chloro-5-methoxyphenyl)amino]quinoxalin-2-yl]sulfamoyl]phenyl]-2-methylpropanamide (SAR 245408 or XL 147); and (S)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) (BYL719).

In some aspects, the combination therapy disclosed herein (*e.g.*, an anti-PD-1 or PD-L1 antibody molecule, alone or in combination with another immunomodulator (*e.g.*, an anti-LAG-3, or anti-TIM-3 antibody molecule) and a compound of Table 1), in combination with a mTOR inhibitor, *e.g.*, one or more mTOR inhibitors chosen from one or more of rapamycin, temsirolimus (TORISEL®), AZD8055, BEZ235, BGT226, XL765, PF-4691502, GDC0980, SF1126, OSI-027, GSK1059615, KU-0063794, WYE-354, Palomid 529 (P529), PF-04691502, or PKI-587. ridaforolimus (formally known as deferolimus, (1*R*,2*R*,4*S*)-4-[(2*R*)-2 [(1*R*,9*S*,12*S*,15*R*,16*E*,18*R*,19*R*,21*R*, 23*S*,24*E*,26*E*,28*Z*,30*S*,32*S*,35*R*)-1,18-dihydroxy-19,30-dimethoxy-15,17,21,23, 29,35-hexamethyl-2,3,10,14,20-pentaoxo-11,36-dioxa-4-azatricyclo[30.3.1.0^{4,9}] hexatriaconta-16,24,26,28-tetraen-12-yl]propyl]-2-methoxycyclohexyl dimethylphosphinate, also known as AP23573 and MK8669, and described in PCT Publication No. WO 03/064383); everolimus (Afinitor® or RAD001); rapamycin (AY22989, Sirolimus®); simapimod (CAS 164301-51-3); emsirolimus, (5-{2,4-Bis[(3S)-3-methylmorpholin-4-yl]pyrido[2,3-*d*]pyrimidin-7-yl}-2-methoxyphenyl)methanol (AZD8055); 2-Amino-8-[*trans*-4-(2-hydroxyethoxy)cyclohexyl]-6-(6-methoxy-3-pyridinyl)-4-methyl-pyrido[2,3-*d*]pyrimidin-7(8*H*)-one (PF04691502, CAS 1013101-36-4); and *N*²-[1,4-dioxo-4-[[4-(4-oxo-8-phenyl-4*H*-1-benzopyran-2-yl)morpholinium-4-yl]methoxy]butyl]-L-arginylglycyl-L-α-aspartylL-serine-, inner salt (SF1126, CAS 936487-67-1), (1r,4r)-4-(4-amino-5-(7-methoxy-1H-indol-2-yl)imidazo[1,5-f][1,2,4]triazin-7-yl)cyclohexanecarboxylic acid (OSI-027); and XL765.

In some aspects, the combination therapy disclosed herein (*e.g.*, an anti-PD-1 or PD-L1 antibody molecule, alone or in combination with another immunomodulator (*e.g.*, an anti-LAG-3, or anti-TIM-3 antibody molecule) and a compound of Table 1), in combination with a BRAF inhibitor, e.g., GSK2118436, RG7204, PLX4032, GDC-0879, PLX4720, and sorafenib tosylate (Bay 43-9006). In further embodiments, a BRAF inhibitor includes, but is not limited to, regorafenib (BAY73-4506, CAS 755037-03-7); tuvizanib (AV951, CAS 475108-18-0); vemurafenib (Zelboraf®, PLX-4032, CAS 918504-65-1); encorafenib (also known as LGX818); 1-Methyl-5-[[2-[5-(trifluoromethyl)-1H-imidazol-2-yl]-4-pyridinyl]oxy]-N-[4-(trifluoromethyl)phenyl-1H-benzimidazol-2-amine (RAF265, CAS 927880-90-8); 5-[1-(2-Hydroxyethyl)-3-(pyridin-4-yl)-1H-pyrazol-4-yl]-2,3-dihydroinden-1-one oxime (GDC-0879, CAS 905281-76-7); 5-[2-[4-[2-(Dimethylamino)ethoxy]phenyl]-5-(4-pyridinyl)-1H-imidazol-4-yl]-2,3-dihydro-1H-Inden-1-one oxime (GSK2118436 or SB590885); (+/-)-Methyl (5-(2-(5-chloro-2-methylphenyl)-1-hydroxy-3-oxo-2,3-dihydro-1H-isoindol-1-yl)-1H-benzimidazol-2-yl)carbamate (also known as XL-281 and BMS908662) and N-(3-(5-chloro-1H-pyrrolo[2,3-b]pyridine-3-carbonyl)-2,4-difluorophenyl)propane-1-sulfonamide (also known as PLX4720).

In some aspects, the combination therapy disclosed herein (*e.g.*, an anti-PD-1 or PD-L1 antibody molecule, alone or in combination with another immunomodulator (*e.g.*, an anti-LAG-3, or anti-TIM-3 antibody molecule) and a compound of Table 1), in combination with a MEK inhibitor. In some embodiments, the combination of the anti-PD-1 antibody and the MEK inhibitor is used to treat a cancer *(e.g.,* a cancer described herein). In some embodiments, the cancer treated with the combination is chosen from a melanoma, a colorectal cancer, a non-small cell lung cancer, an ovarian cancer, a breast cancer, a prostate cancer, a pancreatic cancer, a hematological malignancy or a renal cell carcinoma. In certain embodiments, the cancer includes a BRAF mutation (*e.g.*, a BRAF V600E mutation), a BRAF wildtype, a KRAS wildtype or an activating KRAS mutation. The cancer may be at an early, intermediate or late stage. Any MEK inhibitor can be used in combination including, but not limited to, selumetinib (5-[(4-bromo-2-chlorophenyl)amino]-4-fluoro-N-(2-hydroxyethoxy)-1-methyl-1H-benzimidazole-6-carboxamide, also known as AZD6244 or ARRY 142886, described in PCT Publication No. WO2003077914);ARRY-142886 trametinib dimethyl sulfoxide (GSK-1120212, CAS 1204531-25-80);, G02442104 (also known as GSK1120212), RDEA436, ; N-[3,4-Difluoro-2-[(2-fluoro-4-iodophenyl)amino]-6-methoxyphenyl]-1-[(2R)-2,3-dihydroxypropyl]-cyclopropanesulfonamide (also known as RDEA119 or BAY869766, described in PCT Publication No. WO2007014011); RDEA119/BAY 869766, AS703026, ; G00039805 (also known as AZD-6244 or selumetinib), BIX 02188, ; BIX 02189, ; 2-[(2-Chloro-4-iodophenyl)amino]-N-(cyclopropylmethoxy)-3,4-difluoro-benzamide (also known as CI-1040 or PD184352, described in PCT Publication No. WO2000035436);CI-1040 (PD-184352), N-[(2R)-2,3-Dihydroxypropoxy]-3,4-difluoro-2-[(2-fluoro-4-iodophenyl)amino]- benzamide (also known as PD0325901 and described in PCT Publication No. WO2002006213); PD03259012'-amino-3'-methoxyflavone (also known as PD98059 available from Biaffin GmbH & Co., KG, Germany);, PD98059, 2,3-bis[amino[(2-aminophenyl)thio]methylene]-butanedinitrile (also known as U0126 and described in US Patent No. 2,779,780);U0126, XL-518 (also known as GDC-0973, Cas No. 1029872-29-4, available from ACC Corp.);GDC-0973 (Methanone, [3,4-difluoro-2-[(2-fluoro-4-iodophenyl)amino]phenyl][3- hydroxy-3-(25)-2-piperidinyl- 1 -azetidinyl]-), G-38963, ; and G02443714 (also known as AS703206), or a pharmaceutically acceptable salt or solvate thereof..Additional examples of MEK inhibitors are disclosed in WO 2013/019906, WO 03/077914, WO 2005/121142, WO 2007/04415, WO 2008/024725 and WO 2009/085983. Further examples of MEK inhibitors include, but are not limited to, benimetinib (6-(4-bromo-2-fluorophenylamino)-7-fluoro-3-methyl-3H-benzoimidazole-5-carboxylic acid (2-hydroxyethyoxy)-amide, also known as MEK162, CAS 1073666-70-2, described in PCT Publication No. WO2003077914); 2,3-Bis[amino[(2-aminophenyl)thio]methylene]-butanedinitrile (also known as U0126 and described in US Patent No. 2,779,780); (3S,4R,5Z,8S,9S,11E)-14-(Ethylamino)-8,9,16-trihydroxy-3,4-dimethyl-3,4,9,19-tetrahydro-1H-2-benzoxacyclotetradecine-1,7(8H)-dione] (also known as E6201, described in PCT Publication No. WO2003076424); vemurafenib (PLX-4032, CAS 918504-65-1); (R)-3-(2,3-Dihydroxypropyl)-6-fluoro-5-(2-fluoro-4-iodophenylamino)-8-methylpyrido[2,3-d]pyrimidine-4,7(3H,8H)-dione (TAK-733, CAS 1035555-63-5); pimasertib (AS-703026, CAS 1204531-26-9); 2-(2-Fluoro-4-iodophenylamino)-N-(2-hydroxyethoxy)-1,5-dimethyl-6-oxo-1,6-dihydropyridine-3-carboxamide (AZD 8330); and 3,4-Difluoro-2-[(2-fluoro-4-iodophenyl)amino]-N-(2-hydroxyethoxy)-5-[(3-oxo-[1,2]oxazinan-2-yl)methyl]benzamide (CH 4987655 or Ro 4987655).

In some aspects, the combination therapy disclosed herein (*e.g.*, an anti-PD-1 or PD-L1 antibody molecule, alone or in combination with another immunomodulator (*e.g.*, an anti-LAG-3, or anti-TIM-3 antibody molecule) and a compound of Table 1), in combination with a JAK2 inhibitor, *e.g.*, CEP-701, INCB18424, CP-690550 (tasocitinib). Exemplary JAK inhibitors include, but are not limited to, ruxolitinib (Jakafi®); tofacitinib (CP690550); axitinib (AG013736, CAS 319460-85-0); 5-Chloro-N2-[(1S)-1-(5-fluoro-2-pyrimidinyl)ethyl]-N4-(5-methyl-1H-pyrazol-3-y)-12,4-pyrimidinediamine (AZD1480, CAS 935666-88-9); (9E)-15-[2-(1-Pyrrolidinyl)ethoxy]- 7,12,26-trioxa-19,21,24-triazatetracyclo[18.3.1.12,5.114,18]-hexacosa-1(24),2,4,9,14,16,18(25),20,22-nonaene (SB-1578, CAS 937273-04-6); momelotinib (CYT 387); baricitinib (INCB-028050 or LY-3009104); pacritinib (SB1518); (16E)-14-Methyl-20-oxa-5,7,14,27-tetraazatetracyclo[19.3.1.12,6.18,12]heptacosa-1(25),2,4,6(27),8,10,12(26),16,21,23-decaene (SB 1317); gandotinib (LY 2784544); and N,N-cicyclopropyl-4-[(1,5-dimethyl-1H-pyrazol-3-yl)amino]-6-ethyl-1,6-dihydro-1-methyl-imidazo[4,5-d]pyrrolo[2,3-b]pyridine-7-carboxamide (BMS 911543).

In some embodiments, the combination therapies disclosed herein include paclitaxel or a paclitaxel agent, *e.g.*, TAXOL®, protein-bound paclitaxel (*e.g.*, ABRAXANE®). Exemplary paclitaxel agents include, but are not limited to, nanoparticle albumin-bound paclitaxel (ABRAXANE, marketed by Abraxis Bioscience), docosahexaenoic acid bound-paclitaxel (DHA-paclitaxel, Taxoprexin, marketed by Protarga), polyglutamate bound-paclitaxel (PG-paclitaxel, paclitaxel poliglumex, CT-2103, XYOTAX, marketed by Cell Therapeutic), the tumor-activated prodrug (TAP), ANG105 (Angiopep-2 bound to three molecules of paclitaxel, marketed by ImmunoGen), paclitaxel-EC-1 (paclitaxel bound to the erbB2-recognizing peptide EC-1; *see* Li et al., Biopolymers (2007) 87:225-230), and glucose-conjugated paclitaxel (*e.g.*, 2'-paclitaxel methyl 2-glucopyranosyl succinate, *see* Liu et al., Bioorganic & Medicinal Chemistry Letters (2007) 17:617-620).

In certain aspects, the anti-PD-1 or PD-L1 antibody molecule, alone or in combination with another immunomodulator (*e.g.*, an anti-LAG-3 or anti-TIM-3 antibody molecule), is administered in combination with an antibody against a Killer-cell Immunoglobulin-like Receptors (also referred to herein as an "anti-KIR antibody"). In certain embodiments, the combination of anti-PD-1 antibody molecule and anti-KIR antibody described herein is used to treat a cancer, *e.g.*, a cancer as described herein (*e.g.*, a solid tumor, *e.g.*, an advanced solid tumor).

In one aspect, the anti-PD-1 or PD-L1 antibody molecule, alone or in combination with another immunomodulator (*e.g.*, an anti-LAG-3 or anti-TIM-3 antibody molecule), is administered in combination with a cellular immunotherapy (*e.g.*, Provenge (*e.g.*, Sipuleucel)), and optionally in combination with cyclophosphamide. In certain embodiments, the combination of anti-PD-1 antibody molecule, Provenge and/or cyclophosphamide is used to treat a cancer, *e.g.*, a cancer as described herein (*e.g.*, a prostate cancer, *e.g.*, an advanced prostate cancer).

In another aspect, the anti-PD-1 or PD-L1 antibody molecule, alone or in combination with another immunomodulator (*e.g.*, an anti-LAG-3 or anti-TIM-3 antibody molecule), is administered in combination with a vaccine, *e.g.*, a dendritic cell renal carcinoma (DC-RCC) vaccine. In certain embodiments, the combination of anti-PD-1 antibody molecule and the DC-RCC vaccine is used to treat a cancer, *e.g.,* a cancer as described herein (*e.g.*, a renal carcinoma, *e.g.*, metastatic renal cell carcinoma (RCC) or clear cell renal cell carcinoma (CCRCC)).

In yet another aspect, the anti-PD-1 or PD-L1 antibody molecule, alone or in combination with another immunomodulator (*e.g.*, an anti-LAG-3 or anti-TIM-3 antibody molecule), is administered in combination with chemotherapy, and/or immunotherapy. For example, the anti-PD-1 or PD-L1 antibody molecule can be used to treat a myeloma, alone or in combination with one or more of: chemotherapy or other anti-cancer agents (*e.g.*, thalidomide analogs, *e.g.*, lenalidomide), an anti-TIM-3 antibody, tumor antigen-pulsed dendritic cells, fusions (*e.g.*, electrofusions) of tumor cells and dendritic cells, or vaccination with immunoglobulin idiotype produced by malignant plasma cells. In one aspect, the anti-PD-1 or PD-L1 antibody molecule is used in combination with an anti-TIM-3 antibody to treat a myeloma, *e.g.*, a multiple myeloma.

In one aspect, the anti-PD-1 or PD-L1 antibody molecule, alone or in combination with another immunomodulator (*e.g.*, an anti-LAG-3 or anti-TIM-3 antibody molecule), is used in combination with chemotherapy to treat a lung cancer, *e.g.*, non-small cell lung cancer. In one aspect, the anti-PD-1 or PD-L1 antibody molecule is used with platinum doublet therapy to treat lung cancer.

In yet another aspect, the anti-PD-1 or PD-L1 antibody molecule, alone or in combination with another immunomodulator (*e.g.*, an anti-LAG-3 or anti-TIM-3 antibody molecule), is used to treat a renal cancer, *e.g.*, renal cell carcinoma (RCC) (*e.g.*, clear cell renal cell carcinoma (CCRCC) or metastatic RCC. The anti-PD-1 or PD-L1 antibody molecule can be administered in combination with one or more of: an immune-based strategy (*e.g.*, interleukin-2 or interferon-a), a targeted agent (*e.g.*, a VEGF inhibitor such as a monoclonal antibody to VEGF); a VEGF tyrosine kinase inhibitor such as sunitinib, sorafenib, axitinib and pazopanib; an RNAi inhibitor), or an inhibitor of a downstream mediator of VEGF signaling, *e.g.*, an inhibitor of the mammalian target of rapamycin (mTOR), *e.g.*, everolimus and temsirolimus.

An example of suitable therapeutics for use in combination for treatment of pancreatic cancer includes, but is not limited to, a chemotherapeutic agent, *e.g.*, paclitaxel or a paclitaxel agent (*e.g.*, a paclitaxel formulation such as TAXOL, an albumin-stabilized nanoparticle paclitaxel formulation (*e.g.*, ABRAXANE) or a liposomal paclitaxel formulation); gemcitabine (*e.g.*, gemcitabine alone or in combination with AXP107-11); other chemotherapeutic agents such as oxaliplatin, 5-fluorouracil, capecitabine, rubitecan, epirubicin hydrochloride, NC-6004, cisplatin, docetaxel (*e.g.*, TAXOTERE), mitomycin C, ifosfamide; interferon; tyrosine kinase inhibitor (*e.g.*, EGFR inhibitor (*e.g.*, erlotinib, panitumumab, cetuximab, nimotuzumab); HER2/neu receptor inhibitor (*e.g.*, trastuzumab); dual kinase inhibitor (*e.g.*, bosutinib, saracatinib, lapatinib, vandetanib); multikinase inhibitor (*e.g.*, sorafenib, sunitinib, XL184, pazopanib); VEGF inhibitor (*e.g.*, bevacizumab, AV-951, brivanib); radio immunotherapy (*e.g.*, XR303); cancer vaccine (*e.g.,* GVAX, survivin peptide); COX-2 inhibitor (*e.g.,* celecoxib); IGF-1 receptor inhibitor (*e.g.,* AMG 479, MK-0646); mTOR inhibitor (*e.g.,* everolimus, temsirolimus); IL-6 inhibitor (*e.g.,* CNTO 328); cyclin-dependent kinase inhibitor *(e.g.,* P276-00, UCN-01); Altered Energy Metabolism-Directed (AEMD) compound (*e.g.*, CPI-613); HDAC inhibitor (*e.g.*, vorinostat); TRAIL receptor 2 (TR-2) agonist (*e.g.*, conatumumab); MEK inhibitor (*e.g.*, AS703026, selumetinib, GSK1120212); Raf/MEK dual kinase inhibitor (*e.g.*, RO5126766); Notch signaling inhibitor (*e.g.*, MK0752); monoclonal antibody-antibody fusion protein (*e.g.*, L19IL2); curcumin; HSP90 inhibitor (*e.g.*, tanespimycin, STA-9090); rIL-2;, denileukin diftitox; topoisomerase 1 inhibitor (*e.g.*, irinotecan, PEP02); statin (*e.g.*, simvastatin); Factor VIIa inhibitor (*e.g.,* PCI-27483); AKT inhibitor (*e.g.,* RX-0201); hypoxia-activated prodrug (*e.g.*, TH-302); metformin hydrochloride, gamma-secretase inhibitor (*e.g.*, RO4929097); ribonucleotide reductase inhibitor (*e.g.*, 3-AP); immunotoxin (*e.g.*, HuC242-DM4); PARP inhibitor (*e.g.*, KU-0059436, veliparib); CTLA-4 inhbitor (*e.g.*, CP-675,206, ipilimumab); AdVtk therapy; proteasome inhibitor (*e.g.*, bortezomib (Velcade), NPI-0052); thiazolidinedione (*e.g.*, pioglitazone); NPC-1C; Aurora kinase inhibitor (*e.g.*, R763/AS703569), CTGF inhibitor (*e.g.*, FG-3019); siG12D LODER; and radiation therapy (*e.g.*, tomotherapy, stereotactic radiation, proton therapy), surgery, and a combination thereof. In certain embodiments, a combination of paclitaxel or a paclitaxel agent, and gemcitabine can be used with the anti-PD-1 antibody molecules described herein.

An example of suitable therapeutics for use in combination for treatment of small cell lung cancer includes, but is not limited to, a chemotherapeutic agent, *e.g.*, etoposide, carboplatin, cisplatin, irinotecan, topotecan, gemcitabine, liposomal SN-38, bendamustine, temozolomide, belotecan, NK012, FR901228, flavopiridol); tyrosine kinase inhibitor (*e.g.*, EGFR inhibitor (*e.g.*, erlotinib, gefitinib, cetuximab, panitumumab); multikinase inhibitor (*e.g.*, sorafenib, sunitinib); VEGF inhibitor (*e.g.*, bevacizumab, vandetanib); cancer vaccine (*e.g.*, GVAX); Bcl-2 inhibitor (*e.g.*, oblimersen sodium, ABT-263); proteasome inhibitor (*e.g.*, bortezomib (Velcade), NPI-0052), paclitaxel or a paclitaxel agent; docetaxel; IGF-1 receptor inhibitor (*e.g.*, AMG 479); HGF/SF inhibitor (*e.g.*, AMG 102, MK-0646); chloroquine; Aurora kinase inhibitor (*e.g.*, MLN8237); radio immunotherapy (*e.g.*, TF2); HSP90 inhibitor (*e.g.*, tanespimycin, STA-9090); mTOR inhibitor (*e.g.*, everolimus); Ep-CAM-/CD3-bispecific antibody (*e.g.*, MT110); CK-2 inhibitor (*e.g.*, CX-4945); HDAC inhibitor (*e.g.*, belinostat); SMO antagonist (*e.g.*, BMS 833923); peptide cancer vaccine, and radiation therapy (*e.g.*, intensity-modulated radiation therapy (IMRT), hypofractionated radiotherapy, hypoxia-guided radiotherapy), surgery, and combinations thereof.

An example of suitable therapeutics for use in combination for treatment of non-small cell lung cancer includes, but is not limited to, a chemotherapeutic agent, *e.g.*, vinorelbine, cisplatin, docetaxel, pemetrexed disodium, etoposide, gemcitabine, carboplatin, liposomal SN-38, TLK286, temozolomide, topotecan, pemetrexed disodium, azacitidine, irinotecan, tegafur-gimeracil-oteracil potassium, sapacitabine); tyrosine kinase inhibitor (*e.g.*, EGFR inhibitor (*e.g.*, erlotinib, gefitinib, cetuximab, panitumumab, necitumumab, PF-00299804, nimotuzumab, RO5083945), MET inhibitor (*e.g.*, PF-02341066, ARQ 197), PI3K kinase inhibitor (*e.g.*, XL147, GDC-0941), Raf/MEK dual kinase inhibitor (*e.g.*, RO5126766), PI3K/mTOR dual kinase inhibitor (*e.g.*, XL765), SRC inhibitor (*e.g.*, dasatinib), dual inhibitor (*e.g.*, BIBW 2992, GSK1363089, ZD6474, AZD0530, AG-013736, lapatinib, MEHD7945A, linifanib), multikinase inhibitor (*e.g.*, sorafenib, sunitinib, pazopanib, AMG 706, XL184, MGCD265, BMS-690514, R935788), VEGF inhibitor (*e.g.*, endostar, endostatin, bevacizumab, cediranib, BIBF 1120, axitinib, tivozanib, AZD2171), cancer vaccine (*e.g.*, BLP25 liposome vaccine , GVAX, recombinant DNA and adenovirus expressing L523S protein), Bcl-2 inhibitor (*e.g.*, oblimersen sodium), proteasome inhibitor (*e.g.*, bortezomib, carfilzomib, NPI-0052, MLN9708), paclitaxel or a paclitaxel agent, docetaxel, IGF-1 receptor inhibitor (*e.g.*, cixutumumab, MK-0646, OSI 906, CP-751,871, BIIB022), hydroxychloroquine, HSP90 inhibitor (*e.g.*, tanespimycin, STA-9090, AUY922, XL888), mTOR inhibitor (*e.g.*, everolimus, temsirolimus, ridaforolimus), Ep-CAM-/CD3-bispecific antibody (*e.g.*, MT110), CK-2 inhibitor (*e.g.*, CX-4945), HDAC inhibitor (*e.g.*, MS 275, LBH589, vorinostat, valproic acid, FR901228), DHFR inhibitor (*e.g.*, pralatrexate), retinoid (*e.g.*, bexarotene, tretinoin), antibody-drug conjugate (*e.g.*, SGN-15), bisphosphonate (*e.g.*, zoledronic acid), cancer vaccine (*e.g.*, belagenpumatucel-L), low molecular weight heparin (LMWH) (*e.g.*, tinzaparin, enoxaparin), GSK1572932A, melatonin, talactoferrin, dimesna, topoisomerase inhibitor (*e.g.*, amrubicin, etoposide, karenitecin), nelfinavir, cilengitide, ErbB3 inhibitor (*e.g.*, MM-121, U3-1287), survivin inhibitor (*e.g.*, YM155, LY2181308), eribulin mesylate, COX-2 inhibitor (*e.g.*, celecoxib), pegfilgrastim, Polo-like kinase 1 inhibitor (*e.g.*, BI 6727), TRAIL receptor 2 (TR-2) agonist (*e.g.*, CS-1008), CNGRC peptide-TNF alpha conjugate, dichloroacetate (DCA), HGF inhibitor (*e.g.*, SCH 900105), SAR240550, PPAR-gamma agonist (*e.g.*, CS-7017), gamma-secretase inhibitor (*e.g.*, RO4929097), epigenetic therapy (*e.g.*, 5-azacitidine), nitroglycerin, MEK inhibitor (*e.g.*, AZD6244), cyclin-dependent kinase inhibitor (*e.g.*, UCN-01), cholesterol-Fusl, antitubulin agent (*e.g.*, E7389), farnesyl-OH-transferase inhibitor (*e.g.*, lonafarnib), immunotoxin (*e.g.*, BB-10901, SS1 (dsFv) PE38), fondaparinux, vascular-disrupting agent (*e.g.*, AVE8062), PD-L1 inhibitor (*e.g.*, MDX-1105, MDX-1106), beta-glucan, NGR-hTNF, EMD 521873, MEK inhibitor (*e.g.*, GSK1120212), epothilone analog (*e.g.*, ixabepilone), kinesin-spindle inhibitor (*e.g.*, 4SC-205), telomere targeting agent (*e.g.*, KML-001), P70 pathway inhibitor (*e.g.*, LY2584702), AKT inhibitor (*e.g.*, MK-2206), angiogenesis inhibitor (*e.g.*, lenalidomide), Notch signaling inhibitor (*e.g.*, OMP-21M18), radiation therapy, surgery, and combinations thereof.

An example of suitable therapeutics for use in combination for treatment of ovarian cancer includes, but is not limited to, a chemotherapeutic agent (*e.g.*, paclitaxel or a paclitaxel agent; docetaxel; carboplatin; gemcitabine; doxorubicin; topotecan; cisplatin; irinotecan, TLK286, ifosfamide, olaparib, oxaliplatin, melphalan, pemetrexed disodium, SJG-136, cyclophosphamide, etoposide, decitabine); ghrelin antagonist (*e.g.*, AEZS-130), immunotherapy (*e.g.*, APC8024, oregovomab, OPT-821), tyrosine kinase inhibitor (*e.g.*, EGFR inhibitor (*e.g.*, erlotinib), dual inhibitor (*e.g.*, E7080), multikinase inhibitor (*e.g.*, AZD0530, JI-101, sorafenib, sunitinib, pazopanib), ON 01910.Na), VEGF inhibitor (*e.g.*, bevacizumab, BIBF 1120, cediranib, AZD2171), PDGFR inhibitor (*e.g.*, IMC-3G3), paclitaxel, topoisomerase inhibitor (*e.g.*, karenitecin, Irinotecan), HDAC inhibitor (*e.g.*, valproate, vorinostat), folate receptor inhibitor (*e.g.*, farletuzumab), angiopoietin inhibitor (*e.g.*, AMG 386), epothilone analog (*e.g.*, ixabepilone), proteasome inhibitor (*e.g.*, carfilzomib), IGF-1 receptor inhibitor (*e.g.*, OSI 906, AMG 479), PARP inhibitor (*e.g.*, veliparib, AG014699, iniparib, MK-4827), Aurora kinase inhibitor (*e.g.*, MLN8237, ENMD-2076), angiogenesis inhibitor (*e.g.*, lenalidomide), DHFR inhibitor (*e.g.*, pralatrexate), radioimmunotherapeutic agnet (*e.g.*, Hu3S193), statin (*e.g.*, lovastatin), topoisomerase 1 inhibitor (*e.g.*, NKTR-102), cancer vaccine (*e.g.*, p53 synthetic long peptides vaccine, autologous OC-DC vaccine), mTOR inhibitor (*e.g.*, temsirolimus, everolimus), BCR/ABL inhibitor (*e.g.*, imatinib), ET-A receptor antagonist (*e.g.*, ZD4054), TRAIL receptor 2 (TR-2) agonist (*e.g.*, CS-1008), HGF/SF inhibitor (*e.g.*, AMG 102), EGEN-001, Polo-like kinase 1 inhibitor (*e.g.*, BI 6727), gamma-secretase inhibitor (*e.g.*, RO4929097), Wee-1 inhibitor (*e.g.*, MK-1775), antitubulin agent (*e.g.*, vinorelbine, E7389), immunotoxin (*e.g.*, denileukin diftitox), SB-485232, vascular-disrupting agent (*e.g.*, AVE8062), integrin inhibitor (*e.g.*, EMD 525797), kinesin-spindle inhibitor (*e.g.*, 4SC-205), revlimid, HER2 inhibitor (*e.g.*, MGAH22), ErrB3 inhibitor (*e.g.*, MM-121), radiation therapy; and combinations thereof.

An example of suitable therapeutics for use in combination to treat a myeloma, alone or in combination with one or more of: chemotherapy or other anti-cancer agents (*e.g.*, thalidomide analogs, *e.g.*, lenalidomide), HSCT (Cook, R. (2008) J Manag Care Pharm. 14(7 Suppl):19-25), an anti-TIM-3 antibody (Hallett, WHD et al. (2011) J of American Society for Blood and Marrow Transplantation 17(8):1133-145), tumor antigen-pulsed dendritic cells, fusions (*e.g.*, electrofusions) of tumor cells and dendritic cells, or vaccination with immunoglobulin idiotype produced by malignant plasma cells (reviewed in Yi, Q. (2009) Cancer J. 15(6):502-10).

An example of suitable therapeutics for use in combination to treat a renal cancer, *e.g.*, renal cell carcinoma (RCC) or metastatic RCC. The anti-PD-1 antibody molecule can be administered in combination with one or more of: an immune-based strategy (*e.g.*, interleukin-2 or interferon-a), a targeted agent (*e.g.*, a VEGF inhibitor such as a monoclonal antibody to VEGF, *e.g.*, bevacizumab (Rini, B.I. et al. (2010) J. Clin. Oncol. 28(13):2137-2143)); a VEGF tyrosine kinase inhibitor such as sunitinib, sorafenib, axitinib and pazopanib (reviewed in Pal. S.K. et al. (2014) Clin. Advances in Hematology & Oncology 12(2):90-99)); an RNAi inhibitor), or an inhibitor of a downstream mediator of VEGF signaling, e.g., an inhibitor of the mammalian target of rapamycin (mTOR), *e.g.*, everolimus and temsirolimus (Hudes, G. et al. (2007) N. Engl. J. Med. 356(22):2271-2281, Motzer, R.J. et al. (2008) Lancet 372: 449-456).

An example of suitable therapeutics for use in combination for treatment of chronic myelogenous leukemia (AML) according to the invention includes, but is not limited to, a chemotherapeutic (*e.g.*, cytarabine, hydroxyurea, clofarabine, melphalan, thiotepa, fludarabine, busulfan, etoposide, cordycepin, pentostatin, capecitabine, azacitidine, cyclophosphamide, cladribine, topotecan), tyrosine kinase inhibitor (*e.g.*, BCR/ABL inhibitor (*e.g.*, imatinib, nilotinib), ON 01910.Na, dual inhibitor (*e.g.*, dasatinib, bosutinib), multikinase inhibitor (*e.g.*, DCC-2036, ponatinib, sorafenib, sunitinib, RGB-286638)), interferon alfa, steroids, apoptotic agent (*e.g.*, omacetaxine mepesuccinat), immunotherapy (*e.g.*, allogeneic CD4+ memory Th1-like T cells/microparticle-bound anti-CD3/anti-CD28, autologous cytokine induced killer cells (CIK), AHN-12), CD52 targeting agent (*e.g.*, alemtuzumab), HSP90 inhibitor (*e.g.*, tanespimycin, STA-9090, AUY922, XL888), mTOR inhibitor (*e.g.*, everolimus), SMO antagonist (*e.g.*, BMS 833923), ribonucleotide reductase inhibitor (*e.g.*, 3-AP), JAK-2 inhibitor (*e.g.*, INCB018424), Hydroxychloroquine, retinoid (*e.g.*, fenretinide), cyclin-dependent kinase inhibitor (*e.g.*, UCN-01), HDAC inhibitor (*e.g.*, belinostat, vorinostat, JNJ-26481585), PARP inhibitor (*e.g.*, veliparib), MDM2 antagonist (*e.g.*, RO5045337), Aurora B kinase inhibitor (*e.g.*, TAK-901), radioimmunotherapy (*e.g.*, actinium-225-labeled anti-CD33 antibody HuM195), Hedgehog inhibitor (*e.g.*, PF-04449913), STAT3 inhibitor (*e.g.*, OPB-31121), KB004, cancer vaccine (*e.g.*, AG858), bone marrow transplantation, stem cell transplantation, radiation therapy, and combinations thereof.

An example of suitable therapeutics for use in combination for treatment of chronic lymphocytic leukemia (CLL) includes, but is not limited to, a chemotherapeutic agent (*e.g.*, fludarabine, cyclophosphamide, doxorubicin, vincristine, chlorambucil, bendamustine, chlorambucil, busulfan, gemcitabine, melphalan, pentostatin, mitoxantrone, 5-azacytidine, pemetrexed disodium), tyrosine kinase inhibitor (*e.g.*, EGFR inhibitor (*e.g.*, erlotinib), BTK inhibitor (*e.g.*, PCI-32765), multikinase inhibitor (*e.g.*, MGCD265, RGB-286638), CD-20 targeting agent (*e.g.*, rituximab, ofatumumab, RO5072759, LFB-R603), CD52 targeting agent (*e.g.*, alemtuzumab), prednisolone, darbepoetin alfa, lenalidomide, Bcl-2 inhibitor (*e.g.*, ABT-263), immunotherapy (*e.g.*, allogeneic CD4+ memory Th1-like T cells/microparticle-bound anti-CD3/anti-CD28, autologous cytokine induced killer cells (CIK)), HDAC inhibitor (*e.g.*, vorinostat, valproic acid, LBH589, JNJ-26481585, AR-42), XIAP inhibitor (*e.g.*, AEG35156), CD-74 targeting agent (*e.g.*, milatuzumab), mTOR inhibitor (*e.g.*, everolimus), AT-101, immunotoxin (*e.g.*, CAT-8015, anti-Tac(Fv)-PE38 (LMB-2)), CD37 targeting agent (*e.g.*, TRU-016), radioimmunotherapy (*e.g.*, 131-tositumomab), hydroxychloroquine, perifosine, SRC inhibitor (*e.g.*, dasatinib), thalidomide, PI3K delta inhibitor (*e.g.*, CAL-101), retinoid (*e.g.*, fenretinide), MDM2 antagonist (*e.g.*, RO5045337), plerixafor, Aurora kinase inhibitor (*e.g.*, MLN8237, TAK-901), proteasome inhibitor (*e.g.*, bortezomib), CD-19 targeting agent (e*.g.*, MEDI-551, MOR208), MEK inhibitor (*e.g.*, ABT-348), JAK-2 inhibitor (*e.g.*, INCB018424), hypoxia-activated prodrug (*e.g.*, TH-302), paclitaxel or a paclitaxel agent, HSP90 inhibitor, AKT inhibitor (*e.g.*, MK2206), HMG-CoA inhibitor (*e.g.*, simvastatin), GNKG186, radiation therapy, bone marrow transplantation, stem cell transplantation, and a combination thereof.

An example of suitable therapeutics for use in combination for treatment of acute lymphocytic leukemia (ALL) includes, but is not limited to, a chemotherapeutic agent (*e.g.*, prednisolone, dexamethasone, vincristine, asparaginase, daunorubicin, cyclophosphamide, cytarabine, etoposide, thioguanine, mercaptopurine, clofarabine, liposomal annamycin, busulfan, etoposide, capecitabine, decitabine, azacitidine, topotecan, temozolomide), tyrosine kinase inhibitor (*e.g.*, BCR/ABL inhibitor (*e.g.,* imatinib, nilotinib), ON 01910.Na, multikinase inhibitor (*e.g.*, sorafenib)), CD-20 targeting agent (*e.g.*, rituximab), CD52 targeting agent (*e.g.*, alemtuzumab), HSP90 inhibitor (*e.g.*, STA-9090), mTOR inhibitor (*e.g.*, everolimus, rapamycin), JAK-2 inhibitor (*e.g.*, INCB018424), HER2/neu receptor inhibitor (*e.g.*, trastuzumab), proteasome inhibitor (*e.g.*, bortezomib), methotrexate, asparaginase, CD-22 targeting agent (*e.g.*, epratuzumab, inotuzumab), immunotherapy (*e.g.*, autologous cytokine induced killer cells (CIK), AHN-12), blinatumomab, cyclin-dependent kinase inhibitor (*e.g.*, UCN-01), CD45 targeting agent (*e.g.*, BC8), MDM2 antagonist (*e.g.*, RO5045337), immunotoxin (*e.g.*, CAT-8015, DT2219ARL), HDAC inhibitor (*e.g.*, JNJ-26481585), JVRS-100, paclitaxel or a paclitaxel agent, STAT3 inhibitor (*e.g.*, OPB-31121), PARP inhibitor (*e.g.*, veliparib), EZN-2285, radiation therapy, steroid, bone marrow transplantation, stem cell transplantation, or a combination thereof.

An example of suitable therapeutics for use in combination for treatment of acute myeloid leukemia (AML) includes, but is not limited to, a chemotherapeutic agent (*e.g.*, cytarabine, daunorubicin, idarubicin, clofarabine, decitabine, vosaroxin, azacitidine, clofarabine, ribavirin, CPX-351, treosulfan, elacytarabine, azacitidine), tyrosine kinase inhibitor (*e.g.*, BCR/ABL inhibitor (*e.g.*, imatinib, nilotinib), ON 01910.Na, multikinase inhibitor (*e.g.*, midostaurin, SU 11248, quizartinib, sorafinib)), immunotoxin (*e.g.*, gemtuzumab ozogamicin), DT388IL3 fusion protein, HDAC inhibitor (*e.g.*, vorinostat, LBH589), plerixafor, mTOR inhibitor (*e.g.*, everolimus), SRC inhibitor (*e.g.*, dasatinib), HSP90 inhbitor (*e.g.*, STA-9090), retinoid (*e.g.*, bexarotene, Aurora kinase inhibitor (*e.g.*, BI 811283), JAK-2 inhibitor (*e.g.*, INCB018424), Polo-like kinase inhibitor (*e.g.*, BI 6727), cenersen, CD45 targeting agent (*e.g.*, BC8), cyclin-dependent kinase inhibitor (*e.g.*, UCN-01), MDM2 antagonist (*e.g.*, RO5045337), mTOR inhibitor (*e.g.*, everolimus), LY573636-sodium, ZRx-101, MLN4924, lenalidomide, immunotherapy (*e.g.*, AHN-12), histamine dihydrochloride, radiation therapy, bone marrow transplantation, stem cell transplantation, and a combination thereof.

An example of suitable therapeutics for use in combination for treatment of multiple myeloma (MM) includes, but is not limited to, a chemotherapeutic agent (*e.g.*, melphalan, amifostine, cyclophosphamide, doxorubicin, clofarabine, bendamustine, fludarabine, adriamycin, SyB L-0501), thalidomide, lenalidomide, dexamethasone, prednisone, pomalidomide, proteasome inhibitor *(e.g.,* bortezomib, carfilzomib, MLN9708), cancer vaccine *(e.g.,* GVAX), CD-40 targeting agent (*e.g.*, SGN-40, CHIR-12.12), perifosine, zoledronic acid, Immunotherapy (*e.g.*, MAGE-A3, NY-ESO-1 , HuMax-CD38), HDAC inhibitor (*e.g.*, vorinostat, LBH589, AR-42), aplidin, cycline-dependent kinase inhibitor (*e.g.*, PD-0332991, dinaciclib), arsenic trioxide, CB3304, HSP90 inhibitor (*e.g.*, KW-2478), tyrosine kinase inhibitor (*e.g.*, EGFR inhibitor (*e.g.*, cetuximab), multikinase inhibitor (*e.g.*, AT9283)), VEGF inhibitor (*e.g.*, bevacizumab), plerixafor, MEK inhibitor (*e.g.*, AZD6244), IPH2101, atorvastatin, immunotoxin (*e.g.*, BB-10901), NPI-0052, radioimmunotherapeutic (*e.g.*, yttrium Y 90 ibritumomab tiuxetan), STAT3 inhibitor (*e.g.*, OPB-31121), MLN4924, Aurora kinase inhibitor (*e.g.*, ENMD-2076), IMGN901, ACE-041, CK-2 inhibitor (*e.g.*, CX-4945), radiation therapy, bone marrow transplantation, stem cell transplantation, and a combination thereof.

An example of suitable therapeutics for use in combination for treatment of prostate cancer includes, but is not limited to, a chemotherapeutic agent (*e.g.*, docetaxel, carboplatin, fludarabine), abiraterone, hormonal therapy (*e.g.*, flutamide, bicalutamide, nilutamide, cyproterone acetate, ketoconazole, aminoglutethimide, abarelix, degarelix, leuprolide, goserelin, triptorelin, buserelin), tyrosine kinase inhibitor (*e.g.*, dual kinase inhibitor (*e.g.*, lapatanib), multikinase inhibitor (*e.g.*, sorafenib, sunitinib)), VEGF inhibitor (*e.g.*, bevacizumab), TAK-700, cancer vaccine (*e.g.*, BPX-101, PEP223), lenalidomide, TOK-001, IGF-1 receptor inhibitor (*e.g.*, cixutumumab), TRC105, Aurora A kinase inhibitor (*e.g.*, MLN8237), proteasome inhibitor (*e.g.*, bortezomib), OGX-011, radio immunotherapy (*e.g.*, HuJ591-GS), HDAC inhibitor (*e.g.*, valproic acid, SB939, LBH589), hydroxychloroquine, mTOR inhibitor (*e.g.*, everolimus), dovitinib lactate, diindolylmethane, efavirenz, OGX-427, genistein, IMC-3G3, bafetinib, CP-675,206, radiation therapy, surgery, or a combination thereof.

The combination therapies can be administered in combination with one or more of the existing modalities for treating cancers, including, but not limited to: surgery; radiation therapy (*e.g.*, external-beam therapy which involves three dimensional, conformal radiation therapy where the field of radiation is designed, local radiation (*e.g.*, radition directed to a preselected target or organ), or focused radiation). Focused radiation can be selected from the group consisting of stereotactic radiosurgery, fractionated stereotactic radiosurgery, and intensity-modulated radiation therapy. The focused radiation can have a radiation source selected from the group consisting of a particle beam (proton), cobalt-60 (photon), and a linear accelerator (x-ray), *e.g.,* as decribed in WO 2012/177624.

Radiation therapy can be administered through one of several methods, or a combination of methods, including without limitation external-beam therapy, internal radiation therapy, implant radiation, stereotactic radiosurgery, systemic radiation therapy, radiotherapy and permanent or temporary interstitial brachytherapy. The term "brachytherapy," refers to radiation therapy delivered by a spatially confined radioactive material inserted into the body at or near a tumor or other proliferative tissue disease site. The term is intended without limitation to include exposure to radioactive isotopes (*e.g.* At-211, I-131, I-125, Y-90, Re-186, Re-188, Sm-153, Bi-212, P-32, and radioactive isotopes of Lu). Suitable radiation sources for use as a cell conditioner of the present invention include both solids and liquids. By way of non-limiting example, the radiation source can be a radionuclide, such as I-125, I-131, Yb-169, Ir-192 as a solid source, I-125 as a solid source, or other radionuclides that emit photons, beta particles, gamma radiation, or other therapeutic rays. The radioactive material can also be a fluid made from any solution of radionuclide(s), *e.g.*, a solution of I-125 or I-131, or a radioactive fluid can be produced using a slurry of a suitable fluid containing small particles of solid radionuclides, such as Au-198, Y-90. Moreover, the radionuclide(s) can be embodied in a gel or radioactive micro spheres.

### Nucleic Acids

The disclosure also features nucleic acids comprising nucleotide sequences that encode heavy and light chain variable regions and CDRs or hypervariable loops of the antibody molecules, as described herein. The nucleic acid can comprise a nucleotide sequence as set forth herein, or a sequence substantially identical thereto (*e.g.*, a sequence at least about 85%, 90%, 95%, 99% or more identical thereto, or which differs by no more than 3, 6, 15, 30, or 45 nucleotides from the sequences shown in the tables herein.

### Vectors

Further provided herein are vectors comprising nucleotide sequences encoding an antibody molecule described herein. In one aspect, the vectors comprise nucleotides encoding an antibody molecule described herein. In one aspect, the vectors comprise the nucleotide sequences described herein. The vectors include, but are not limited to, a virus, plasmid, cosmid, lambda phage or a yeast artificial chromosome (YAC).

Numerous vector systems can be employed. For example, one class of vectors utilizes DNA elements which are derived from animal viruses such as, for example, bovine papilloma virus, polyoma virus, adenovirus, vaccinia virus, baculovirus, retroviruses (Rous Sarcoma Virus, MMTV or MOMLV) or SV40 virus. Another class of vectors utilizes RNA elements derived from RNA viruses such as Semliki Forest virus, Eastern Equine Encephalitis virus and Flaviviruses.

Additionally, cells which have stably integrated the DNA into their chromosomes may be selected by introducing one or more markers which allow for the selection of transfected host cells. The marker may provide, for example, prototropy to an auxotrophic host, biocide resistance (*e.g.*, antibiotics), or resistance to heavy metals such as copper, or the like. The selectable marker gene can be either directly linked to the DNA sequences to be expressed, or introduced into the same cell by cotransformation. Additional elements may also be needed for optimal synthesis of mRNA. These elements may include splice signals, as well as transcriptional promoters, enhancers, and termination signals.

Once the expression vector or DNA sequence containing the constructs has been prepared for expression, the expression vectors may be transfected or introduced into an appropriate host cell. Various techniques may be employed to achieve this, such as, for example, protoplast fusion, calcium phosphate precipitation, electroporation, retroviral transduction, viral transfection, gene gun, lipid based transfection or other conventional techniques. In the case of protoplast fusion, the cells are grown in media and screened for the appropriate activity.

Methods and conditions for culturing the resulting transfected cells and for recovering the antibody molecule produced are known to those skilled in the art, and may be varied or optimized depending upon the specific expression vector and mammalian host cell employed, based upon the present description.

### Cells

The disclosure also provides host cells comprising a nucleic acid encoding an antibody molecule as described herein.

In one aspect, the host cells are genetically engineered to comprise nucleic acids encoding the antibody molecule.

In one aspect, the host cells are genetically engineered by using an expression cassette. The phrase "expression cassette," refers to nucleotide sequences, which are capable of affecting expression of a gene in hosts compatible with such sequences. Such cassettes may include a promoter, an open reading frame with or without introns, and a termination signal. Additional factors necessary or helpful in effecting expression may also be used, such as, for example, an inducible promoter.

The disclosure also provides host cells comprising the vectors described herein.

The cell can be, but is not limited to, a eukaryotic cell, a bacterial cell, an insect cell, or a human cell. Suitable eukaryotic cells include, but are not limited to, Vero cells, HeLa cells, COS cells, CHO cells, HEK293 cells, BHK cells and MDCKII cells. Suitable insect cells include, but are not limited to, Sf9 cells.

The following Examples illustrate the disclosure and provide specific embodiments, however without limiting the scope of the disclosure.

### EXAMPLES

### Example 1: Effects of Targeted Agents on PD-L1 Modulation

This example evaluates the effects of selected therapeutic agents (*e.g.*, INC280, MEK162, LGX818 and LDK378) on PD-L1 (CD274) modulation. Selected therapeutic agents were examined by real time PCR and flow cytometry on PD-L1 levels. Significant inhibition of PD-L1 by INC280, INC424, MEK162, LGX818 and LDK378 on tumor cells was observed.

### INC280 downregulation of PD-L1 protein

PD-L1 (CD274) expression was analyzed in cancer cell lines treated with INC280. Cells were obtained from ATCC and cultured *in vitro* following ATCC directions. The cell lines used were previously characterized by the Cancer Cell Line Encyclopedia Project (www.broadinstitute.org/ccle/home).

Cells plated in six-well culture plates were treated with the INC280 at different concentrations (10 nM, 100 nM, and 1000 nM) for 24, 48 and 72 hours. Equal amount of vehicle (DMSO) was used as a control. Cells were washed with PBS and then harvested using a cell scraper.

For each reaction, 0.5-1 x 10⁶ cells were stained with 20µL of anti-human monoclonal PD-L1 - PE antibody, clone M1H1 (BD) for 30-60 minutes at 4°C. Cells were washed twice and data was acquired using a Canto II with FACSDiva software (BD Bioscience). Data analysis was performed using FlowJo software (Tree Star). Mean fluorescence intensity (MFI) was determined by gating on single cells. Unstained cells were used as a gating control.

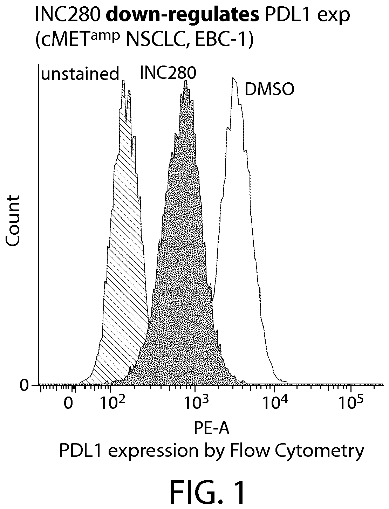

*In vitro* treatment of EBC-1 cells (Non-Small Cell Lung Cancer (NSCLC) with cMET amplification) with INC280 led to significant downregulation of surface expression of PD-L1 as observed by flow cytometry **(****Figure 1**). The results presented herein suggest that INC280 functions as a PD-L1/PD-1 inhibitor.

### INC280, MEK162, INC424, LGX818 and LDK 378 downregulate PD-L1 mRNA

TaqMan RT PCR assays were developed to detect changes of expression levels of PD-L1 (CD274) in cell lines and xenograft tumors. mRNA was isolated from frozen cell pellets or tumor fragments using the Qiagen RNeasy Mini kit. Isolated RNA was frozen at -80°C. RNA quality was checked and RNA was quantified using a 2100 Agilent Bioanalyzer following the protocol for the Agilent RNA 6000 Nano Kit. cDNA was prepared using a High Capacity RNA-to cDNA Kit (Applied Biosystems).

Real-time PCR reactions were carried out in 20µl total volume, including 10µl of Universal PCR master mix (Applied Biosystems), 1µl of human PD-L1 (CD274) probe/primer set (Applied Biosystems), and 8 µl of cDNA. Each sample was run in triplicate. The amount of cDNA produced from 25-50 ng of RNA in the reverse transcription reaction was used in each PCR reaction. Due to difference in mRNA levels between PD-L1 and GAPDH, the two real-time PCR reactions were done in separate tubes using same amount of cDNA. The real-time PCR reaction was run on the C1000 Thermal Cycle (BioRad) with the cycle program as follows: a 10 minute incubation at 95°C followed by 40 cycles of 95°C for 15 seconds and 60°C for 1 minute. After the reaction was complete, the PD-L1 average Ct was normalized relative to each Ct value from the GAPDH reference reaction. Each normalized logarithmic value was then converted into a linear value.

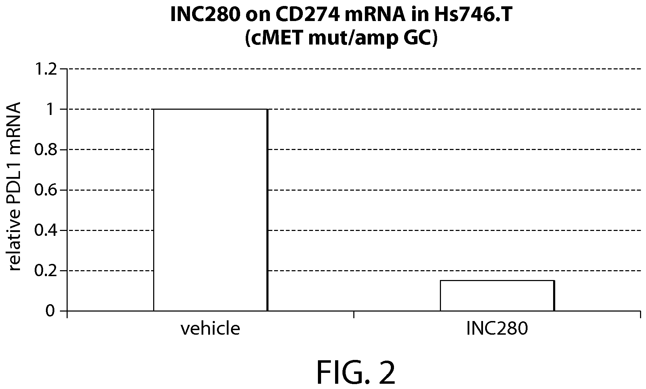

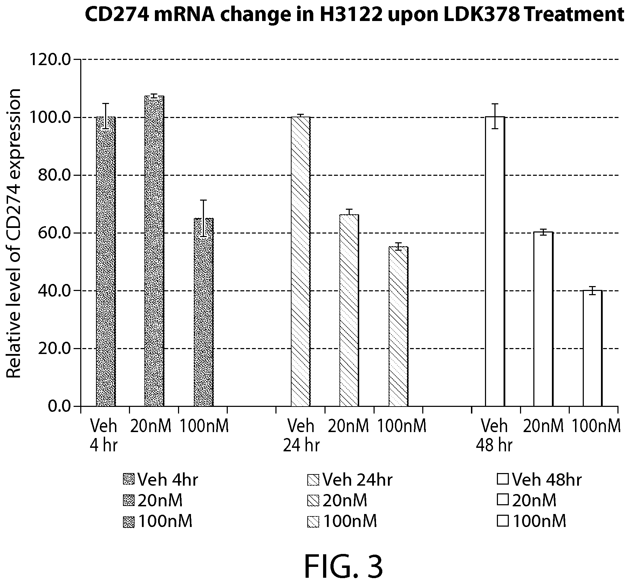

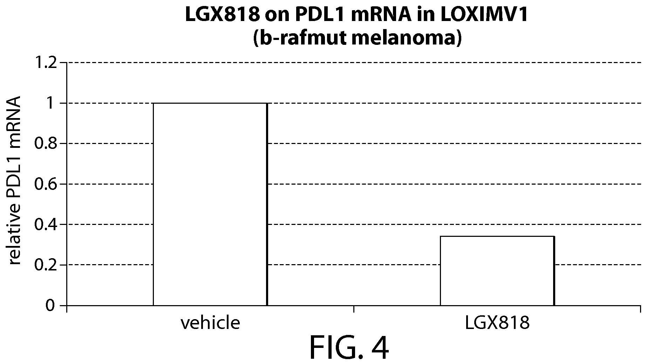

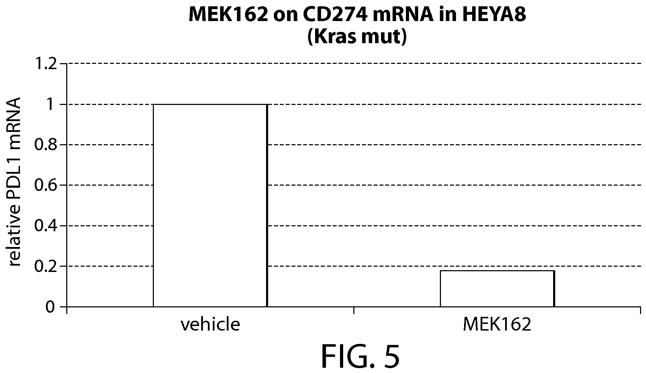

Inhibition of PD-L1 expression (mRNA) by INC280 was observed in a Hs.746.T tumor (gastric cancer cell with cMET amplification & mutation) xenograft (**Figure 2**). Inhibition of PD-L1 mRNA by LDK378 was observed in H3122 (Non-Small Cell Lung Cancer (NSCLC) with ALK translocation) *in vitro* (**Figure 3**). Downregulation of PD-L1 mRNA by LGX818, and MEK162 was observed in tumor xenograft models bearing LOXIMV1 (BRAF mutant melanoma, **Figure 4**) and HEYA8 (KRAF mutant ovarian cancer, **Figure 5**) tumors, respectively. Downregulation of PD-L1 mRNA by INC424 was observed in tumor xenograft models bearing UKE-1 (Myeloproliferative Neoplasm (MPN) line with JAK2V617F mutation,

### Figure 6).

The results presented herein demonstrate a role of INC280, MEK162, INC424, LGX818 and LDK 378 in the regulation of immunecheckpoint molecules on cancer. The observed inhibition of PD-L1 expression by these agents suggests that these targeted agents may have immune-modulatory activities, in addition to their effects on cancer signaling. Thus, the results presented herein suggest that administration of targeted agents with inhibitors of immunecheckpoint inhibitors such as PD-1, PD-L1, LAG-3 and/or TIM-3 will achieve a more potent reversal of the immunecheckpoint-mediated immune suppression.

### Example 2: Effects of LCL161 on Immune Stimulation

This example evaluates the effects of LCL161 on immune stimulation *in vitro.*

Blood was obtained from normal healthy donors. Peripheral blood mononuclear cells (PBMCs) were isolated by centrifuging blood in CPT™ tubes for 18 minutes at 1800 x g. Cells were washed twice with cold PBS, enumerated and then stimulated in 96-well round bottomed tissue culture plates (500,000 cells per well) for 5 days at 37 °C, 5% CO₂. Cells were either left untreated (DMSO control) or treated with different concentrations of LCL161 (1, 50, 100, or 1000 nM). For the cytokine analyses, cells were stimulated with a suboptimal anti-CD3 stimulus (0.005 ug/ml of soluble clone UCHT1). At day 5 post stimulation, supernatants were collected and analyzed for cytokines (Luminex).

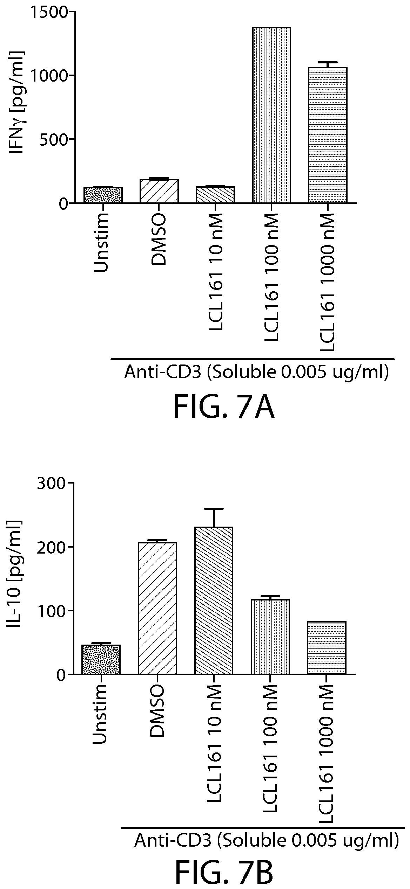

As shown in **Figures 7A-7B****,** LCL161 treatment led to increases in immune-active cytokine, IFN-gamma, *in vitro,* with a corresponding reduction in immune-suppressive cytokine IL-10.

For the proliferation analyses, cells were labeled with 5uM carboxyfluorescein diacetate succinimidyl ester (CFSE) before stimulation, and then treated with 1 ug/ml soluble anti-CD3. Covalently bound CFSE is divided equally between daughter cells, allowing discrimination of successive rounds of cell division and to track proliferating cells (Lyons et al., Curr Protoc Cytom. 2013; Chapter 9: Unit 9.11). PBMCs were stimulated in the presence of increasing concentrations of LCL161 (or DMSO control) for 5 days. At day 5 post stimulation, cells were harvested, stained with anti-CD4, -CD8, -PD-1, or -CD127, followed by FACS analysis. Results for one donor are shown. Similar results were obtained with PBMCs from 4 other donors.

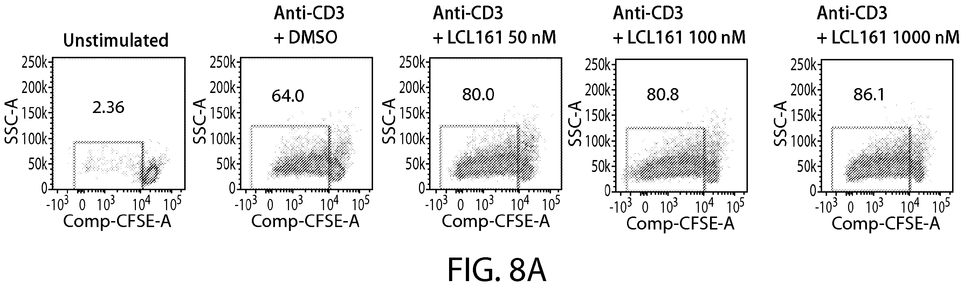

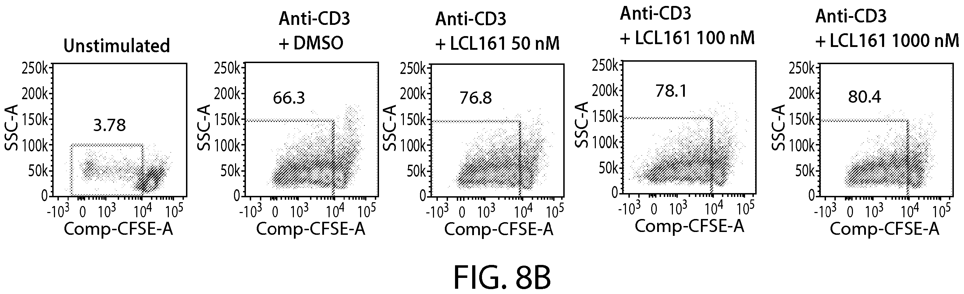

As shown in **Figures 8A-8B****,** LCL161 enhanced proliferation of human CD4+ and CD8+ T cells *in vitro.* These results indicate enhancement of CFSE dilution by LCL161, indicative of increased lymphocyte proliferation under LCL161.

### Example 3: Effects of LCL161 on Immune Checkpoint Modulation

This example evaluates the effects of LCL161 on immune checkpoint modulation *in vitro.*

PBMCs were isolated from healthy donors via BD CPT™ vacutainer tubes. 1 million cells/mL were plated in RPMI + 10% FBS +/- 100ng/mL of anti-CD3 antibody +/- DMSO or 100nM LCL161 for 4 days. Cells were harvested, washed, stained with a panel of metal-conjugated antibodies listed below in Table 2 for analysis by CyTOF mass cytometry. Data were visualized by SPADE using Cytobank webware. SPADE and its use are described, e.g., in Peng et al., Nature Biotechnology, 29, 886-891 (2011); Sean et al, Science, 332 (6030): 687-696 (2011).

**A Panel of Metal-Conjugated Antibodies for Analysis by CyTOF Mass Cytometry**

| **Metal label** | **Specificity** |
|---|---|
| 141Pr | CD235a/b |
| 142Nd | CD19 |
| 145Nd | CD4 |
| 146Nd | CD8a |
| 147Sm | CD20 |
| 148Nd | CD16 |
| 149Sm | CD66 |
| 151Eu | CD123 |
| 153Eu | TIM-3 |
| 154Sm | CD45 |
| 156Gd | PD-L1 |
| 159Tb | CD11c |
| 160Gd | CD14 |
| 165Ho | LAG-3 |
| 167Er | CD27 |
| 169Tm | CD45RA |
| 170Er | CD3 |
| 172Yb | CD38 |
| 174Yb | HLA-DR |
| 175Lu | PD-1 |
| 191Ir | DNA1 |
| 193Ir | DNA2 |
| 195Pt | Live/Dead |

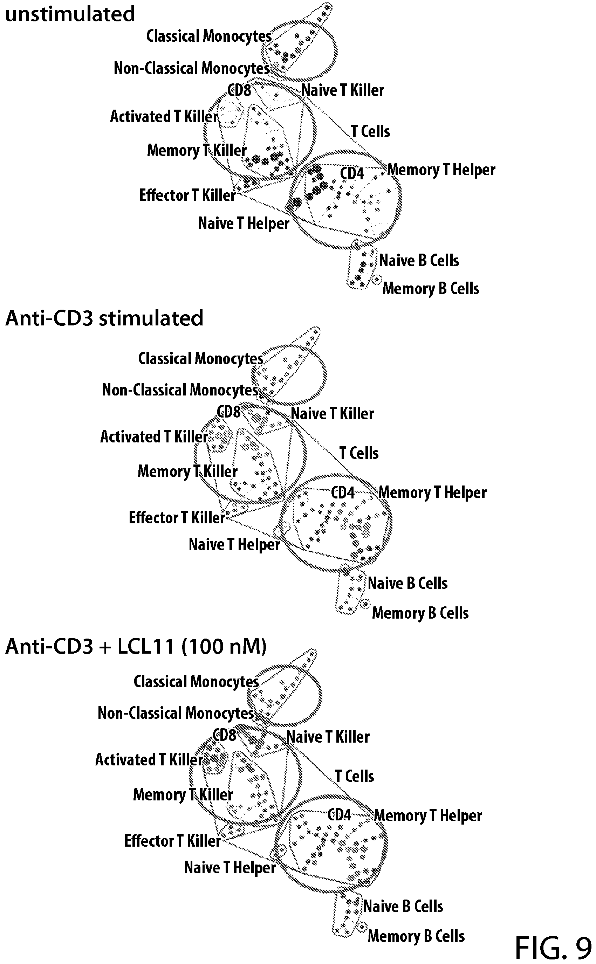

**Figure 9** shows an increase in TIM-3 expression in several nodes of several cell types including: monocytes, naive, memory, and activated T killer cells as well as memory T helper cells. This is direct evidence of LCL161-mediated immunecheckpoint TIM-3 induction. It provides, at least in part, the scientific rationale for combining LCL161 and immune checkpoint modulators, *e.g.*, anti-TIM-3 antibody, in cancer therapy.

### Example 4: Effects of LCL161/Anti-PD-1 Combination on Immune Modulation

This example evaluates the effects of LCL161/anti-PD-1 combination on immune checkpoint modulation *in vivo.*

C57B1/6 mice were implanted with 1x10⁶MC38 murine colon carcinoma cells/mouse and randomized based on tumor measurements on day 5. The mice received a dose ofLCL161 (50mg/kg, po), anti-mouse PD-1 (10mg/kg, i.v.), or both, on the day of randomization. In the control group, mice were dosed with Vehicle (p.o.) and Isotype (mIgG1, 10mg/kg, i.v.). Seven days post-treatment, the animals were euthanized, and the tumors were collected for molecular analysis.

For genomic expression analysis, total RNA was extracted from the aforementioned samples. mRNA expression of mRNA was analyzed on a customized panel of ∼1050 genes on the Nanostring platform (NanoString Technologies).

Gene signatures were derived from mRNA-sequencing data representing 27 separate indications available as part of The Cancer Genome Atlas (TCGA). For each indication, 5,000 genes were clustered into sets with very high correlation across samples. Clustering was performed using the Affinity propagation algorithm (Frey and Dueck (2007) Science 315: 972-976) on the gene-gene Pearson correlation matrix. The 5,000 genes clustered in each indication were comprised of a curated set of 1,000 cell lineage markers and genes involved in immune processes, as well as the 4,000 most variably expressed genes in that indication.

Clusters were annotated to identify co-expressed genes representing specific cell types or immune processes. Annotations included mean log expression level by immune cell type (using the Immgen consortium expression data, immgen.org), mean log expression level by normal tissue type (using GTEx data, www.gtexportal.org), and gene set enrichment using the MSigDB collection (calculated as the Fisher's exact test p-value testing the null hypothesis of random overlap between cluster genes and MSigDB gene sets). Based on these annotations, clusters that were not enriched for genes involved in immune processes were removed.

Gene signatures were generated by pooling clusters from all indications and identifying those with consistent annotations (*e.g.*, enriched expression in common cell types or common MSigDB pathway enrichment). Genes from these pooled clusters were then assessed for correlation on an indication-by-indication basis. Only genes whose high level of correlation was preserved across 80% (22/27) of indications or more were included in the final signature.

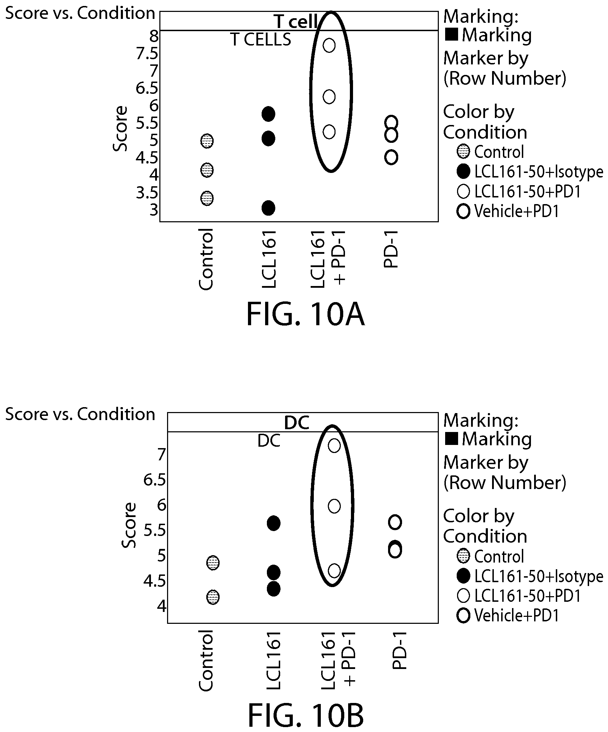

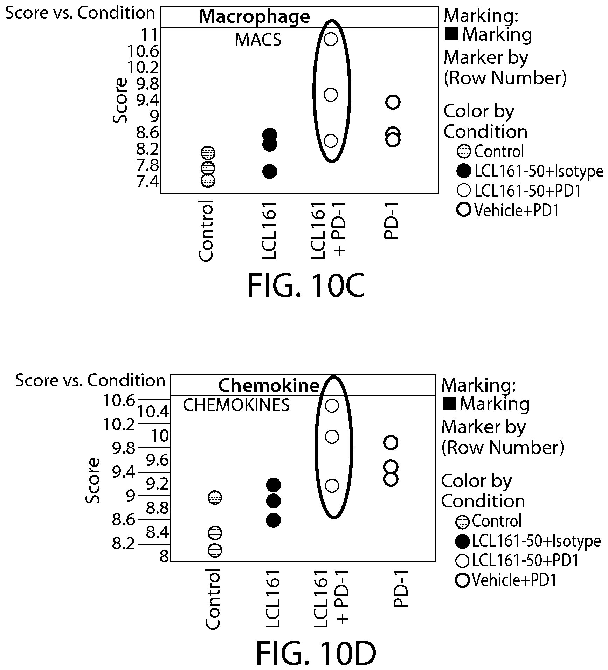

The Nanostring gene signature analysis shows that combination treatment using LCL161 and anti-PD-1 elevated expression signatures related to T cells, dendritic cells, macrophages and chemokine expression (**Figures 10A-10D**). The signature scores with the combination is higher than that of each of the monotherapy. These data indicate that the LCL161/anti-PD-1 combination was immune-stimulatory and the combination of LCL161 with immunecheckpoint therapies would further enhance anti-tumor immunity.

### Example 5: Efficacy of LCL161/Anti-PD-1 Combination

This example evaluates the efficacy of the LCL161/anti-PD-1 combination *in vivo.*

C57B1/6 mice were implanted with 1x10⁶ MC38 cells/mouse and randomized based on tumor measurements on day 4. Vehicle and LCL161 were given twice a day, every week, by p.o. administration. Isotype and anti-mouse PD-1 were given once per week, by i.v. administration. Two treatment schedules were tested: 1) anti-mouse PD-1 was administred three days after administration of LCL161; or 2) LCL161 and anti-mouse PD-1 were administered concurrently. The design for the studies is summarized below.

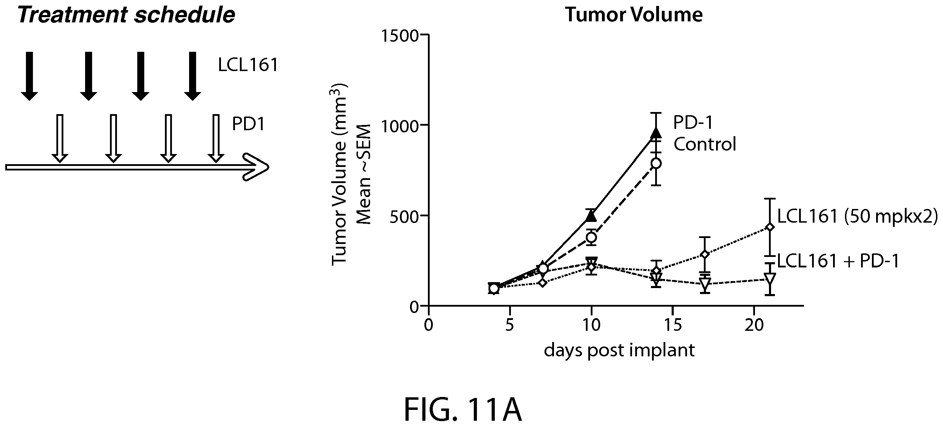

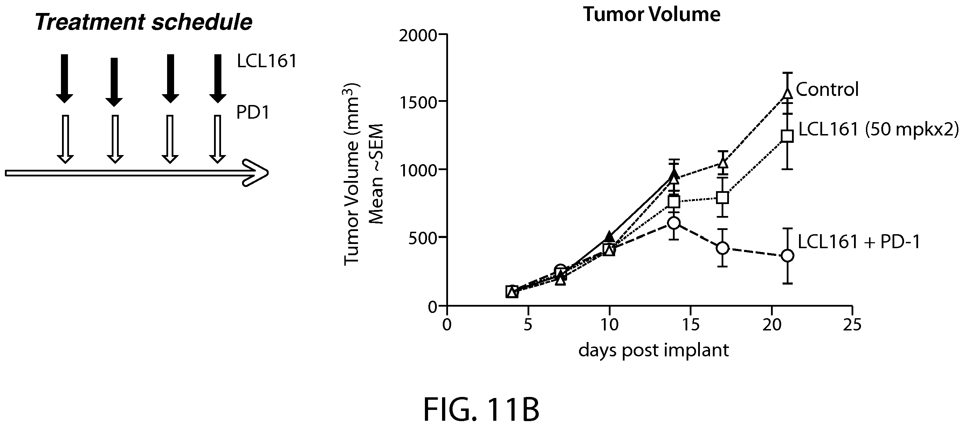

Tumor dimensions and body weights were collected and recorded twice a week. As shown in **Figures 11A-11B****,** the LCL161/anti-PD-1 combination demonstrated anti-tumor efficacy.

| **Group** | **Treatment (Schedule 1)** | **mice/group** |
|---|---|---|
| 1 | Vehicle, bid (1x/week) -starting on day 4 -, po + Isotype mIgG1 (MOPC-21) - 10mpk, 1x/week-starting on day 7-, iv | 9 |
| 2 | LCL161, 50mg/kg, bid (1x/week) -starting on day 4-, po + Isotype mIgG1 (MOPC-21) - 10mpk, 1x/week-starting on day 7-, iv | 9 |
| 3 | Anti-PD-1 (1D2) - 10mpk, 1x/week-starting on day7, iv | 10 |
| 4 | LCL161, 50mg/kg, bid (1x/week) -starting on day 4-, po + Anti-PD-1 (1D2) - 10mpk, 1x/week starting on dav 7-, iv | 9 |

| **Group** | **Treatment (Schedule 2)** | **mice/group** |
|---|---|---|
| 1 | Vehicle, bid (1x/week) -starting on day 7-, po + Isotype mIgG1 (MOPC-21) - 10mpk, 1x/week-starting on day 7-, iv | 9 |
| 2 | LCL161, 50mg/kg, bid (1x/week) -starting on day 7-, po + Isotype mIgG1 (MOPC-21) - 10mpk, 1x/week-starting on day 7-, iv | 9 |
| 3 | Anti-PD-1 (1D2) - 10mpk, 1x/week-starting on day 7, iv | 10 |
| 4 | LCL161, 50mg/kg, bid (1x/week) -starting on day 7 -, po + Anti-PD-1 (1D2) - 10mpk, 1x/week-starting on dav 7-, iv | 9 |

### Summary:

Thedata on *in vitro* mechanism-of-action supports the immune-stimulatory roles of LCL161. The comprehensive immune profiling by CyTOF indicates the connection between immunecheckpoint (TIM-3) and LCL161. Furthermore, *in vivo* experiments demonstrate synergistic effects with LCL161 and PD-1 in broader immune stimulation and anti-tumor efficacy. Taken together, the data presented in Examples 2-5 demonstrate the combination benefit of LCL161 with immunecheckpoint therapies in cancer.

### Example 6: Dose Escalation and Expansion Study of the LDK (Certinib) and Nivolumab Combination

Efficacy and safety of the ceritinib (LDK378) and nivolumab combination can be assessed in an open-label, multi-center dose escalation and expansion study. In addition to the safety and efficacy, the tolerability and PK/PD of combination of ceritinib and nivolumab for the treatment of patients with metastatic, ALK-positive non-small cell luncer cancer (NSCLC) can also be evaluated. The study can begin with a screening period of up to and including 28 days prior to the first dose of the study drugs to assess eligibility. The treatment period can begin on the first day of the first cycle. The cycles are 28 days long.

Treatment with ceritinib and nivolumab may for example continue until the patient experiences unacceptable toxicity that precludes further treatment and/or disease progression.

In cases of isolated brain progression or other local progression, patients may in addition receive palliative radiotherapy.

The study can include a dose-escalation phase and a dose-expansion phase.

### 1. Dose-escalation phase

The dose-escalation phase of the study can evaluate the maximum tolerated dose (MTD)/ recommended dose for expansion (RDE) of the combination of oral daily ceritinib with a low-fat meal and intravenous nivolumab every 2 weeks (Q2W) based on dose limiting toxicities (DLTs) using a Bayesian Logistic Regression Model (BLRM). For example, 12 patients are enrolled in this phase of the study. The initial dose level of ceritinib can be 450 mg daily and nivolumab is administered at the dose of 3 mg/kg Q2W. The provisional dose levels are as follows:
- [-1 dose cohort] ceritinib 300 mg + nivolumab (3 mg/kg)
- [1st dose cohort] ceritinib 450 mg + nivolumab (3 mg/kg)
- [2nd dose cohort] ceritinib 600 mg + nivolumab (3 mg/kg)

The MTD is the highest drug dosage of both agents not expected to cause DLT in more than 35% of the treated patients in the first 6 weeks of treatment. The final recommended MTD/RDE for combination ceritinib and nivolumab is based on the recommendation from the BLRM, and on an overall assessment of safety taking into consideration tolerability and pharmacokinetic data from subsequent cycles at the tested doses. If the MTD for combination ceritinib and nivolumab is not established after the evaluation of all planned dose levels including the target doses of ceritinib (600 mg with low-fat meal) and nivolumab (3 mg/kg), the RDE is determined after the evaluation of all available safety, PK, and efficacy data.

### 2. Dose-expansion phase

Once the MTD of the combination has been declared and/or the RDE is determined, additional patients are evaluated in the expansion phase of the study at the RDE combination dose. For example, 60 patients are enrolled into the expansion phase of the study. The expansion phase evaluates the safety and preliminary efficacy of the ceritinib and nivolumab combination at the RDE and consists of 2 arms (approximately 30 patients in each arm):
- Arm 1: ALK inhibitor-treated (Prior treatment with any ALK inhibitor except ceritinib is allowed.)
- Arm 2: ALK inhibitor-naive

The data cut-off for the primary clinical study report can occur once all patients in the expansion phase have completed at least 6 cycles (24 weeks) of treatment or have discontinued earlier.

### Example 7: Effects of the LDK378 and Nivolumab Combination in Humans

Eight patients were enrolled to the first dose cohort in the study just as outlined in the Example 6 and below is the data of the only patient with a valid tumor assessment. A partial response was observed with this patient. A second assessment is required to fully confirm the response.

Patient assessed was a 64 year old Caucasian male with diagnosed stage IV NSCLC. Sites of disease included lung, adrenal and abdominal lymph nodes. The patient received one prior chemotherapy regimen of cisplatin and pemetrexed and achieved a partial response. Additional medical conditions include adrenal insufficiency, mitral valve prolapse, hypercholesterolemia, and urolithiasis.

The patient started study treatment with LDK378 450mg QD (oral), administered with a low fat meal, in combination with Nivolumab 3mg/kg every 2 weeks (intravenous). 29 days after the first dose of the study medications (combination of LDK378 + Nivolumab) the patient presented with fever, abdominal pain, nausea, and vomiting. Abdominal ultrasound was negative but computerized tomogram (CT) of the abdomen demonstrated acute pancreatitis. In addition, there were elevations in lipase, amylase, ALT, AST, bilirubin, ALP, and GGT. The patient was hospitalized and treatment with study medication LDK378 was temporarily interrupted. Treatment with intravenous fluids, paracetamol, Contramal (tramadol hydrochloride) and Litican (alizapride) was administered. In the following days the patient's laboratory results improved and the patient's condition improved; there were no more complaints of pain, fever, nausea and vomiting. All supporting medications were stopped and the patient was discharged from the hospital.

At a later evaluation at the clinic there were no complaints (no fever, vomiting, nausea or abdominal pain). After the patient had been discharged from the hospital, the patient did not receive pain medication or anti-emetics. Blood chemistry showed:
Lipase and amylase within normal limits, Gr1: bilirubin, AST, and ALT Gr2: Alkaline Phosphatase Grade 3: GGT
1 day after the evaluation at the clinic LDK378 was restarted at a reduced dose of 300mg daily. Nivolumab treatment was restarted about a week later.

Patient had vomited once without nausea or abdominal pain. He was afebrile although once had a fever of 38 degrees. Patient had no physical complaints.

Lab values when Nivolumab was restarted: AST: 120 U/L, ALT: 139 U/L, Bilirubin total 33 Umol/L, Alkaline Phosphatase 551 U/L. Amylase and Lipase were normal.

LDK378 was discontinued and restarted again at 300 mg dose.

### Tumor assessment:

CT scan at the first tumor assessment demonstrated a 62.9% decrease in overall target lesions in the right adrenal gland and abdominal lymph nodes from the baseline CT scan. There is also a non-target lesion in the Left lower lobe of the lung which was assessed as present.

| **RECIST Target lesion** | | | | |
|---|---|---|---|---|
| Location | Adrenal, lesion #1 | | | |
| | Lesion diameter | | | |
| Baseline | 17 mm | | | |
| Tumor Assessment | 0 mm | | | |
| | | | | |

| Location | Other lymph nodes (abdominal), lesion #2 | | | |
|---|---|---|---|---|
| | Lesion diameter | | | |
| Baseline | 22 mm | | | |
| Tumor Assessment | 7 mm | | | |
| | | | | |

| Location | Other lymph nodes (abdominal), lesion #3 | | | |
|---|---|---|---|---|
| | Lesion diameter | | | |
| Baseline | 23 mm | | | |
| Tumor Assessment | 16 mm | | | |

| **RECIST Non-Target lesion** | | | | |
|---|---|---|---|---|
| Location | Lung, lower lobe | | | |
| | Lesion status | | | |
| Baseline | Present | | | |
| Tumor Assessment | Present | | | |
| | | | | |

| Location | Other lymph nodes (abdominal) | | | |
|---|---|---|---|---|
| | Lesion diameter | | | |
| Evaluation | 22 mm | | | |
| Baseline | 7 mm | | | |

### Example 8: A Phase II, Multicenter, Open-Label Study of EGF816 in Combination with Nivolumab in Adult Patients with EGFR Mutated Non-Small Cell Lung Cancer

Currently approved EGFR TKIs are effective in activated EGFR mutant NSCLC, however nearly all patients develop resistance. Harnessing the immune system to treat patients with non-small cell lung cancer (NSCLC) is a novel and exciting new treatment approach.

Concurrent treatment with an immune checkpoint inhibitor along with a targeted therapy is considered to be safe and is expected to result in durable and sustained responses. Nivolumab is combined with the third generation EGFR TKI, EGF816, in EGFR T790M NSCLC patients who have developed resistance to EGFR TKI treatment. It is expected that the combination of Nivolumab with the EGFR inhibitor, EGF816, provides sustained clinical benefit to NSCLC patients whose tumors have become resistant to EGFR TKI treatment through acquiring T790M mutation by stimulating the host immune system and inhibiting EGFR T790M.

This study is a phase II, multicenter, open-label study of EGF816 in combination with Nivolumab in adult patients with EGFR mutated non-small cell lung cancer, e.g., patients with NSCLC progressing on standard of care (*i.e.*, erlotinib or gefitinib for EGFR-mutant NSCLC).

An exemplary dose of EGF816 is 150 mg qd on a continuous daily dose for EGF816 (capsule formulation). Different EGF816 doses may also be used. EGF816 is administered prior to Nivolumab. A minimum of 1 hour must pass from the time of EGF816 administration to the administration of EGF816.

The dose and schedule of Nivolumab is 3 mg/kg every 2 weeks. This dose and schedule selection is based on results of safety, efficacy, and exposure-response analyses obtained from studies. This dose and schedule of Nivolumab has been safely combined with other EGFR inhibitors (*e.g.*, erlotinib) at registered doses as well as with other standard of care therapies.

This is a phase II, multi-center, open-label study of patients with advanced NSCLC.

Patients are allocated based on their EGFR status *e.g.,* EGFR-T790M NSCLC.

Suitable patients may be patients with advanced, recurrent or metastatic/unresectable EGFRT790M NSCLC progressing on standard of care (*i.e.*, erlotinib, gefitinib or other approved EGFR TKI).

EGFR mutation status may be determined by tests available in the art, *e.g.,* QIAGEN therascreen® EGFR test. The therascreen EGFR RGQ PCR Kit is an FDA-approved, qualitative real-time PCR assay for the detection of specific mutations in the EGFR oncogene. Evidence of EGFR mutation can be obtained from existing local data and testing of tumor samples.EGFR mutation status may be determined from any available tumor tissue.

Patients are treated as follows:
EGF816 is administered prior to nivolumab+Nivolumab
A cycle will be defined as 28 days.

At least six patients of each group constitute a safety monitoring cohort for that group.

For each cohort, patients are treated with either Nivolumab 3mg/kg every two weeks and EGF816 at 150mg qd (once daily).

As part of the safety monitoring cohort, steady state PK profile for EGF816 is collected on Cycle 1 day 15; and trough samples for Nivolumab is collected on Cycle 1 Day 15.

The treatment period begins on Cycle 1 Day 1. The study treatment is administered during 28-days cycles. Patients are treated until unacceptable toxicity, progressive disease, treatment discontinuation at the discretion of the investigator, or withdrawal of consent.

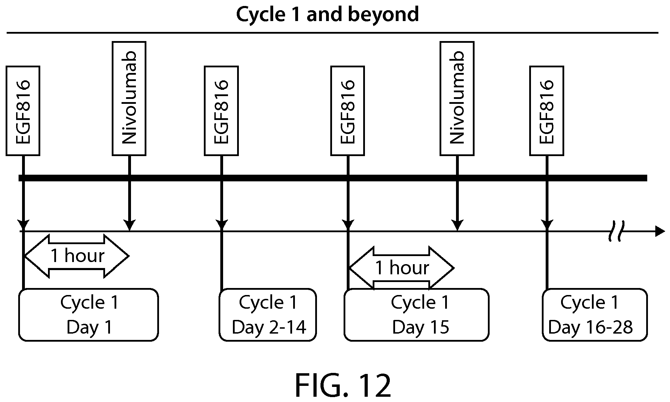

The sequence of drug administration for patients enrolled in the Phase II trial that will be treated with EGF816 and Nivolumab is shown in **Figure 12****.**

**Table 2: Trial objectives and related endpoints**

| **Objective** | **Endpoint** |
|---|---|
| **Primary** | |
| To estimate the clinical activity of Nivolumab in combination with EGF816 | 6 month PFS rate using RECIST version 1.1 (6mo PFS rate=6 cycles=168 days) |

| **Secondary** | |
|---|---|
| To evaluate the preliminary antitumor activity of EGF816 and Nivolumab | ORR, DCR, other PFS measures, OS |
| To characterize the safety and tolerability of EGF816 and Nivolumab | Safety, incidence and severity of AEs, including changes in hematology and chemistry values, vital signs and ECGs |
| | Tolerability: Dose interruptions, reductions, and dose intensity |
| To evaluate PK of EGF816 and Nivolumab in the combination setting | PK parameters of Nivolumab and EGF816 as Cmax, AUC and Cmin |

| **Exploratory** | |
|---|---|
| Explore correlation of baseline PD-L1 & other immune checkpoint molecules levels in relation to disease progression | Baseline levels of PD-L1 & other immune checkpoint molecules in tumor |

**Table 3: Dose and treatment schedule**

| **Study treatments** | **Pharmaceutical form and route of administration** | **Dose** | **Frequency and/or Regimen** |
|---|---|---|---|
| EGF816 | Capsule for oral use | 150 mg | Daily |
| Nivolumab | Solution for injection | 3mg/kg | Every two weeks |

## Claims

1. A combination comprising an anti-PD-1 antibody chosen from Nivolumab, Pembrolizumab or Pidilizumab, and LCL161, for use in a method of treating a cancer or a hematopoiesis disorder in a subject, wherein LCL161 is (S)-N-((S)-1-cyclohexyl-2-((S)-2-(4-(4-fluorobenzoyl)thiazol-2-yl)pyrrolidin-1-yl)-2-oxoethyl)-2-(methylamino)propanamide or a pharmaceutically acceptable salt thereof.

2. The combination for use according to claim 1, wherein the combination is for use in a method of treating cancer.

3. The combination for use of preceding claim 1 or claim 2, wherein the anti-PD-1 antibody and LCL161 are administered together in a single composition or administered separately in two or more different compositions or dosage forms.

4. The combination for use of preceding claim 1 or claim 2, wherein the anti-PD-1 antibody is administered concurrently with, prior to, or subsequent to LCL161.

5. The combination for use according to any of claims 1-4, wherein the cancer is treated by reducing growth, survival, or viability, or all, of a cancer cell.

6. The combination for use of any of claims 1-5, wherein the anti-PD-1 antibody comprises:
the heavy chain amino acid sequence of SEQ ID NO: 2 and the light chain amino acid sequence of SEQ ID NO: 3; or
the heavy chain amino acid sequence of SEQ ID NO: 4 and the light chain amino acid sequence of SEQ ID NO: 5.

7. The combination for use of any preceding claim, wherein the cancer is a solid tumor, or a soft tissue tumor chosen from a hematological cancer, leukemia, lymphoma, myeloma, or multiple myeloma, or a metastatic lesion of any of the aforesaid cancers;
a solid tumor from the lung, breast, ovarian, lymphoid, gastrointestinal, anal, genitals and genitourinary tract, renal, urothelial, bladder cells, prostate, pharynx, CNS, brain, neural or glial cells, head and neck, skin, melanoma, pancreas, colon, rectum, renal-cell carcinoma, liver, lung, non-small cell lung cancer, small intestine or the esophagus;
a hematological cancer chosen from a Hogdkin lymphoma, a non-Hodgkin lymphoma, a lymphocytic leukemia, or a myeloid leukemia.

8. The combination for use of claim 7, wherein the cancer is a solid tumor, a breast cancer, a colon cancer, or a pancreatic cancer; or a hematological malignancy, optionally multiple myeloma.

9. The combination for use of any of claims 1-8, wherein the subject is a human, optionally a human patient having cancer, or a human patient at risk of having a cancer.

10. The combination for use of any of claims 1-9, wherein the anti-PD-1 antibody molecule is administered by injection, optionally subcutaneously or intravenously, at a dose of 1 to 30 mg/kg, 5 to 25 mg/kg, 10 to 20 mg/kg, 1 to 5 mg/kg, or 3 mg/kg, optionally once a week to once every 2, 3, or 4 weeks, optionally wherein:
(i) the anti-PD-1 antibody molecule is administered at a dose from 1 to 20 mg/kg every other week;
(ii) the anti-PD-1 antibody molecule, optionally Nivolumab, is administered intravenously at a dose from 1 mg/kg to 3 mg/kg, *e.g.,* 1 mg/kg, 2 mg/kg or 3 mg/kg, every two weeks; or
(iii) the anti-PD-1 antibody molecule, optionally Nivolumab, is administered intravenously at a dose of 2 mg/kg at 3-week intervals.

11. The combination for use of any of claims 1-10, wherein LCL161 is administered at an oral dose of 10-3000 mg, *e.g.*, 20-2400 mg, 50-1800 mg, 100-1500 mg, 200-1200 mg, 300-900 mg, *e.g.*, 600 mg, 900 mg, 1200 mg, 1500 mg, 1800 mg, 2100 mg, or 2400 mg.

12. A composition, or a kit comprising one or more compositions or dosage forms, comprising an anti-PD-1 antibody and a second therapeutic agent, wherein:
(i) the anti-PD-1 antibody is chosen from Nivolumab, Pembrolizumab, or Pidilizumab; and
(ii) the second therapeutic agent is LCL161, wherein LCL161 is (S)-N-((S)-1-cyclohexyl-2-((S)-2-(4-(4-fluorobenzoyl)thiazol-2-yl)pyrrolidin-1-yl)-2-oxoethyl)-2-(methylamino)propanamide or a pharmaceutically acceptable salt thereof.

13. The combination for use of any of claims 1-11, or the composition or kit of claim 12, wherein LCL161 has the following structure: 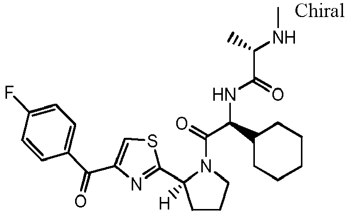 or a pharmaceutically acceptable salt thereof.

14. The composition or kit of claim 12 or claim 13, wherein the anti-PD-1 antibody comprises:
the heavy chain amino acid sequence of SEQ ID NO: 2 and the light chain amino acid sequence of SEQ ID NO: 3; or
the heavy chain amino acid sequence of SEQ ID NO: 4 and the light chain amino acid sequence of SEQ ID NO: 5.

## Patentansprüche

1. Kombination, umfassend einen Anti-PD-1-Antikörper, ausgewählt aus Nivolumab, Pembrolizumab oder Pidilizumab, und LCL161 für die Verwendung in einem Verfahren zum Behandeln von Krebs oder einer Blutbildungsstörung bei einem Subjekt, wobei LCL161 (S)-N-((S)-1-Cyclohexyl-2-((S)-2-(4-(4-fluorbenzoyl)thiazol-2-yl)pyrrolidin-1-yl)-2-oxoethyl)-2-(methylamino)propanamid oder ein pharmazeutisch unbedenkliches Salz davon ist.

2. Kombination für die Verwendung nach Anspruch 1, wobei die Kombination für die Verwendung in einem Verfahren für das Behandeln von Krebs bestimmt ist.

3. Kombination für die Verwendung nach dem vorhergehenden Anspruch 1 oder Anspruch 2, wobei der Anti-PD-1-Antikörper und LCL161 zusammen in einer einzelnen Zusammensetzung oder separat in zwei oder mehr unterschiedlichen Zusammensetzungen oder Dosierungsformen verabreicht werden.

4. Kombination für die Verwendung nach dem vorhergehenden Anspruch 1 oder Anspruch 2, wobei der Anti-PD-1-Antikröper gleichzeitig mit, vor oder nach LCL161 verabreicht wird.

5. Kombination für die Verwendung nach einem der Ansprüche 1-4, wobei der Krebs durch das Reduzieren des Wachstums, des Überlebens oder der Lebensfähigkeit, oder allem davon, einer Krebszelle behandelt wird.

6. Kombination für die Verwendung nach einem der Ansprüche 1-5, wobei der Anti-PD-1-Antikörper Folgendes umfasst:
die Schwerketten-Aminosäuresequenz von SEQ ID NO: 2 und die Leichtketten-Aminosäuresequenz von SEQ ID NO: 3; oder
die Schwerketten-Aminosäuresequenz von SEQ ID NO: 4 und die Leichtketten-Aminosäuresequenz von SEQ ID NO: 5.

7. Kombination für die Verwendung nach einem der vorhergehenden Ansprüche, wobei es sich bei dem Krebs um Folgendes handelt: einen soliden Tumor oder einen Weichteiltumor, der ausgewählt ist aus einem hämatologischen Krebs, einer Leukämie, einem Lymphom, einem Myelom oder einem multiplen Myelom oder einer metastasenbildenden Läsion einer der vorhergehenden Krebsarten;
einen soliden Tumor der Lunge, der Brust, der Eierstöcke, des Lymphsystems, des Gastrointestinaltrakts, des Anus, der Genitalien und des Urogenitaltrakts, der Nieren, der Harnwege, der Blasenzellen, der Prostata, des Pharynx, des ZNS, des Gehirns, der Nerven- oder Gliazellen, des Kopfs und des Halses, der Haut, ein Melanom, der Bauchspeicheldrüse, des Darms, des Rektums, ein Nierenzellkarzinom, der Leber, Lunge, ein nicht kleinzelliges Lungenkarzinom, des Dünndarms oder der Speiseröhre;
einen hämatologischen Krebs, ausgewählt aus einem Hodgkin-Lymphom, einem Non-Hodgkin-Lymphom, einer lymphatischen Leukämie oder einer myeloischen Leukämie.

8. Kombination für die Verwendung nach Anspruch 7, wobei der Krebs ein solider Tumor, Brustkrebs, Darmkrebs oder Bauchspeicheldrüsenkrebs oder eine hämatologische Malignität, optional ein multiples Myelom, ist.

9. Kombination für die Verwendung nach einem der Ansprüche 1-8, wobei das Subjekt ein Mensch ist, optional ein menschlicher Patient, der an Krebs leidet, oder ein menschlicher Patient, bei dem ein Krebsrisiko besteht.

10. Kombination für die Verwendung nach einem der Ansprüche 1-9, wobei das Anti-PD-1-Antikörpermolekül durch Injektion, optional subkutan oder intravenös, mit einer Dosis von 1 bis 30 mg/kg, 5 bis 25 mg/kg, 10 bis 20 mg/kg, 1 bis 5 mg/kg oder 3 mg/kg, optional einmal wöchentlich bis einmal alle 2, 3 oder 4 Wochen, verabreicht wird, wobei optional:
(i) das Anti-PD-1-Antikörpermolekül mit einer Dosis von 1 bis 20 mg/kg alle 2 Wochen verabreicht wird;
(ii) das Anti-PD-1-Antikörpermolekül, optional Nivolumab, intravenös mit einer Dosis von 1 mg/kg bis 3 mg/kg, *z. B.* 1 mg/kg, 2 mg/kg oder 3 mg/kg, alle zwei Wochen verabreicht wird; oder
(iii) das Anti-PD-1-Antikörpermolekül, optional Nivolumab, intravenös mit einer Dosis von 2 mg/kg in Intervallen von 3 Wochen verabreicht wird.

11. Kombination für die Verwendung nach einem der Ansprüche 1-10, wobei LCL161 mit einer oralen Dosis von 10-3000 mg, *z. B.* 20-2400 mg, 50-1800 mg, 100-1500 mg, 200-1200 mg, 300-900 mg, *z. B.* 600 mg, 900 mg, 1200 mg, 1500 mg, 1800 mg, 2100 mg oder 2400 mg, verabreicht wird.

12. Zusammensetzung oder Satz, bestehend aus einer oder mehreren Zusammensetzungen oder Dosierungsformen, umfassend einen Anti-PD-1-Antikörper und ein zweites Therapeutikum, wobei:
(i) der Anti-PD-1-Antikörper ausgewählt ist aus Nivolumab, Pembrolizumab oder Pidilizumab; und
(ii) das zweite Therapeutikum LCL161 ist, wobei LCL161 (S)-N-((S)-1-Cyclohexyl-2-((S)-2-(4-(4-fluorbenzoyl)thiazol-2-yl)pyrrolidin-1-yl)-2-oxoethyl)-2-(methylamino)propanamid oder ein pharmazeutisch unbedenkliches Salz davon ist.

13. Kombination für die Verwendung nach einem der Ansprüche 1-11 oder Zusammensetzung oder Satz nach Anspruch 12, wobei LCL161 die folgende Struktur aufweist: 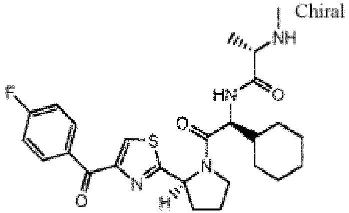 oder ein pharmazeutisch unbedenkliches Salz davon.

14. Zusammensetzung oder Satz nach Anspruch 12 oder Anspruch 13, wobei der Anti-PD-1-Antikörper Folgendes umfasst:
die Schwerketten-Aminosäuresequenz von SEQ ID NO: 2 und die Leichtketten-Aminosäuresequenz von SEQ ID NO: 3; oder
die Schwerketten-Aminosäuresequenz von SEQ ID NO: 4 und die Leichtketten-Aminosäuresequenz von SEQ ID NO: 5.

## Revendications

1. Combinaison comprenant un anticorps anti-PD-1 choisi parmi le Nivolumab, le Pembrolizumab ou le Pidilizumab, et du LCL161, destiné à être utilisé dans un procédé de traitement d'un cancer ou d'un trouble de l'hémopoïèse chez un sujet, dans laquelle le LCL161 est le (S)-N-((S)-1-cyclohexyl-2-((S)-2-(4-(4-fluorobenzoyl)thiazol-2-yl)pyrrolidin-1-yl)-2-oxoéthyl)-2-(méthylamino)propanamide ou un sel pharmaceutiquement acceptable de celui-ci.

2. Combinaison destinée à être utilisée selon la revendication 1, dans laquelle la combinaison est destinée à être utilisée dans un procédé de traitement du cancer.

3. Combinaison destinée à être utilisée selon la revendication 1 ou la revendication 2, dans laquelle l'anticorps anti-PD-1 et le LCL161 sont administrés ensemble en une composition unique ou administrés séparément en deux compositions ou formes galéniques différentes ou plus.

4. Combinaison destinée à être utilisée selon la revendication 1 ou la revendication 2 précédente, dans laquelle l'anticorps anti-PD-1 est administré simultanément avec, avant, ou après le LCL161.

5. Combinaison destinée à être utilisée selon l'une quelconque des revendications 1 à 4, dans laquelle le cancer est traité en réduisant la croissance, la survie, ou la viabilité d'une cellule cancéreuse, ou les trois.

6. Combinaison destinée à être utilisée selon l'une quelconque des revendications 1 à 5, dans laquelle l'anticorps anti-PD-1 comprend :
la séquence d'acides aminés de chaîne lourde de la SEQ ID NO : 2 et la séquence d'acides aminés de chaîne légère de la SEQ ID NO : 3 ;
ou la séquence d'acides aminés de chaîne lourde de la SEQ ID NO : 4 et la séquence d'acides aminés de chaîne légère de la SEQ ID NO : 5.

7. Combinaison destinée à être utilisée selon l'une quelconque des revendications précédentes, dans laquelle le cancer est une tumeur solide, ou une tumeur de tissu mou choisie parmi un cancer hématologique, une leucémie, un lymphome, un myélome, ou un myélome multiple, ou une lésion métastasique de l'un quelconque des cancers susdits ;
une tumeur solide du poumon, du sein, de l'ovaire, lymphoïde, gastro-intestinale, anale, des organes génitaux, et de l'appareil génito-urinaire, rénale, urothéliale, des cellules de la vessie, de la prostate, du pharynx, du SNC, du cerveau, des cellules neurales ou gliales, de la tête et du cou, de la peau, de mélanome, du pancréas, du côlon, du rectum, de carcinome de cellules rénales, du foie, du poumon, de cancer du poumon non à petites cellules, de l'intestin grêle ou de l'œsophage ;
un cancer hématologique choisi parmi un lymphome hodgkinien, un lymphome non hodgkinien, une leucémie lymphocytaire, ou une leucémie myéloïde.

8. Combinaison destinée à être utilisée selon la revendication 7, dans laquelle le cancer est une tumeur solide, un cancer du sein, un cancer du côlon, ou un cancer du pancréas ; ou une malignité hématologique, facultativement un myélome multiple.

9. Combinaison destinée à être utilisée selon l'une quelconque des revendications 1 à 8, dans laquelle le sujet est un humain, facultativement un patient humain atteint d'un cancer, ou un patient humain susceptible d'être atteint d'un cancer.

10. Combinaison destinée à être utilisée selon l'une quelconque des revendications 1 à 9, dans laquelle la molécule d'anticorps anti-PD-1 est administrée par injection, facultativement par voie sous-cutanée ou intraveineuse, à une dose de 1 à 30 mg/kg, 5 à 25 mg/kg, 10 à 20 mg/kg, 1 à 5 mg/kg, ou 3 mg/kg, facultativement une fois par semaine à une fois toutes les 2, 3, ou 4 semaines, facultativement dans laquelle :
(i) la molécule d'anticorps anti-PD-1 est administrée à une dose de 1 à 20 mg/kg une semaine sur deux ;
(ii) la molécule d'anticorps anti-PD-1, facultativement du Nivolumab, est administrée par voie intraveineuse à une dose de 1 mg/kg à 3 mg/kg, par exemple 1 mg/kg, 2 mg/kg ou 3 mg/kg, toutes les deux semaines ; ou
(iii) la molécule d'anticorps anti-PD-1, facultativement du Nivolumab, est administrée par voie intraveineuse à une dose de 2 mg/kg à des intervalles de 3 semaines.

11. Combinaison destinée à être utilisée selon l'une quelconque des revendications 1 à 10, dans laquelle le LCL161 est administré à une dose orale de 10 à 3 000 mg, par exemple 20 à 2 400 mg, 50 à 1 800 mg, 100 à 1 500 mg, 200 à 1 200 mg, 300 à 900 mg, par exemple 600 mg, 900 mg, 1 200 mg, 1 500 mg, 1 800 mg, 2 100 mg, ou 2 400 mg.

12. Composition, ou trousse comprenant une ou plusieurs compositions ou formes galéniques, comprenant un anticorps anti-PD-1 et un deuxième agent thérapeutique, dans laquelle :
(i) l'anticorps anti-PD-1 est choisi parmi le Nivolumab, le Pembrolizumab, ou le Pidilizumab ; et
(ii) le deuxième agent thérapeutique est le LCL161, dans laquelle le LCL161 est le (S)-N-((S)-1-cyclohexyl-2-((S)-2-(4-(4-fluorobenzoyl)thiazol-2-yl)pyrrolidin-1-yl)-2-oxoéthyl)-2-(méthylamino)propanamide ou un sel pharmaceutiquement acceptable de celui-ci.

13. Combinaison destinée à être utilisée selon l'une quelconque des revendications 1 à 11, ou composition ou trousse selon la revendication 12, dans laquelle le LCL161 a la structure suivante : 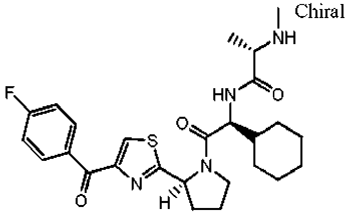 ou un sel pharmaceutiquement acceptable de celui-ci.

14. Composition ou trousse selon la revendication 12 ou la revendication 13, dans laquelle l'anticorps anti-PD-1 comprend :
la séquence d'acides aminés de chaîne lourde de la SEQ ID NO : 2 et la séquence d'acides aminés de chaîne légère de la SEQ ID NO : 3 ;
ou la séquence d'acides aminés de chaîne lourde de la SEQ ID NO : 4 et la séquence d'acides aminés de chaîne légère de la SEQ ID NO : 5.


### SEQUENCE LISTING

<110> NOVARTIS AG
<120> COMBINATION THERAPIES
<130> C2160-7006WO
<140>
   <141>
<150> 62/059,832
   <151> 2014-10-03
<160> 7
<170> PatentIn version 3.5
<210> 1
   <211> 5
   <212> PRT
   <213> Unknown
<220>
   <221> source
   <223> /note="Description of Unknown: PD-1 motif peptide"
<400> 1
   Met Tyr Pro Pro Tyr
   1               5
<210> 2
   <211> 440
   <212> PRT
   <213> Artificial Sequence
<220>
   <221> source
   <223> /note="Description of Artificial Sequence: Synthetic polypeptide"
<400> 2
   Gln Val Gln Leu Val Glu Ser Gly Gly Gly Val Val Gln Pro Gly Arg
   1               5                   10                  15
   Ser Leu Arg Leu Asp Cys Lys Ala Ser Gly Ile Thr Phe Ser Asn Ser
               20                  25                  30
   Gly Met His Trp Val Arg Gln Ala Pro Gly Lys Gly Leu Glu Trp Val
           35                  40                  45
   Ala Val Ile Trp Tyr Asp Gly Ser Lys Arg Tyr Tyr Ala Asp Ser Val
       50                  55                  60
   Lys Gly Arg Phe Thr Ile Ser Arg Asp Asn Ser Lys Asn Thr Leu Phe
   65                  70                  75                  80
   Leu Gln Met Asn Ser Leu Arg Ala Glu Asp Thr Ala Val Tyr Tyr Cys
                   85                  90                  95
   Ala Thr Asn Asp Asp Tyr Trp Gly Gln Gly Thr Leu Val Thr Val Ser
               100                 105                 110
   Ser Ala Ser Thr Lys Gly Pro Ser Val Phe Pro Leu Ala Pro Cys Ser
           115                 120                 125
   Arg Ser Thr Ser Glu Ser Thr Ala Ala Leu Gly Cys Leu Val Lys Asp
       130                 135                 140
   Tyr Phe Pro Glu Pro Val Thr Val Ser Trp Asn Ser Gly Ala Leu Thr
   145                 150                 155                 160
   Ser Gly Val His Thr Phe Pro Ala Val Leu Gln Ser Ser Gly Leu Tyr
                   165                 170                 175
   Ser Leu Ser Ser Val Val Thr Val Pro Ser Ser Ser Leu Gly Thr Lys
               180                 185                 190
   Thr Tyr Thr Cys Asn Val Asp His Lys Pro Ser Asn Thr Lys Val Asp
           195                 200                 205
   Lys Arg Val Glu Ser Lys Tyr Gly Pro Pro Cys Pro Pro Cys Pro Ala
       210                 215                 220
   Pro Glu Phe Leu Gly Gly Pro Ser Val Phe Leu Phe Pro Pro Lys Pro
   225                 230                 235                 240
   Lys Asp Thr Leu Met Ile Ser Arg Thr Pro Glu Val Thr Cys Val Val
                   245                 250                 255
   Val Asp Val Ser Gln Glu Asp Pro Glu Val Gln Phe Asn Trp Tyr Val
               260                 265                 270
   Asp Gly Val Glu Val His Asn Ala Lys Thr Lys Pro Arg Glu Glu Gln
           275                 280                 285
   Phe Asn Ser Thr Tyr Arg Val Val Ser Val Leu Thr Val Leu His Gln
       290                 295                 300
   Asp Trp Leu Asn Gly Lys Glu Tyr Lys Cys Lys Val Ser Asn Lys Gly
   305                 310                 315                 320
   Leu Pro Ser Ser Ile Glu Lys Thr Ile Ser Lys Ala Lys Gly Gln Pro
                   325                 330                 335
   Arg Glu Pro Gln Val Tyr Thr Leu Pro Pro Ser Gln Glu Glu Met Thr
   340 345 350
   Lys Asn Gln Val Ser Leu Thr Cys Leu Val Lys Gly Phe Tyr Pro Ser
   355 360 365
   Asp Ile Ala Val Glu Trp Glu Ser Asn Gly Gln Pro Glu Asn Asn Tyr
   370 375 380
   Lys Thr Thr Pro Pro Val Leu Asp Ser Asp Gly Ser Phe Phe Leu Tyr
   385 390 395 400
   Ser Arg Leu Thr Val Asp Lys Ser Arg Trp Gln Glu Gly Asn Val Phe
   405 410 415
   Ser Cys Ser Val Met His Glu Ala Leu His Asn His Tyr Thr Gln Lys
   420 425 430
   Ser Leu Ser Leu Ser Leu Gly Lys
   435 440
<210> 3
   <211> 214
   <212> PRT
   <213> Artificial Sequence
<220>
   <221> source
   <223> /note="Description of Artificial Sequence: Synthetic polypeptide"
<400> 3
   Glu Ile Val Leu Thr Gln Ser Pro Ala Thr Leu Ser Leu Ser Pro Gly
   1 5 10 15
   Glu Arg Ala Thr Leu Ser Cys Arg Ala Ser Gln Ser Val Ser Ser Tyr
   20 25 30
   Leu Ala Trp Tyr Gln Gln Lys Pro Gly Gln Ala Pro Arg Leu Leu Ile
   35 40 45
   Tyr Asp Ala Ser Asn Arg Ala Thr Gly Ile Pro Ala Arg Phe Ser Gly
   50 55 60
   Ser Gly Ser Gly Thr Asp Phe Thr Leu Thr Ile Ser Ser Leu Glu Pro
   65 70 75 80
   Glu Asp Phe Ala Val Tyr Tyr Cys Gln Gln Ser Ser Asn Trp Pro Arg
   85 90 95
   Thr Phe Gly Gln Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 4
   <211> 447
   <212> PRT
   <213> Artificial Sequence
<220>
   <221> source
   <223> /note="Description of Artificial Sequence: Synthetic polypeptide"
<400> 4
   Gln Val Gln Leu Val Gln Ser Gly Val Glu Val Lys Lys Pro Gly Ala
   1               5                   10                  15
   Ser Val Lys Val Ser Cys Lys Ala Ser Gly Tyr Thr Phe Thr Asn Tyr
               20                  25                  30
   Tyr Met Tyr Trp Val Arg Gln Ala Pro Gly Gln Gly Leu Glu Trp Met
           35                  40                  45
   Gly Gly Ile Asn Pro Ser Asn Gly Gly Thr Asn Phe Asn Glu Lys Phe
       50                  55                  60
   Lys Asn Arg Val Thr Leu Thr Thr Asp Ser Ser Thr Thr Thr Ala Tyr
   65                  70                  75                  80
   Met Glu Leu Lys Ser Leu Gln Phe Asp Asp Thr Ala Val Tyr Tyr Cys
                   85                  90                  95
   Ala Arg Arg Asp Tyr Arg Phe Asp Met Gly Phe Asp Tyr Trp Gly Gln
               100                 105                 110
   Gly Thr Thr Val Thr Val Ser Ser Ala Ser Thr Lys Gly Pro Ser Val
           115                 120                 125
   Phe Pro Leu Ala Pro Cys Ser Arg Ser Thr Ser Glu Ser Thr Ala Ala
       130                 135                 140
   Leu Gly Cys Leu Val Lys Asp Tyr Phe Pro Glu Pro Val Thr Val Ser
   145                 150                 155                 160
   Trp Asn Ser Gly Ala Leu Thr Ser Gly Val His Thr Phe Pro Ala Val
                   165                 170                 175
   Leu Gln Ser Ser Gly Leu Tyr Ser Leu Ser Ser Val Val Thr Val Pro
               180                 185                 190
   Ser Ser Ser Leu Gly Thr Lys Thr Tyr Thr Cys Asn Val Asp His Lys
           195                 200                 205
   Pro Ser Asn Thr Lys Val Asp Lys Arg Val Glu Ser Lys Tyr Gly Pro
       210                 215                 220
   Pro Cys Pro Pro Cys Pro Ala Pro Glu Phe Leu Gly Gly Pro Ser Val
   225                 230                 235                 240
   Phe Leu Phe Pro Pro Lys Pro Lys Asp Thr Leu Met Ile Ser Arg Thr
                   245                 250                 255
   Pro Glu Val Thr Cys Val Val Val Asp Val Ser Gln Glu Asp Pro Glu
               260                 265                 270
   Val Gln Phe Asn Trp Tyr Val Asp Gly Val Glu Val His Asn Ala Lys
           275                 280                 285
   Thr Lys Pro Arg Glu Glu Gln Phe Asn Ser Thr Tyr Arg Val Val Ser
       290                 295                 300
   Val Leu Thr Val Leu His Gln Asp Trp Leu Asn Gly Lys Glu Tyr Lys
   305                 310                 315                 320
   Cys Lys Val Ser Asn Lys Gly Leu Pro Ser Ser Ile Glu Lys Thr Ile
                   325                 330                 335
   Ser Lys Ala Lys Gly Gln Pro Arg Glu Pro Gln Val Tyr Thr Leu Pro
               340                 345                 350
   Pro Ser Gln Glu Glu Met Thr Lys Asn Gln Val Ser Leu Thr Cys Leu
           355                 360                 365
   Val Lys Gly Phe Tyr Pro Ser Asp Ile Ala Val Glu Trp Glu Ser Asn
       370                 375                 380
   Gly Gln Pro Glu Asn Asn Tyr Lys Thr Thr Pro Pro Val Leu Asp Ser
   385                 390                 395                 400
   Asp Gly Ser Phe Phe Leu Tyr Ser Arg Leu Thr Val Asp Lys Ser Arg
                   405                 410                 415
   Trp Gln Glu Gly Asn Val Phe Ser Cys Ser Val Met His Glu Ala Leu
               420                 425                 430
   His Asn His Tyr Thr Gln Lys Ser Leu Ser Leu Ser Leu Gly Lys
           435                 440                 445
<210> 5
   <211> 218
   <212> PRT
   <213> Artificial Sequence
<220>
   <221> source
   <223> /note="Description of Artificial Sequence: Synthetic polypeptide"
<400> 5
   Glu Ile Val Leu Thr Gln Ser Pro Ala Thr Leu Ser Leu Ser Pro Gly
   1               5                   10                  15
   Glu Arg Ala Thr Leu Ser Cys Arg Ala Ser Lys Gly Val Ser Thr Ser
               20                  25                  30
   Gly Tyr Ser Tyr Leu His Trp Tyr Gln Gln Lys Pro Gly Gln Ala Pro
           35                  40                  45
   Arg Leu Leu Ile Tyr Leu Ala Ser Tyr Leu Glu Ser Gly Val Pro Ala
       50                  55                  60
   Arg Phe Ser Gly Ser Gly Ser Gly Thr Asp Phe Thr Leu Thr Ile Ser
   65                  70                  75                  80
   Ser Leu Glu Pro Glu Asp Phe Ala Val Tyr Tyr Cys Gln His Ser Arg
                   85                  90                  95
   Asp Leu Pro Leu Thr Phe Gly Gly Gly Thr Lys Val Glu Ile Lys Arg
               100                 105                 110
   Thr Val Ala Ala Pro Ser Val Phe Ile Phe Pro Pro Ser Asp Glu Gln
           115                 120                 125
   Leu Lys Ser Gly Thr Ala Ser Val Val Cys Leu Leu Asn Asn Phe Tyr
       130                 135                 140
   Pro Arg Glu Ala Lys Val Gln Trp Lys Val Asp Asn Ala Leu Gln Ser
   145                 150                 155                 160
   Gly Asn Ser Gln Glu Ser Val Thr Glu Gln Asp Ser Lys Asp Ser Thr
                   165                 170                 175
   Tyr Ser Leu Ser Ser Thr Leu Thr Leu Ser Lys Ala Asp Tyr Glu Lys
               180                 185                 190
   His Lys Val Tyr Ala Cys Glu Val Thr His Gln Gly Leu Ser Ser Pro
           195                 200                 205
   Val Thr Lys Ser Phe Asn Arg Gly Glu Cys
       210                 215
<210> 6
   <211> 118
   <212> PRT
   <213> Artificial Sequence
<220>
   <221> source
   <223> /note="Description of Artificial Sequence: Synthetic polypeptide"
<400> 6
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ile Met Met Trp Val Arg Gln Ala Pro Gly Lys Gly Leu Glu Trp Val
           35                  40                  45
   Ser Ser Ile Tyr Pro Ser Gly Gly Ile Thr Phe Tyr Ala Asp Lys Gly
       50                  55                  60
   Arg Phe Thr Ile Ser Arg Asp Asn Ser Lys Asn Thr Leu Tyr Leu Gln
   65                  70                  75                  80
   Met Asn Ser Leu Arg Ala Glu Asp Thr Ala Val Tyr Tyr Cys Ala Arg
                   85                  90                  95
   Ile Lys Leu Gly Thr Val Thr Thr Val Asp Tyr Trp Gly Gln Gly Thr
               100                 105                 110
   Leu Val Thr Val Ser Ser
           115
<210> 7
   <211> 110
   <212> PRT
   <213> Artificial Sequence
<220>
   <221> source
   <223> /note="Description of Artificial Sequence: Synthetic polypeptide"
<400> 7
   Gln Ser Ala Leu Thr Gln Pro Ala Ser Val Ser Gly Ser Pro Gly Gln
   1               5                   10                  15
   Ser Ile Thr Ile Ser Cys Thr Gly Thr Ser Ser Asp Val Gly Gly Tyr
               20                  25                  30
   Asn Tyr Val Ser Trp Tyr Gln Gln His Pro Gly Lys Ala Pro Lys Leu
           35                  40                  45
   Met Ile Tyr Asp Val Ser Asn Arg Pro Ser Gly Val Ser Asn Arg Phe
       50                  55                  60
   Ser Gly Ser Lys Ser Gly Asn Thr Ala Ser Leu Thr Ile Ser Gly Leu
   65                  70                  75                      80
   Gln Ala Glu Asp Glu Ala Asp Tyr Tyr Cys Ser Ser Tyr Thr Ser Ser
                   85                  90                  95
   Ser Thr Arg Val Phe Gly Thr Gly Thr Lys Val Thr Val Leu
               100                 105                 110